# EUROPEAN PATENT APPLICATION

(11) **EP 3 794 939 A1**
(43) Date of publication of application: **24.03.2021**
(21) Application number: 19198975.5
(22) Date of filing: 23.09.2019
(51) Int. Cl.: A01H 1/08, C07K 14/415

(54) **GENERATION OF HAPLOIDS BASED ON MUTATION OF SAD2**

(71) Applicant: Leibniz-Institut für Pflanzengenetik und Kulturpflanzenforschung (IPK), 06466 Seeland OT Gatersleben (DE)
(72) Inventor: DEMIDOV, Dmitri, 06466 Seeland OT Gatersleben (DE); HOUBEN, Andreas, 06466 Seeland OT Gatersleben (DE); FUCHS, Jörg, 06466 Seeland OT Gatersleben (DE); WEISS, Oda, 06466 Seeland OT Gatersleben (DE); MEIER, Karla, 06466 Seeland OT Gatersleben (DE); LERMONTOVA, Inna, 06466 Seeland OT Gatersleben (DE)
(74) Representative: Schrell, Andreas

(57) **Abstract**

The invention identifies plants carrying certain mutations, which can be used as haploid inducers or doubled haploid inducers in plant breeding. According to the invention, significant haploid induction and even doubled haploid induction rates can be observed when at least one functional mutation within a SAD-homology domain is present, wherein the functional mutation affects the expression of certain SAD-homology consensus sequences. The present invention further provides haploid and doubled haploid plants, which are obtained by contacting a first gamete from a plant as identified according to the invention with a second gamete from a plant expressing a wild-type SAD-homology domain to generate a zygote. Furthermore, the present invention relates to a method for identifying a plant having the activity of a haploid inducer or a doubled haploid inducer in a plant population.

## Description

### Technical Field

The invention identifies plants carrying certain mutations, which can be used as haploid inducers or doubled haploid inducers in plant breeding. According to the invention, significant haploid induction and even doubled haploid induction rates can be observed when at least one functional mutation within a SAD-homology domain is present in the plant, wherein the functional mutation affects the expression of certain SAD-homology consensus sequences. The present invention further provides haploid and doubled haploid plants, which are obtained by contacting a first gamete from a plant as identified according to the invention with a second gamete from a plant expressing a wild-type SAD-homology domain to generate a zygote. Corresponding methods for generating haploid plant cells or for directly generating doubled haploid plant cells without the need for a chromosome doubling step are also provided. Furthermore, the present invention relates to a method for identifying a plant having the activity of a haploid inducer or a doubled haploid inducer in a plant population.

### Background

Extensive population screening, even with the latest molecular breeding tools, is both laborious and costly. Efficient plant breeding aims at selecting favourable traits in a germplasm of interest. Heterogeneous breeding material in usually complex plant genomes significantly hampers the plant breeding process so that the selection process may be very cumbersome and time and cost intensive.

The generation and use of haploids is one of the most powerful biotechnological means to improve cultivated plants. The advantage of haploids for breeders is that homozygosity can be achieved already in the first generation after dihaploidization or more generally doubled haploidization by chemical means or by spontaneous chromosome doubling, creating doubled haploid plants, without the need of laborious backcrossing steps to obtain a high degree of homozygosity. Furthermore, the value of haploids in plant research and breeding lies in the fact that the founder cells of doubled haploids are products of meiosis, so that resultant populations constitute pools of diverse recombinant and at the same time genetically fixed individuals. The generation of doubled haploids thus provides not only perfectly useful genetic variability to select from with regard to crop improvement, but is also a valuable means to produce mapping populations, recombinant inbreds as well as instantly homozygous mutants and transgenic lines.

Haploid plants can be obtained by interspecific crosses, in which one parental genome is eliminated after fertilization. It was shown that genome elimination after fertilization could be induced by modifying a centromere protein, the centromere-specific histone CENH3 in *Arabidopsis thaliana* (Ravi and Chan, Haploid plants produced by centromere-mediated genome elimination, Nature, Vol. 464, 2010, 615-619). With the modified haploid inducer lines, haploidization occurred in the progeny when a haploid inducer plant was crossed with a wild-type plant. Interestingly, the haploid inducer line was stable upon selfing, suggesting that a competition between modified and wild-type centromere in the developing hybrid embryo results in centromere inactivation of the inducer parent and consequently in uniparental chromosome elimination.

Haploidization is thus an efficient tool to reduce the length of breeding schemes to create new cultivars of interest. Phenomena like gynogenesis and androgenesis induction can be important during haploid induction. Whilst gynogenesis is a process in which the embryo genome originates exclusively from female origin, following embryogenesis stimulation by a male gamete, androgenesis refers to the development of embryos that contain only the male nuclear genetic background.

In diverse publications, e.g. Portemer et al. 2015 (Portemer, V., Renne, C., Guillebaux, A., & Mercier, R. (2015). Large genetic screens for gynogenesis and androgenesis haploid inducers in Arabidopsis thaliana failed to identify mutants. Frontiers in plant science, 6, 147.), large genetic screens have thus been conducted in an attempt to screen for gynogenesis or androgenesis haploid inducer events. Still, these screens failed to identify specific mutants or a direct correlation between a mutated gene and its haploid induction potential.

To define a more systematic approach, the inventors of the present invention tested selected T-DNA mutants of *Arabidopsis thaliana* for their ability to induce the generation of haploids in crosses with wild-type. This screening has resulted in the selection of a few candidate genes, whereby the most promising candidates are SAD2 (AT2G31660) and SAD1 gene (AT3G59020). Based on these findings, large *in silico* screens were performed to identify consensus sequences based on the SAD family of proteins in major agricultural crop plants to provide proof-of-principle of the broad applicability of SAD mutants in (di)haploid induction, which findings were in turn confirmed by inducer screens with selected plant material. In view of the fact that the nomenclature of the *sad* genes derived from plants strongly varies and is driven by the specific phenotype the relevant authors initially attributed to the characterized mutants, all genes or proteins having a certain sequence identity to SAD2 according to SEQ ID NO: 1 (nucleic acid sequence) or SEQ ID NO: 2 (amino acid sequence) will be referred to as SAD-homology (nucleic acid or amino acid) sequence or protein herein, respectively.

SAD2 (Sensitive to ABA (abscisic acid) and Drought2) was described in Verslues et al. (2006). Mutation of SAD2, an importin β-domain protein in Arabidopsis, alters abscisic acid sensitivity. The Plant Journal, 47(5), 776-787.), where the authors studied sad2-1 mutant *Arabidopsis* plant. SAD2 was found to encode an importin beta-domain family protein likely to be involved in nuclear transport. SAD2 was expressed at a low level in all tissues examined except flowers, but SAD2 expression was not inducible by ABA or stress. Subcellular localization of GFP-tagged SAD2 showed a predominantly nuclear localization, consistent with a role for SAD2 in nuclear transport. Knockout of the closest importin beta homolog of SAD2 in Arabidopsis (sad2-1 mutant) indicates that SAD2 plays a specific role in ABA signaling. Analysis of ABA and stress sensitivity in double mutants of sad2-1 and sad1 suggested that SAD2 acts upstream of or has additive effects with the sad1 gene. Gao et al. ((2008). SAD2 in Arabidopsis functions in trichome initiation through mediating GL3 function and regulating GL1, TTG1 and GL2 expression. Journal of integrative plant biology, 50(7), 906-917.) report that the importin beta-like protein SAD2, is required for trichome initiation. Mutations in SAD2 disrupted trichome initiation resulting in reduced trichome number, but had no effect on trichome development or root hair number and development. Thereby it seems that SAD2 is in the same pathway as two transcription factors (GLABROUS1 (GL1) and GLABRA3 (GL3)).

SAD1 and SAD2, by alignments, have been shown to comprise a so-called Cse1 domain. From previous studies on SAD1 and SAD2 in other contexts, the Cse1 domain is thought to be crucial for the functionality of SAD1 and SAD2 being involved in the exchange of macromolecules between the nucleus and cytoplasm which takes place through nuclear pore complexes within the nuclear membrane. Active transport of large molecules through these pore complexes require carrier proteins, called karyopherins (importins and exportins), which shuttle between the two compartments. Still, the Cse1 family of proteins is found in a variety of different taxa from yeasts to mammals and in quite different classes of proteins. Therefore, it was an object to more precisely define those regions within SAD1 and SAD2 which could be attributed to the effects observed in this invention to identify reliable consensus sequences responsible for the specific new functional effects of SAD1 and SAD2 described herein.

So far, the SAD family of proteins in plants has not yet been associated with haploid or even spontaneous doubled haploid or dihaploid induction events at all. As detailed above, doubled haploid induction naturally usually occurs only in very rare events. Therefore, any induced haploid system requires an additional step of chromosome doubling, e.g. by chemical means. Many of those chemicals traditionally used in chromosome doubling, e.g. colchicine, are rather toxic. Therefore, there exists a great need in defining strategies to obtain doubled haploids from induced haploids in a time-efficient manner preferably without the need of additional chromosome doubling steps further complicating rapid breeding.

Therefore, it was an object of the present invention to explore the haploid induction potential of new mutant plant genes and the corresponding proteins. In particular, it was an object to define promising mutants or knock-outs based on the systematic manipulation of new candidate genes/proteins suitable to be used as maternal and/or paternal haploid inducers for providing effective new means for haploid, or even doubled haploid or dihaploid, induction in relevant agricultural crop plants to expedite breeding programs.

### Summary of the Invention

In a first aspect, the present invention relates to the use of a plant seed, plant cell or part of a plant comprising a nucleotide sequence encoding an amino acid sequence comprising a SAD-homology domain, wherein the SAD-homology domain comprises at least one functional mutation, wherein the at least one functional mutation results in a decreased or abolished expression of the amino acid sequence comprising a SAD-homology domain in comparison to the cognate wild-type amino acid sequence, wherein the functional mutation affects the expression of a SAD-homology domain consensus sequence as set forth in any one of SEQ ID NOs: 19, 25 and 26, or an amino acid sequence having at least 90%, 91%, 92%, 93%, 94%, 95%, 96%, 97%, 98%, or 99% sequence identity to the sequence set forth in any one of SEQ ID NOs: 19, 25 and 26 for the induction of a haploid or a doubled haploid plant.

In one embodiment of the various aspects of the present invention, the nucleotide or amino acid sequence comprising a SAD-homology domain is selected from a sequence as set forth in any one of SEQ ID NOs: 1 to 4, 6 to 18 and 20 to 24, or a nucleotide or an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence set forth in any one of SEQ ID NOs: 1 to 4, 6 to 18 and 20 to 24.

In another embodiment of the various aspects of the present invention, the functional mutation is selected from a point mutation in the coding sequence, or in a regulatory sequence, a frameshift mutation, an insertion mutation, or a knock-out or knock-down of/in a nucleic acid sequence encoding the sequence comprising a SAD-homology domain or a regulatory sequence thereof, wherein the functional mutation results in a loss of expression or loss of transcription.

In one embodiment of the various aspects of the present invention, generating a zygote from the plant, seed, or plant cell and a wild-type plant or a plant expressing a wild-type SAD-homology domain comprising amino acid sequence yields at least 0.5%, preferably at least 1.0%, at least 2.0%; at least 3.0%, at least 4.0% at least 5.0%, at least 6.0%, at least 7.0% at least 8.0%, at least 9.0%, at least 10.0%, at least 11.0%, at least 12.0%, at least 13.0%, at least 14.0% or at least 15.0% haploid progeny.

In a further embodiment according to the various aspects of the present invention, generating a zygote from the plant, seed, or plant cell and a wild-type plant or a plant expressing a wild-type SAD-homology domain comprising amino acid sequence yields at least 0.5%, preferably at least 1.0%, at least 2.0%; at least 3.0%, at least 4.0% at least 5.0%, at least 6.0%, at least 7.0%, at least 8.0%, at least 9.0%, at least 10.0%, at least 11.0%, at least 12.0%, at least 13.0%, at least 14.0% or at least 15.0% doubled haploid progeny.

In another embodiment of the various aspects of the present invention, the plant, seed, or plant cell is a paternal and/or maternal haploid or doubled haploid inducer.

In yet another embodiment of the various aspects of the present invention, the nucleotide and/or amino acid sequence encoding or comprising the SAD-homology domain comprises at least one endogenous gene or at least one transgene.

According to another aspect, the present invention relates to a haploid plant obtained by contacting a first gamete from a plant as defined in any of the embodiments above with a second a gamete from a plant expressing an amino acid sequence comprising a wild-type SAD-homology domain to generate a zygote.

According to a further aspect, the present invention relates to a doubled haploid plant obtained by converting the haploid plant according to the aspect described above into a doubled haploid plant, preferably a spontaneous doubled haploid plant directly obtained by contacting a first gamete from a plant as defined in any of the embodiments above with a second a gamete from a plant expressing an amino acid sequence comprising a wild-type SAD-homology domain.

According to one aspect, the present invention relates to a nucleic acid sequence encoding a consensus sequence as set forth in any one of SEQ ID NOs: 19, 25 and 26, or a sequence having at least 90%, 91%, 92%, 93%, 94%, 95%, 96%, 97%, 98%, or 99% sequence identity thereto, or a nucleotide sequence or an amino acid sequence as set forth in any one of SEQ ID NOs: 1 to 4, 6 to 18 and 20 to 24, or a sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity thereto.

According to another aspect, the present invention relates to a method of generating a haploid or doubled haploid plant cell, comprising the steps of:
a) providing a first gamete from a plant having the activity of a haploid or doubled haploid inducer as defined in any of the embodiments described above;
b) contacting the first gamete from step a), preferably a male gamete, with a second gamete of a wild-type plant, or of a plant comprising a wild-type SAD-homology domain comprising amino acid sequence or a sequence encoding the same to generate a F1 zygote;
c1) obtaining a haploid cell by elimination of the chromosomes of the plant having the activity of a haploid inducer from the F1 zygote, or
c2) directly obtaining a doubled haploid F1 zygote cell.

In one embodiment the method described above comprises, in addition to steps a) to c) the following steps:
d1) growing the haploid cell under conditions to obtain a haploid plant or a part thereof; and
e1) obtaining a haploid plant or part thereof; or
d2) growing the doubled haploid cell under conditions to obtain a doubled haploid plant or a part thereof; and
e2) obtaining a doubled haploid plant or part thereof.

In one aspect, the present invention relates to a method of directly generating a doubled haploid plant cell comprising the steps of:
a) providing a first gamete from a plant having the activity of a doubled haploid inducer as defined in any of the embodiments described above,
b) contacting the first gamete from step a), preferably a male gamete, with a second gamete of a wild-type plant, or of a plant comprising a wild-type SAD-homology domain comprising amino acid sequence or a sequence encoding the same to generate a F1 zygote;
c) directly obtaining a doubled haploid cell as F1 zygote.

In one embodiment, the method of directly generating a doubled haploid plant cell described above, further comprises, in addition to steps a) to c), the following steps:
d) growing the doubled haploid cell; and
e) obtaining a doubled haploid plant, part thereof, or seed.

According to another aspect, the present invention relates to a method for identifying a plant having activity of a haploid inducer or a doubled haploid inducer comprising the step of:
a) providing a population of plants comprising a nucleotide sequence encoding an amino acid sequence comprising a SAD-homology domain;
b) screening the plant population for the presence of at least one functional mutation in the SAD-homology domain, wherein the at least one functional mutation results in a decreased or abolished expression of the amino acid sequence comprising a SAD-homology domain in comparison to the cognate wild-type amino acid sequence, wherein the functional mutation affects the expression of a SAD-homology domain consensus sequence as set forth in any one of SEQ ID NOs: 19, 25 and 26, or an amino acid sequence having at least 90%, 91%, 92%, 93%, 94%, 95%, 96%, 97%, 98%, or 99% sequence identity to the sequence set forth in any one of SEQ ID NOs: 19, 25 and 26; and
c) optionally, obtaining a plant having activity of a haploid or a doubled haploid inducer.

The present invention will now be disclosed in more detail based on the disclosure below, the non-limiting Examples as well as the attached Sequence Listing.

### Definitions

The terms "plant" or "plant cell" or "part of a plant" as used herein refer to a plant organism, a plant organ, differentiated and undifferentiated plant tissues and derivatives and progeny thereof. Plant cells include without limitation, for example, cells from seeds, from mature and immature cells or organs, including embryos, meristematic tissues, seedlings, callus tissues in different differentiation states, leaves, flowers, roots, shoots, male or female gametophytes, sporophytes, pollen, pollen tubes and microspores, protoplasts, macroalgae and microalgae. A part of a plant includes leaves, stems, roots, emerged radicles, flowers, flower parts, petals, fruits, pollen, pollen tubes, anther filaments, ovules, embryo sacs, egg cells, ovaries, zygotes, embryos, zygotic embryos, somatic embryos, apical meristems, vascular bundles, pericycles, seeds, roots, and cuttings.

A "SAD-homology domain" in the context of the present disclosure refers to amino acid sequence which comprises at least one of the consensus sequences as set forth in any one of SEQ ID NOs: 19, 25 and 26 or which comprises at least one amino acid sequence having at least 90% sequence identity to any one of the sequences set forth in SEQ ID NOs: 19, 25 and 26. In different plants, SAD-homology domains may have different names and varying degrees of homology among each other, i.e. a range from 70 to 90 % sequence identity is found. Examples for certain plants are given in SEQ ID NOs: 1 to 4, 6 to 8 and 20 to 24. In the context of the present disclosure, a SAD-homology domain comprises at least one of the consensus sequences of SEQ ID NOs: 19, 25 and 26 or a sequence having at least 90% sequence identity with one of these consensus sequences. A SAD-homology domain may also comprise two or all three of the consensus sequences of SEQ ID NOs: 19, 25 and 26 or a sequence having at least 90% sequence identity with one, two or all three of the consensus sequences. SAD-homology domains can be identified by using standard sequence search and alignment tools.

A "consensus sequence" in the context of the present disclosure refers to a sequence of nucleotide or amino acid residues, which represents the most frequent residues for each position in an alignment of sequences. The consensus sequences set forth in SEQ ID NOs: 19, 25 and 26 were the highest conserved sequences, i.e. having the highest frequency of one specific residue at each position, found in an alignment of SAD1/SAD1 sequences thus representing common identifying motifs having a common functional effect in the context of the relevant SAD1/SAD2 protein when expressed.

A "functional mutation" in the context of the present invention has an effect on the expression of the target amino acid sequence. "Functional" implies that a mutation inserted into a nucleic acid sequence has an effect on the amount of correctly transcribed and/or translated product so that the resulting protein is not, to a lesser extent, or is only present in modified form in a cell in comparison to a cell comprising no functional mutation in the respective gene and being able to express the functional wild-type protein. Thus, a functional mutation can be a loss-of-function mutation, i.e. when the expression product is not functional due to its modified, e.g. truncated, form. In particular, a functional mutation results in a decreased or abolished expression of the target sequence meaning that the target sequence is not expressed at all or is expressed in a lesser amount in a cell compared to a cell comprising no functional mutation in the respective sequence. A functional mutation can be a point mutation, and insertion, deletion or substitution of one or more nucleotides, a knock-out or knock down, a stop codon insertion or a frame-shift mutation. A functional mutation may also be present in a regulatory sequence and thus affect the expression of the target sequence.

"Expression" of a gene in the context of the present invention covers all steps involved in the conversion of the information contained in a gene or nucleic acid molecule, into a "gene product" or "expression product". A "gene product" or "expression product" can be the direct transcriptional product of a gene or nucleic acid molecule (e.g., mRNA, tRNA, rRNA, antisense RNA, ribozyme, structural RNA or any other type of RNA) or a protein produced by translation of an mRNA. The term expression thus covers transcription, including its regulation, initiation and termination as well as translation into nucleic acid sequence.

A "loss of expression" functional mutation in a protein in the context of the present invention refers to a mutation in a protein that causes loss of its native or wild-type function. Said terms thus refers to a mutation in a polynucleotide encoding a protein that causes loss-of- function of said protein. A protein may still be produced from the polynucleotide comprising the loss of expression mutation, but the protein can no longer perform its function or cannot perform its function effectively, for example because a specific point mutation was introduced into the active site of the SAD-homology protein, or because a stop codon has been introduced into the coding sequence resulting in a truncated and thus non-functional SAD-homology protein.

A "loss of transcription mutation" results in loss of expression of the mutated gene or the product encoded by said mutated gene, e.g. by inserting a mutation into the regulatory sequence of the SAD-homology protein encoding sequence, or wherein RNA interference is used, to diminish or abolish transcription of a RNA otherwise encoding a SAD-homology protein. Furthermore, said term comprises a knock-out of a SAD-homology sequence in a plant cell, seed, plant or plant material used as haploid or doubled haploid inducer.

A "regulatory sequence" in the context of the present disclosure refers to a nucleic acid sequence, which controls when and how much expression occurs of a coding region. Regulatory sequences can e.g. represent promoter regions, binding sites for transcription factors, i.e. activators, repressors.

A "knock-out" or "knock-down" of a target gene in the context of the present disclosure refers to a full or partial inactivation of the target gene, respectively. In case of a knock-down, the expression of the target gene is usually reduced, while in case of a "knock-out", no (functional) gene is expressed at all.

A "haploid plant" or "haploid plant cell" herein refers to a plant or a plant cell having only one set of chromosomes each one not being part of a pair. The number of chromosomes in a single set is called the haploid number, given the symbol n. "Gametes" are haploid cells, of which two combine in fertilization to form a "zygote" with *n* pairs of chromosomes, i.e. 2n chromosomes in total. Each chromosome pair comprises one chromosome from each gamete, called homologous chromosomes. Cells and organisms with pairs of homologous chromosomes are "diploid".

"Doubled haploid" in the context of the present disclosure refers to a genotype formed when haploid cells undergo chromosome doubling. Doubled haploids may be produced spontaneously or by chromosome doubling from haploid cells, organisms or material Therefore, doubled haploids are homozygous. For polyploid cell (2n= 3x, 4x, 5, 6x, or more), the corresponding polyhaploids may contain more than one copy of the same haploid genome.

A "plant having activity of a haploid inducer" or a "haploid inducer" or a "haploid inducer line" in the context of the present invention is a plant or plant line, which was genetically modified to have the capability to produce haploid offspring in at least 0.1%, at least 0.2%, 0.3%, 0.4%, 0.5%, 0.6%, 0.7%, 0.8%, 0.9%, preferably at least 1.0%, preferably at least 2.0%, least 3.0%, at least 4.0%, at least 5.0%, at least 6.0%, at least 7.0%, at least 8.0%, at least 9.0%, at least 10.0%, at least 11.0%, at least 12.0%, at least 13.0%, at least 14.0%, at least 15.0% of cases when combined in fertilization with a wild-type plant. Since the chromosomes of the haploid inducer are eliminated, the resulting haploid progeny only comprises genetic material of the wild-type parent.

A "plant having activity of a doubled haploid inducer" or a "doubled haploid inducer" or a "doubled haploid inducer line" in the context of the present invention is a plant or plant line, which was genetically modified to have the capability to produce doubled haploid offspring in at least 0.1%, at least 0.2%, 0.3%, 0.4%, 0.5%, 0.6%, 0.7%, 0.8%, 0.9%, preferably at least 1.0%, preferably at least 2.0%, least 3.0%, at least 4.0%, at least 5.0%, at least 6.0%, at least 7.0%, at least 8.0%, at least 9.0%, at least 10.0%, at least 11.0%, at least 12.0%, at least 13.0%, at least 14.0%, at least 15.0% of cases when combined in fertilization with a wild-type plant. The chromosomes of the doubled haploid inducer are eliminated and the resulting doubled haploid progeny only comprises genetic material of the wild-type parent. The doubled haploid progeny is formed spontaneously after fertilization and no chromosome doubling step is necessary.

A nucleic acid sequence that is "endogenous" to a cell or organism refers to a nucleic acid molecule that naturally occurs in the genome of this cell or organism. On the other hand, a nucleic acid molecule that is "exogenous" or to a cell or organism or a "transgene" refers to a nucleic acid molecule that does not naturally occur in this cell or organism but has been introduced by a transgenic event. The term transgenic event shall expressly also include at least one targeted mutation introduced into at least one allele of an endogenous gene e.g. introduced by means of gene editing.

A "gamete" in the context of the present disclosure is a haploid cell that fuses with another haploid cell in fertilization during sexual reproduction. Plants, which reproduce sexually, produce male and female gametes. A haploid inducer or doubled haploid inducer activity may be linked to a male or female gamete or it may be independent of sex. Thus, a plant can be a "paternal and/or maternal" haploid or doubled haploid inducer depending on whether contacting a male or female gamete, or either, with a wild-type gamete results in (doubled) haploid progeny.

By "contacting a first and a second gamete", a zygote is generated. However, if one gamete is derived from a plant having the activity of a haploid inducer or a doubled haploid inducer, while the other one is derived from a wild-type plant, sexual crossing, i.e. the combination of the two genomes into a diploid genome with two sets of chromosomes, is suppressed in some cases, resulting in a haploid or doubled haploid F1 zygote. This is due to chromosome elimination of the (doubled) haploid inducer and the conservation of the genome of the wild-type parent. In contrast, by sexual crossing, genes of both parents are mixed by homologous recombination in order to obtain new genetic combinations and traits. Obtaining a haploid or doubled haploid cell from the F1 zygote by elimination of the chromosomes of the haploid inducer or the doubled haploid inducer therefore does not occur by a natural process but is the result of genetic engineering of an artificial haploid inducer or doubled haploid inducer plant.

A "not naturally occurring" gamete refers to an artificial gamete, which does not occur in nature but has been genetically modified, in particular, to have the activity of a (doubled) haploid inducer.

Whenever the present disclosure relates to the percentage of identity of nucleic acid or amino acid sequences to each other these values define those values as obtained by using the EMBOSS Water Pairwise Sequence Alignments (nucleotide) program (www.ebi.ac.uk/Tools/psa/emboss_water/nucleotide.html) nucleic acids or the EMBOSS Water Pairwise Sequence Alignments (protein) program (www.ebi.ac.uk/Tools/psa/emboss_water/) for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a protein can be "codon-optimized" if the respective sequence is to be used in another organism in comparison to the original organism a molecule originates from.

In the context of the present invention, the sequence identity is to be determined with respect to the full length of the respective sequence given under a SEQ ID NO.

Sequence alignments can be conducted, for example, using the tool multiple sequence alignment Clustal Omega (EMBL-EBI; https://www.ebi.ac.uk/Tools/msa/clustalo/) using the default parameters. The default parameters are: Output format (ClustalW with character counts), dealign input sequences (no), mbed-like clustering guide-tree (yes), mbed-like clustering iteration (yes), number of combined iterations (default(0)),max guide tree iterations(default),max hmm iterations(default), order(aligned). The results were displayed, using the programme JalView (Waterhouse, A.M., Procter, J.B., Martin, D.M.A, Clamp, M. and Barton, G. J. (2009)) with shown consensus histogram and conservation histogram.

### Detailed description

The present invention relates to several aspects, which provide means and methods to achieve haploid induction or even doubled haploid induction in plant breeding. In particular, the present invention identifies a new target gene, which was not previously associated with haploid induction, and which - by functional mutation - is surprisingly able to not only facilitate haploid induction but, in a significant amount of cases, spontaneous doubled haploid progeny is formed. This obviates the need of a chromosome doubling step in order to obtain homozygous fertile offspring for further breeding.

In a first aspect, the present invention relates to the use of a plant seed, plant cell or part of a plant comprising a nucleotide sequence encoding an amino acid sequence comprising a SAD-homology domain, wherein the SAD-homology domain comprises at least one functional mutation, wherein the at least one functional mutation results in a decreased or abolished expression of the amino acid sequence comprising a SAD-homology domain in comparison to the cognate wild-type amino acid sequence, wherein the functional mutation affects the expression of a SAD-homology domain consensus sequence as set forth in any one of SEQ ID NOs: 19, 25 and 26, or an amino acid sequence having at least 90%, 91%, 92%, 93%, 94%, 95%, 96%, 97%, 98%, or 99% sequence identity to the sequence set forth in any one of SEQ ID NOs: 19, 25 and 26 for the induction of a haploid or a doubled haploid plant.

SAD proteins have not previously been associated with haploid induction. In the context of the present invention, it was surprisingly found out that by introducing a functional mutation within a SAD-homology domain in a plant, significant haploid induction rates can be achieved. Even more surprisingly, in a significant amount of cases, doubled haploid progeny is formed spontaneously, which can be used in further breeding without the need to perform a chromosome doubling step using e.g. chemical treatment with colchicine.

SAD proteins comprise a region comprising a Cse1 domain. This region ranges from position 304 to position 467 referenced to the Arabidopsis thaliana amino acid sequence (SEQ ID NO: 2). This region is higher conserved than the complete sequence (see Table 1 below). Within this region there is between position 351 and 389 a sequence which is almost identical between all SAD targets identified so far.

**Table 1: SAD2 protein sequence identities and similarities compared to Arabidopsis thaliana reference amino acid sequence of SEQ ID NO: 2**

| | Complete protein sequence | | region comprising Cse-1 domain | |
|---|---|---|---|---|
| | Identity | Similarity | Identity | Similarity |
| *B. vulgaris ssp. vulgaris* | 75.2 % | 87.6 % | 81.7 % | 91.5 % |
| *B. napus* A genome | 90.5 % | 94.5 % | 88.4 % | 94.5 % |
| *B. napus* C genome | 90.0 % | 94.3 % | 87.8 % | 94.5 % |
| *B. oleracea* | 90.2 % | 94.3 % | 89.0 % | 95.1 % |
| *C. annuum* | 76.5 % | 89.0 % | 81.1 % | 90.9 % |
| *C. sativus* | 74.7 % | 88.2 % | 82.3 % | 91.5 % |
| *G. max* | 72.6 % | 85.6 % | 80.5 % | 90.9 % |
| *H. annuus* homolog 1 | 74.4 % | 86.6 % | 79.3 % | 89.0 % |
| *H. annuus* homolog 2 | 74.1 % | 86.7 % | 79.9 % | 88.4 % |
| *H. annuus* homolog 3 | 74.2 % | 86.7 % | 76.8 % | 88.4 % |
| *S. lycopersicum* | 75.6 % | 87.9 % | 80.5 % | 91.5% |
| *S. bicolor* | 70.2 % | 84.8 % | 74.4 % | 90.2 % |
| *Z. mays* | 70.2 % | 85.6 % | 73.2 % | 90.2 % |

As indicated above, a Cse1 domain was described to be essential for the function of SAD1/SAD2. However, Cse1 domains are widely distributed over proteins in different taxa fulfilling different functions. A targeted analysis to identify a possible contribution of selected positions within a Cse1 domain in SAD1/SAD2 proteins in the context of the herein observed and studied effect of (doubled) haploid induction has not been conducted so far. Therefore, three highly conserved consensus sequences have been identified within the Cse1 domain (SEQ ID NOs: 19, 25 and 26), which represent core regions of interest for the effects studied herein and which can serve as basis for transforming the relevant teaching to other SAD1/SAD2 homologous sequences in various target plants.

Notably, in SEQ ID NO: 19, the positions shown as Xaa may be any naturally occurring amino acid.

In SEQ ID NO: 25, Xaa can be any naturally occurring amino acid. In a preferred embodiment, Xaa at position (6) can be any naturally occurring amino acid selected from the group of amino acids containing carboxylic chains, for example glutamic acid and aspartic acid. In another preferred embodiment, Xaa at position (11) can be any naturally occurring amino acid selected from the group consisting of amino acids with aliphatic side chains, for example valine, leucine and isoleucine. In yet another preferred embodiment, Xaa at position (19) can be any naturally occurring amino acid selected from the group of amino acids with aliphatic hydroxyl side chains and cysteine. In another preferred embodiment, Xaa at position (21) can be any naturally occurring amino acid selected from the group of small amino acids and amino acids with aliphatic hydroxyl side chains. In yet another preferred embodiment, Xaa at position (71) and Xaa at position (73) can be any naturally occurring amino acid selected from the group of aromatic amino acids, for example phenylalanine and tyrosine. In another preferred embodiment, Xaa at position (78) can be any naturally occurring amino acid selected from the group consisting of amino acids with aliphatic hydroxyl side chains and carboxamide side chains, for example asparagine and serine and Xaa at position (81) can be any naturally occurring amino acid selected from the group consisting of small amino acids and amino acids with hydroxyl side chains, for example alanine and threonine.

It was further deduced that in SEQ ID NO: 26, Xaa can be any naturally occurring amino acid. In a preferred embodiment, Xaa at position (6) can be any naturally occurring amino acid selected from the group consisting of amino acids with aliphatic side chains, for example valine, leucine and isoleucine. In another preferred embodiment, Xaa at position (13) can be any naturally occurring amino acid selected from the group consisting of amino acids with aliphatic side chains and proline. In yet another preferred embodiment, Xaa at position (14) can be any naturally occurring amino acid selected from the group of amino acids with carboxylic side chains, for example glutamic acid and aspartic acid. In another preferred embodiment, Xaa at position (20) can be any naturally occurring amino acid selected from the group of small amino acids, for example glycine and alanine. In yet another preferred embodiment, Xaa at position (28) can be any naturally occurring amino acid selected from the group consisting of amino acids with aliphatic side chains. In another preferred embodiment, Xaa at position (37) can be any naturally occurring amino acid selected from the group consisting of amino acids with aliphatic hydroxyl side chains. In yet another preferred embodiment, Xaa at position (40) can be any naturally amino acid selected from the group consisting of small amino acids.

Based on the various embodiments and aspects disclosed herein, comparable consensus sequences can be identified in a plant germplasm of interest. The relevant information on the genotype as present in a germplasm of interest can then be used to either introduce a targeted mutation or a transgene, preferably in all relevant alleles, to obtain a plant material suitable as haploid or doubled haploid inducer as further detailed herein below for subsequent breeding efforts to rapidly speed up product development cycles for obtaining plants carrying agronomically favorable traits.

The mechanism of doubled haploid induction remains to be elucidated. However, it is assumed that it is related to the disturbed function of SAD2 as β-importin (Zhao et al. (2007). SAD2, an importin β-like protein, is required for UV-B response in Arabidopsis by mediating MYB4 nuclear trafficking. The Plant Cell, 19(11), 3805-3818.) during embryogenesis and reduced stress tolerance (Verslues *et al.,* 2006) or disturbed function of micro RNA pathway (Wang et al., (2011). An importin β protein negatively regulates microRNA activity in Arabidopsis. The Plant Cell, 23(10), 3565-3576.). In the Arabidopsis genome, second homologs of SAD2 gene have been identified, designated as a SAD1 gene (At3g59020), which can be used for dihaploid production too.

In a preferred embodiment of the use, all alleles and homologs encoding a SAD-homology domain present in the plant carry functional mutations as described above. Thus, in a preferred embodiment, the plant is homozygous for the functional mutation.

A functional mutation in the SAD-homology domain as required for (doubled) haploid induction may be a point mutation but it may also disrupt the SAD-homology domain e.g. by insertion or deletion of multiple nucleotides so that in the plant used according to the invention, the consensus sequences defined above may no longer be present. However, before the introduction of the functional mutation, at least one of the consensus sequences must have been present to define a SAD-homology domain. SAD-homology domains can be identified by sequence search and alignment. As described in detail below, several SAD-homology domains have been found in different crop plants as presented by SEQ ID NOs: 1 to 4, 6 to 18 and 20 to 24. Using the same methods as described herein, a skilled person can identify more SAD-homology domains accordingly.

In one embodiment, the nucleotide or amino acid sequence comprising a SAD-homology domain is selected from a sequence as set forth in any one of SEQ ID NOs: 1 to 4, 6 to 18 and 20 to 24, or a nucleotide or an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence set forth in any one of SEQ ID Nos: 1 to 4, 6 to 18 and 20 to 24.

A functional mutation has an effect on the amount of correctly transcribed and/or translated product so that the resulting protein is not present, to a lesser extent, or is only present in modified form in a cell in comparison to a cell comprising no functional mutation in the respective gene and being able to express the functional wild-type protein.

In one embodiment, the functional mutation is selected from a point mutation in the coding sequence, or in a regulatory sequence, a frameshift mutation, an insertion mutation, or a knock-out or knock-down of/in a nucleic acid sequence encoding the sequence comprising a SAD-homology domain or a regulatory sequence thereof, wherein the functional mutation results in a loss of expression or loss of transcription.

A functional mutation can e.g. be a point mutation, an insertion, deletion or substitution of one or more nucleotides in an active site of the protein, a knock-out or knock-down, a stop codon insertion or a frame-shift mutation. A functional mutation may also be present in a regulatory sequence and thus affect the expression of the SAD-homology domain consensus sequence. A skilled person is aware of a number of techniques for the introduction of such mutations. The mutations can e.g. be introduced by random mutagenesis, in particular chemical mutagenesis, preferably via EMS (ethylmethane sulfonate)-induced or ENU (N-ethyl-N-nitrosourea)-induced TILLING or by targeted mutagenesis, preferably by means of meganucleases, Zinc Finger nucleases, TALENs or CRISPR/Cas such as CRISPR/Cas9 or CRISPR/Cpf1, or by means of base editor systems. For a site-directed modification, genome editing tools, i.e. site-specific effectors such as nucleases, nickases, recombinases, transposases, base editors are available. These effectors have the capacity to introduce a single- or double-strand cleavage into a genomic target site, or have the capacity to introduce a targeted modification, including a point mutation, an insertion, or a deletion, into a genomic target site of interest. A double strand break can then repaired by non-homologous end-joining or homology-directed repair, e.g. using a repair template. In a preferred embodiment, T-DNA insertion by *Agrobacterium* is used to introduce the mutation.

Gametes of plants carrying an above described mutation provide haploid induction rates of up to 15% when forming a zygote with a gamete expressing the wild-type SAD-homology domain. The genome of the plant carrying the mutation is eliminated so that the haploid progeny exclusively carries genomic material from the wild-type parent.

Preferred is an embodiment of the use described above, wherein generating a zygote from the plant, seed, or plant cell and a wild-type plant or a plant expressing a wild-type SAD-homology domain comprising amino acid sequence yields at least 0.5%, preferably at least 1.0%, at least 2.0%; at least 3.0%, at least 4.0% at least 5.0%, at least 6.0%, at least 7.0% at least 8.0%, at least 9.0%, at least 10.0%, at least 11.0%, at least 12.0%, at least 13.0%, at least 14.0% or at least 15.0% haploid progeny.

It is particularly surprising that, in a significant amount of cases, when a gamete of plants carrying an above described mutation forms a zygote with a gamete expressing the wild-type SAD-homology domain, doubled haploid offspring is formed directly, which is homozygous for the wild-type genome.

In a preferred embodiment of the use described above, generating a zygote from the plant, seed, or plant cell and a wild-type plant or a plant expressing a wild-type SAD-homology domain comprising amino acid sequence yields at least 0.5%, preferably at least 1.0%, at least 2.0%; at least 3.0%, at least 4.0% at least 5.0%, at least 6.0%, at least 7.0%, at least 8.0%, at least 9.0%, at least 10.0%, at least 11.0%, at least 12.0%, at least 13.0%, at least 14.0% or at least 15.0% doubled haploid progeny.

In some cases, a mixture of haploid and doubled haploid offspring is obtained. Surprising is in particular the large portion of spontaneously formed doubled haploid progeny.

The gamete of the plant carrying an above described mutation may be the male (i.e. pollen) or female gamete (i.e. egg cell /ovule) in forming a zygote to generate haploid or doubled haploid progeny. Thus, the haploid or doubled haploid inducer activity may be paternal or maternal.

In one embodiment of the use described above, the plant, seed, or plant cell is a paternal and/or maternal haploid or doubled haploid inducer.

In a preferred embodiment, the gamete from the plant, seed, or plant cell carrying the functional mutation in the SAD-homology domain is a male gamete and the plant, seed, or plant cell is therefore a paternal haploid or doubled haploid inducer.

In one embodiment of the use described above, the nucleotide and/or amino acid sequence encoding or comprising the SAD-homology domain comprises at least one endogenous gene or at least one transgene.

It is possible to obtain the haploid or doubled haploid inducer activity by either introducing the at least one mutation in an endogenous nucleotide sequence or introducing the sequence as a transgene. Thus, a completely transgene-free approach may be chosen or a transgenic plant according to the invention may be provided. This can be effected by inserting a desired mutation based on the findings on conserved SAD1/SAD2 consensus sequences as identified herein, for example, by using a transient genome editing technique to introduce a desired mutation into at least one, preferably all, alleles of a *sad1* and/or *sad2* gene of interest in a germplasm of a plant of interest.

When a transgenic plant having the activity of a (doubled) haploid inducer is used in a method for obtaining a (doubled) haploid plant as described herein, the genome of the haploid inducer is eliminated resulting in a transgene-free (doubled) haploid plant. Therefore, non-transgenic haploid plants can be provided, which is advantageous due to the regulatory limitations imposed on transgenic plants.

The plant seed, plant cell or part of a plant used according to the invention in any of the embodiments described above may be derived from any agronomically important crop plants carrying a SAD-homology domain.

In particular, the plant seed, plant cell or part of a plant may be derived from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

In a further embodiment of the use described above, the cell or part of the plant is selected from the group consisting of leaves, stems, roots, emerged radicles, flowers, flower parts, petals, fruits, pollen, pollen tubes, anther filaments, ovules, embryo sacs, egg cells, ovaries, zygotes, embryos, zygotic embryos, somatic embryos, apical meristems, vascular bundles, pericycles, seeds, roots, and cuttings.

According to another aspect, the present invention relates to a haploid plant obtained by contacting a first gamete from a plant as described above with a second a gamete from a plant expressing an amino acid sequence comprising a wild-type SAD-homology domain to generate a zygote.

Haploid offspring is obtained in a significant amount of cases when a gamete from a plant carrying a functional mutation in the SAD-homology domain is contacted with a gamete from a plant expressing an amino acid sequence comprising a wild-type SAD-homology domain to generate a zygote. The genome of the plant carrying the functional mutation has been eliminated in the haploid offspring, so that the haploid plant only contains genetic material from the plant expressing an amino acid sequence comprising a wild-type SAD-homology domain. The plant is therefore not genetically modified. Subsequently, the haploid plant can be subjected to a chromosome doubling, e.g. by colchicine treatment, to generate a doubled haploid plant being homozygous for a desired trait. This plant can be further used in breeding protocols.

According to a further aspect, the present invention relates to a doubled haploid plant obtained by converting the haploid plant described above into a doubled haploid plant, preferably a spontaneous doubled haploid plant directly obtained by contacting a first gamete from a plant as defined in any of the embodiments described above with a second a gamete from a plant expressing an amino acid sequence comprising a wild-type SAD-homology domain.

A homozygous doubled haploid plant can be obtained by subjecting the haploid plant described above to chromosome doubling, e.g. by colchicine treatment. Advantageously, however, doubled haploid plants are also formed spontaneously obviating the chromosome doubling step and the associated additional effort and handling of toxic substances. The doubled haploid plant exclusively comprises genetic material from the parent expressing the wild-type SAD-homology domain as the genome of the SAD-homology domain mutant has been eliminated. The doubled haploid plant is therefore homozygous for desired traits of the wild-type parent, not genetically modified and can directly be used in further breeding protocols.

The haploid or doubled haploid plant according to the aspects described above may be selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

According to a further aspect, the present invention also relates to a nucleic acid sequence encoding a consensus sequence as set forth in any one of SEQ ID NOs: 19, 25 and 26, or a sequence having at least 90%, 91%, 92%, 93%, 94%, 95%, 96%, 97%, 98%, or 99% sequence identity thereto, or a nucleotide sequence or an amino acid sequence as set forth in any one of SEQ ID NOs: 1 to 4, 6 to 18 and 20 to 24, or a sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity thereto.

The consensus sequences of SEQ ID NOs: 19, 25 and 26, represent highly conserved motifs identified in the SAD-homology domains of different plants represented by SEQ ID NOs: 1 to 4, 6 to 18 and 20 to 24. They therefore represent the targets for a functional mutation resulting in the haploid or doubled haploid induction activity according to the present invention. SAD-homology domains, e.g. as represented by SEQ ID NOs: 1 to 4, 6 to 18 and 20 to 24, have not previously been associated with haploid induction. Thus, it was surprising that a functional mutation in this domain is capable of conferring to a plant the activity of a haploid inducer. Even more surprising is the fact that such mutations also confer to a plant the activity of a doubled haploid inducer.

Haploid induction for obtaining plant cells containing only the chromosome set found after meiosis in male (sperm cells) or female (egg cells) gametes is a relevant process in plant breeding. The numerous methods to obtain haploid plants for different cultivars of commercial relevance can be roughly classified into two categories. Firstly, *in vitro* methods are based on the culture of haploid cells and their differentiation into haploid embryos and ultimately haploid plants. Both male (microspores or pollen) and female haploid cells (megaspores or ovules) are used, depending on the responsiveness of the cells in a given species. Secondly, *in situ* methods make use of particular pollination techniques using irradiated pollen, inter-specific crosses or so-called 'inducer lines'. The skilled person is well aware of suitable techniques for in vitro or in vivo haploid induction in relevant plants, and particular crop, species like in *Zea mays,* see for example, Prigge und Melchinger, Production of Haploids and Doubled Haploids in Maize, Plant Cell Culture Protocols (2012).

As it is known to the skilled person, doubled haploids can be created when a haploid (n) plant undergoes genome doubling due to the exposure to a doubling agent or mitotic inhibitor, the most common chromosome doubling agent being colchicine. This doubling results in genetically uniform diploid offspring with two identical genomes (2n). Further chromosome doubling techniques are well-known to the skilled person for various crops.

For *Zea mays,* a common method of producing haploid plants is *in vivo* maternal haploid induction requiring a specific inducer genotype. When an inducer is crossed to a maize donor plant, progeny will segregate into diploid (2n) and haploid (n) classes. Further methods are disclosed in, for example, Vanous et al., Generation of maize (Zea may) doubled haploids via traditional methods, Current Protocols in n Plant Biology: 147-157, 2017. Various protocols are available to the skilled person for doubled haploid generation for other relevant plant species.

According to yet another aspect, the present invention relates to a method of generating a haploid or doubled haploid plant cell, comprising the steps of:
a) providing a first gamete, preferably a not naturally occurring first gamete, from a plant having the activity of a haploid or doubled haploid inducer as defined in any of the embodiments above;
b) contacting the first gamete from step a), preferably a male gamete, with a second gamete of a wild-type plant, or of a plant comprising a wild-type SAD-homology domain comprising amino acid sequence or a sequence encoding the same to generate a F1 zygote;
c1) obtaining a haploid cell by elimination of the chromosomes of the plant having the activity of a haploid inducer from the F1 zygote, or
c2) directly obtaining a doubled haploid F1 zygote cell.

In step a) of the method, a first gamete is provided, which is derived from a plant comprising a nucleotide sequence encoding an amino acid sequence comprising a SAD-homology domain, wherein the SAD-homology domain comprises at least one functional mutation.

The first gamete is preferably a not naturally occurring gamete, because it has been genetically modified to have haploid or doubled haploid inducer activity. The plant from which the gamete is derived has been obtained by introducing at least one functional mutation, which results in a decreased or abolished expression of the amino acid sequence comprising a SAD-homology domain in comparison to the cognate wild-type amino acid sequence. The functional mutation affects the expression of a SAD-homology domain consensus sequence as set forth in any one of SEQ ID NOs: 19, 25 and 26, or an amino acid sequence having at least 90%, 91%, 92%, 93%, 94%, 95%, 96%, 97%, 98%, or 99% sequence identity to the sequence set forth in any one of SEQ ID NOs: 19, 25 and 26.

In an embodiment of the method described above, the plant from which the first gamete is derived, is the female parent, and the plant from which the second gamete is derived, is the male parent, or the plant from which the first gamete is derived is the male parent, and the plant from which the second gamete is derived, is the female parent. Preferably, the plant from which the first gamete is derived, is the male parent, and the plant from which the second gamete derived, is the female parent.

Contacting the first gamete with the second gamete in step b) results in the formation of an F1 zygote, from which in a certain amount of cases, the chromosomes of the first gamete are eliminated and no sexual crossing of genomes occurs. Furthermore, in a certain amount of cases, the chromosomes of the second gamete double spontaneously. Thus, haploid and/or doubled haploid offspring is obtained in step c1) and/or c2). The haploid and/or doubled haploid offspring is the result of genetic engineering of an artificial haploid or doubled haploid inducer plant and the step of selectively contacting a gamete from such a plant with a gamete from a wild-type plant or a plant expressing wild-type SAD-homology domain. The haploid or doubled haploid progeny is therefore not formed by processes occurring in nature. The haploid and/or doubled haploid cell obtained in step c1) or c2) only comprises genetic material from the second gamete and thus does not comprise the genetic modification of the first gamete, which confers the haploid or doubled haploid inducer activity.

In one embodiment, the haploid cell obtained in step c1) may be subjected to a step of chromosome doubling, e.g. by colchicine treatment.

The doubled haploid cell obtained in step c2) is homozygous and can directly be propagated.

In one embodiment of the method described above, the amino acid sequence comprising a SAD-homology domain is selected from an amino acid sequence as set forth in any one of SEQ ID NOs: 1 to 4, 6 to 18 and 20 to 24, or an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence set forth in any one of SEQ ID NOs: 1 to 4, 6 to 18 and 20 to 24.

The haploid or doubled haploid plant obtained in step c1) and/or c2) may be selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

In one embodiment of the method described above, the functional mutation is selected from a point mutation in the coding sequence, or in a regulatory sequence, a frameshift mutation, an insertion mutation, or a knock-out or knock-down of/in a nucleic acid sequence encoding the sequence comprising a SAD-homology domain or a regulatory sequence thereof, wherein the functional mutation results in a loss of expression or loss of transcription.

In another embodiment of the method described above, contacting the first and second gamete in step b) yields at least 0.5%, preferably at least 1.0%, at least 2.0%; at least 3.0%, at least 4.0% at least 5.0%, at least 6.0%, at least 7.0% at least 8.0%, at least 9.0%, at least 10.0%, at least 11.0%, at least 12.0%, at least 13.0%, at least 14.0% or at least 15.0% haploid progeny in step c1) and/or yields at least 0.5%, preferably at least 1.0%, at least 2.0%; at least 3.0%, at least 4.0% at least 5.0%, at least 6.0%, at least 7.0%, at least 8.0%, at least 9.0%, at least 10.0%, at least 11.0%, at least 12.0%, at least 13.0%, at least 14.0% or at least 15.0% doubled haploid progeny in step c2).

In one embodiment, the method described above comprises in addition to steps a) to c) the following steps:
d1) growing the haploid cell under conditions to obtain a haploid plant or a part thereof; and
e1) obtaining a haploid plant or part thereof; or
d2) growing the doubled haploid cell under conditions to obtain a doubled haploid plant or a part thereof; and
e2) obtaining a doubled haploid plant or part thereof.

The method described above produces doubled haploid plant lines, which can be used in breeding for efficiently developing desirable traits. Doubled haploid lines can also be used as parents in hybrid production as explained in further detail below.

In one aspect, the present invention relates to a method of directly generating a doubled haploid plant cell comprising the steps of:
a) providing a first gamete, preferably a not naturally occurring first gamete, from a plant having the activity of a doubled haploid inducer as defined in any of the embodiments above,
b) contacting the first gamete from step a), preferably a male gamete, with a second gamete of a wild-type plant, or of a plant comprising a wild-type SAD-homology domain comprising amino acid sequence or a sequence encoding the same to generate a F1 zygote;
c) directly obtaining a doubled haploid cell as F1 zygote.

Surprisingly, in a certain amount of cases, the chromosomes of the second gamete double spontaneously when the chromosomes of the first gamete are eliminated. The doubled haploid cell obtained in step c) only comprises genetic material from the second gamete and thus does not comprise the genetic modification of the first gamete, which confers the haploid or doubled haploid inducing activity. The doubled haploid cell obtained in step c) is homozygous and can directly be propagated.

The first gamete is preferably a not naturally occurring gamete, because it has been genetically modified to have haploid or doubled haploid inducer activity. The plant from which the gamete is derived has been obtained by introducing at least one functional mutation, which results in a decreased or abolished expression of the amino acid sequence comprising a SAD-homology domain in comparison to the cognate wild-type amino acid sequence. The functional mutation affects the expression of a SAD-homology domain consensus sequence as set forth in any one of SEQ ID NOs: 19, 25 and 26, or an amino acid sequence having at least 90%, 91%, 92%, 93%, 94%, 95%, 96%, 97%, 98%, or 99% sequence identity to the sequence set forth in any one of SEQ ID NOs: 19, 25 and 26.

In an embodiment of the method described above, the plant from which the first gamete is derived, is the female parent, and the plant from which the second gamete is derived, is the male parent, or the plant from which the first gamete is derived, is the male parent, and the plant from which the second gamete is derived, is the female parent. Preferably, the plant from which the first gamete is derived, is the male parent, and the plant from which the second gamete is derived, is the female parent.

In one embodiment of the method described above, the amino acid sequence comprising a SAD-homology domain is selected from an amino acid sequence as set forth in any one of SEQ ID NOs: 1 to 4, 6 to 18 and 20 to 24, or an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence set forth in any one of SEQ ID NOs: 1 to 4, 6 to 18 and 20 to 24.

The doubled haploid plant obtained in step c) may be selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

In one embodiment of the method described above, the functional mutation is selected from a point mutation in the coding sequence, or in a regulatory sequence, a frameshift mutation, an insertion mutation, or a knock-out or knock-down of/in a nucleic acid sequence encoding the sequence comprising a SAD-homology domain or a regulatory sequence thereof, wherein the functional mutation results in a loss of expression or loss of transcription.

In another embodiment of the method described above, contacting the first and second gamete in step b) yields at least 0.5%, preferably at least 1.0%, at least 2.0%; at least 3.0%, at least 4.0% at least 5.0%, at least 6.0%, at least 7.0%, at least 8.0%, at least 9.0%, at least 10.0%, at least 11.0%, at least 12.0%, at least 13.0%, at least 14.0% or at least 15.0% doubled haploid progeny in step c2).

In one embodiment of the method of generating a doubled haploid plant, plant material or seed described above, the method further comprises, in addition to steps a) to c) according to claim 13, the following steps:
d) growing the doubled haploid cell; and
e) obtaining a doubled haploid plant, part thereof, or seed.

The methods described herein produce (doubled) haploid plant lines, which are highly useful in breeding protocols for efficiently developing desirable traits. Doubled haploid lines can be directly used as parents in hybrid production. In hybrid breeding, two different inbred lines are crossed with the aim of obtaining superior traits compared to either of the parents. The inbred lines are genetically uniform due to generations of self-crossing. Using the methods according to the invention, however, genetically uniform parents can be produced in just one generation, even without a chromosome doubling step. This greatly expedites and facilitates the production of hybrid plant lines with superior characteristics.

According to another aspect, the present invention relates to a method for identifying a plant having activity of a haploid inducer or a doubled haploid inducer comprising the step of:
a) providing a population of plants comprising a nucleotide sequence encoding an amino acid sequence comprising a SAD-homology domain;
b) screening the plant population for the presence of at least one functional mutation in the SAD-homology domain, wherein the at least one functional mutation results in a decreased or abolished expression of the amino acid sequence comprising a SAD-homology domain in comparison to the cognate wild-type amino acid sequence, wherein the functional mutation affects the expression of a SAD-homology domain consensus sequence as set forth in any one of SEQ ID NOs: 19, 25 and 26, or an amino acid sequence having at least 90%, 91%, 92%, 93%, 94%, 95%, 96%, 97%, 98%, or 99% sequence identity to the sequence set forth in any one of SEQ ID NOs: 19, 25 and 26; and
c) optionally, obtaining a plant having activity of a haploid or a doubled haploid inducer.

The population of plants provided in step a) can be a naturally occurring population or it can be obtained by performing mutagenesis. For example, T-DNA insertion by *Agrobacterium,* ethyl methanesulfonate (EMS) mutagenesis or ENU (N-ethyl-N-nitrosourea)-induced TILLING can be used to provide SAD-homology domain mutants conferring haploid or doubled haploid inducer activity.

The screening step b) can be performed by various techniques available to the skilled person. For example, phenotype screening using certain markers, PCR methods involving specific primers and probes, deep sequencing and comparison with wild-type sequences can be employed. The skilled person is well aware of how to design primers to identify mutations in a specific target sequence.

In a preferred embodiment, all alleles and homologs encoding a SAD-homology domain present in the plant identified and optionally obtained in step c) carry functional mutations as described above. Thus, in a preferred embodiment, the plant is homozygous for the functional mutation.

In one embodiment of the method for identifying a plant having activity of a haploid inducer or a doubled haploid inducer described above, the nucleotide or amino acid sequence comprising a SAD-homology domain is selected from a sequence as set forth in any one of SEQ ID NOs: 1 to 4, 6 to 18 and 20 to 24, or a nucleotide or an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence set forth in any one of SEQ ID Nos: 1 to 4, 6 to 18 and 20 to 24.

In another embodiment of the method for identifying a plant having activity of a haploid inducer or a doubled haploid inducer described above, the functional mutation is selected from a point mutation in the coding sequence, or in a regulatory sequence, a frameshift mutation, an insertion mutation, or a knock-out or knock-down of/in a nucleic acid sequence encoding the sequence comprising a SAD-homology domain or a regulatory sequence thereof, wherein the functional mutation results in a loss of expression or loss of transcription.

In a further embodiment of the method for identifying a plant having activity of a haploid inducer or a doubled haploid inducer described above, generating a zygote from the plant, seed, or plant cell and a wild-type plant or a plant expressing a wild-type SAD-homology domain comprising amino acid sequence yields at least 0.5%, preferably at least 1.0%, at least 2.0%; at least 3.0%, at least 4.0% at least 5.0%, at least 6.0%, at least 7.0%, at least 8.0%, at least 9.0%, at least 10.0%, at least 11.0%, at least 12.0%, at least 13.0%, at least 14.0% or at least 15.0% haploid and/or doubled haploid progeny.

The identified plant, optionally obtained in step c) may be a paternal and/or a maternal haploid or doubled haploid inducer. Preferably, the identified plant is a paternal haploid or doubled haploid inducer.

The plant obtained in step c) can be used for haploid or doubled haploid induction in the context of any embodiment of the uses and methods for generating haploid or doubled haploid plants described above.

A functional mutation in the SAD-homology domain identified in the screening step b) can be introduced in a targeted manner into any plant having a SAD-homology domain to produce a plant having haploid or doubled haploid inducer activity. Methods and tools are available to the skilled person to introduce a targeted mutation in a SAD-homology domain, which results in a decreased or abolished expression of the amino acid sequence comprising a SAD-homology domain in comparison to the cognate wild-type amino acid sequence, resulting in the production of a plant or part of a plant having haploid and/or doubled haploid inducer activity. In particular, a targeted mutation is directed at modifying the expression of any one of the SAD-homology domain consensus sequence as set forth in any one of SEQ ID NOs: 19, 25 and 26. For a site-directed modification of the identified target sequence, genome editing tools such as site-specific effectors, e.g. nucleases, nickases, recombinases, transposases, base editors are available. These effectors can introduce a single- or double-strand cleavage into a genomic target site, or a targeted modification, including a point mutation, an insertion, or a deletion, into a genomic target site of interest. A double strand break can then repaired by non-homologous end-joining or homology-directed repair, e.g. using a repair template. Thus, one a plant having activity of a haploid inducer or a doubled haploid inducer can be produced.

Based on the above disclosure, plant material having the function of a haploid or even doubled haploid inducer can thus be rapidly identified and/or introduced into a germplasm of interest to drastically enhance breeding efforts.

The present invention will now be described in more detail based on the enclosed non-limiting Examples.

### Examples

### Example 1

T-DNA mutants of Arabidopsis thaliana were analyzed for their ability to induce the generation of haploids in crosses with wild-type. This screening resulted in the selection of a few candidate genes, whereby one of these genes is SAD2 (AT2G31660). It was found that pollination of the trichomeless *glabra1 A. thaliana* mutant (gl1) with pollen of the SAD2 (Sensitive to ABA and Drought 2) T-DNA insertion mutant (WiscDsLox477-480P7; https://www.arabidopsis.org/servlets/TairObject?type=germplasm&name=WiscDsLox477-480P7) resulted in 3-15% doubled haploid seedlings which only carried the maternal genome. The gl1 mutant had served as a tool for preselecting potential haploid or doubled haploid progeny.

To eliminate potential self-pollination products from double haploids, ecotype-specific PCR-markers were used for genotyping of F1 seedlings (*glabra1* mutant was generated in Ler-0 ecotype and the SAD2 T-DNA mutants in Col-0).

### WiscDsLox477-480P7:

Sequence tagged T-DNA insertion line, generated by vacuum infiltration of Columbia (Col) plants with Agrobacterium tumefaciens vector WiscDs-Lox; Basta herbicide was employed for selection of plants carrying a T-DNA; each T1 transformant has been maintained individually. This line can be utilized as in Ds Launchpad experiments; the T-DNA includes a Ds transposon/launchpad construction so that transposition can be induced by crossing to an Ac line; selection of resulting transposants is by Basta and hygromycin; LoxP sites are also present so that regions between transposed Ds elements and the empty donor site can be subsequently removed by crossing to a Cre line (Woody, Austin-Phillips, Amasino, Krysan: "The WiscDsLox T-DNA collection: an Arabidopsis community resource generated by using an improved high-throughput T-DNA sequencing pipeline", J. Plant Res., 2007, 120(1): 157-65).

**Insertion Flanking Sequence of** WiscDsLox477-480P7 is represented by the sequence set forth in SEQ ID NO: 5.

Further suitable mutants were found at http://www.arabidopsis.org/ under the given designations.

### Example 2 - Determination of consensus sequences

Originating from our experimental data, other SAD homology proteins in various crop species were searched using the BLAST-N from NCBI at the U.S. National Library of Medicine using an expect threshold of 10, a word size of 28 and Match/Mismatch Scores of 1 and -2 with linear gap costs. The found sequences were taken for pairwise alignment to search for conserved regions. Multi Sequence Alignment by Clustal Omega was done with no dealigned input sequences, an MBED-like clustering guide-tree and a MBED-like clustering iteration. The number of combined iterations, the maximum guide tree iterations and the maximum HMM iterations were set on default. The data thus obtained can be immediately analyzed by MView (version 1.65). From this bioinformatical alignment, consensus sequences can be concluded as all aligned sequences show highly conserved regions. With regard to possible functional motifs and domains the consensus sequences can be determined by selecting these highly conserved regions which show only a small variation in amino acid sequence and an optional conserved functional domain such as a peptide binding site. The deriving consensus sequences from this alignment are SEQ ID NO: 19, SEQ ID NO: 25 and SEQ ID NO: 26. These *in silico* generated consensus motifs built the basis for additional experimental work.

## Claims

1. Use of a plant seed, plant cell or part of a plant comprising a nucleotide sequence encoding an amino acid sequence comprising a SAD-homology domain, wherein the SAD-homology domain comprises at least one functional mutation, wherein the at least one functional mutation results in a decreased or abolished expression of the amino acid sequence comprising a SAD-homology domain in comparison to the cognate wild-type amino acid sequence, wherein the functional mutation affects the expression of a SAD-homology domain consensus sequence as set forth in any one of SEQ ID NOs: 19, 25 and 26, or an amino acid sequence having at least 90%, 91%, 92%, 93%, 94%, 95%, 96%, 97%, 98%, or 99% sequence identity to the sequence set forth in any one of SEQ ID NOs: 19, 25 and 26 for the induction of a haploid or a doubled haploid plant.

2. The use according to claim 1, wherein the nucleotide or amino acid sequence comprising a SAD-homology domain is selected from a sequence as set forth in any one of SEQ ID NOs: 1 to 4, 6 to 18 and 20 to 24, or a nucleotide or an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence set forth in any one of SEQ ID NOs1 to 4, 6 to 18 and 20 to 24.

3. The use according to claim 1 or 2, wherein the functional mutation is selected from a point mutation in the coding sequence, or in a regulatory sequence, a frameshift mutation, an insertion mutation, or a knock-out or knock-down of/in a nucleic acid sequence encoding the sequence comprising a SAD-homology domain or a regulatory sequence thereof, wherein the functional mutation results in a loss of expression or loss of transcription.

4. The use according to any of the preceding claims, wherein generating a zygote from the plant, seed, or plant cell and a wild type plant or a plant expressing a wild-type SAD-homology domain comprising amino acid sequence yields at least 0.5%, preferably at least 1.0%, at least 2.0%; at least 3.0%, at least 4.0% at least 5.0%, at least 6.0%, at least 7.0% at least 8.0%, at least 9.0%, at least 10.0%, at least 11.0%, at least 12.0%, at least 13.0%, at least 14.0% or at least 15.0% haploid progeny.

5. The use according to any of the preceding claims, wherein generating a zygote from the plant, seed, or plant cell and a wild type plant or a plant expressing a wild-type SAD-homology domain comprising amino acid sequence yields at least 0.5%, preferably at least 1.0%, at least 2.0%; at least 3.0%, at least 4.0% at least 5.0%, at least 6.0%, at least 7.0%, at least 8.0%, at least 9.0%, at least 10.0%, at least 11.0%, at least 12.0%, at least 13.0%, at least 14.0% or at least 15.0% doubled haploid progeny.

6. The use according to any of the preceding claims, wherein the plant, seed, or plant cell is a paternal and/or maternal haploid or doubled haploid inducer.

7. The use according to any of the preceding claims, wherein the nucleotide and/or amino acid sequence encoding or comprising the SAD-homology domain comprises at least one endogenous gene or at least one transgene.

8. A haploid plant obtained by contacting a first gamete from a plant as defined in any of claims 1 to 7 with a second a gamete from a plant expressing an amino acid sequence comprising a wild-type SAD-homology domain to generate a zygote.

9. A doubled haploid plant obtained by converting the haploid plant according to claim 8 into a doubled haploid plant, preferably a spontaneous doubled haploid plant directly obtained by contacting a first gamete from a plant as defined in any of claims 1 to 7 with a second a gamete from a plant expressing an amino acid sequence comprising a wild-type SAD-homology domain.

10. A nucleic acid sequence encoding a consensus sequence as defined in claim 1, or a sequence having at least 90%, 91%, 92%, 93%, 94%, 95%, 96%, 97%, 98%, or 99% sequence identity thereto, or a nucleotide sequence or an amino acid sequence as defined in claim 2, or a sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity thereto.

11. A method of generating a haploid or doubled haploid plant cell, comprising the steps of:
a) providing a first gamete from a plant having the activity of a haploid or doubled haploid inducer as defined in any of claims 1 to 7;
b) contacting the first gamete from step a), preferably a male gamete, with a second gamete of a wild-type plant, or of a plant comprising a wild-type SAD-homology domain comprising amino acid sequence or a sequence encoding the same to generate a F1 zygote;
c1) obtaining a haploid cell by elimination of the chromosomes of the plant having the activity of a haploid inducer from the F1 zygote, or
c2) directly obtaining a doubled haploid F1 zygote cell.

12. The method of generating a haploid plant according to claim 11, wherein the method comprises, in addition to steps a) to c) the following steps:
d1) growing the haploid cell under conditions to obtain a haploid plant or a part thereof; and
e1) obtaining a haploid plant or part thereof; or
d2) growing the doubled haploid cell under conditions to obtain a doubled haploid plant or a part thereof; and
e2) obtaining a doubled haploid plant or part thereof.

13. A method of directly generating a doubled haploid plant cell comprising the steps of:
a) providing a first gamete from a plant having the activity of a doubled haploid inducer as defined in any of claims 1 to 7,
b) contacting the first gamete from step a), preferably a male gamete, with a second gamete of a wild-type plant, or of a plant comprising a wild-type SAD-homology domain comprising amino acid sequence or a sequence encoding the same to generate a F1 zygote;
c) directly obtaining a doubled haploid cell as F1 zygote.

14. A method of generating a doubled haploid plant, plant material or seed, wherein the method further comprises, in addition to steps a) to c) according to claim 13, the following steps:
d) growing the doubled haploid cell; and
e) obtaining a doubled haploid plant, part thereof, or seed.

15. Method for identifying a plant having activity of a haploid inducer or a doubled haploid inducer comprising the step of:
a) providing a population of plants comprising a nucleotide sequence encoding an amino acid sequence comprising a SAD-homology domain;
b) screening the plant population for the presence of at least one functional mutation in the SAD-homology domain, wherein the at least one functional mutation results in a decreased or abolished expression of the amino acid sequence comprising a SAD-homology domain in comparison to the cognate wild-type amino acid sequence, wherein the functional mutation affects the expression of a SAD-homology domain consensus sequence as set forth in any one of SEQ ID NOs: 19, 25 and 26, or an amino acid sequence having at least 90%, 91%, 92%, 93%, 94%, 95%, 96%, 97%, 98%, or 99% sequence identity to the sequence set forth in any one of SEQ ID NOs: 19, 25 and 26; and
c) optionally, obtaining a plant having activity of a haploid or a doubled haploid inducer.


SEQUENCE LISTING

<110>  Leibniz Institut für Pflanzengenetik und
       Kulturpflanzenforschung Gatersleben IPK

<120>  Generation of haploids based on manipulation of SAD2

<130>  207438

<160>  26

<170>  PatentIn version 3.5

<210>  1
<211>  3123
<212>  DNA
<213>  Artificial Sequence

<220>
<223>  cDNA SAD2 of Arabidopsis thaliana

<400>  1
atggatctgc atagcctcgc tttgatcctc cgaaccgccg ctcttagccc tattcccgat      60

gagcgtaaag tttctgagca gcaactcaat cagttggagc acacgcccca gcatttggtg     120

aggttattgc agatagccgt ggatggaaat tgtgacatgg cagtgcgtca aattgctagt     180

attcagttca aaaatttaat tgctaagaac tggtcacctg aggattgtgg tcctgcagta     240

aggcagcagc agatatttga gagtgacaaa gaattggtga gggacaatat tctcgtctat     300

gtcacccaag ttccaacctt actcagatca caacttggag agtctctcaa gacaattatt     360

tatgctgact acccagaaca atggccacgt cttctggatt gggtgaagta caacttgcaa     420

aatcaacaaa tttatggggc tttgtttgtg ttgcggatac tctctagaaa atatgagttc     480

aagtcagatg aggagaggac accggtttcc cgcattgtgg aggagacatt tcctcaactt     540

ttgactattt ttaatgggct tatccagata ccaaatccgt ctctggagat agcagaactt     600

atgaagttga tatgcaaaat attttggtca tccatatatc tggagcttcc aaggcaatta     660

tttgatctaa acgtctttaa tgcctggatg gttctgttct tgagtgtttc ggaacgtcct     720

gttccagttg aaggtcagcc tatggatccg gaacttagaa aatcttgggg ctggtggaag     780

gtcaagaagt ggacagtgca cattttaaac aggctctata gtcggtttgg agatccaaaa     840

cttcaaagtc cagaaaacaa acctttcgcc caaatgtttc aaaagaatta tgcaggcaga     900

attttggaag gccacctaaa tttcttgaac acaattcgtg ttggaggcta tcttcctgac     960

agagttatca atcttctcct tcagtactta agcaacagca tttcgaagaa tagcatgtac    1020

aagttgcttc tgcctcggct ggatgttctg ctttttgaga ttgttttccc tctaatgtgc    1080

tttaatgata atgatcaaaa gctttgggag gaagaccctc atgaatatgt gaggaaaggt    1140

tacaatatca ttgaagactt gtacagtccg cgaacagcgt ccatggattt tgtaaatgag    1200

ttggttcgaa aacgtgggaa agaaaacctt ccgaagtttg ttaagtttgt cgttgagatc    1260

ttcttgagtt atgaaaaagc tactgtagag gagaagcctt atcgccaaaa agatggtgcg 1320

atgctggctg ttggagctct ttgtgacaaa ttgaagcaaa cggatcccta taaatctcaa 1380

ctagagctaa tgttggtgca acatattttt cctgatttca atagtccagt tggacatctt 1440

agagcaaagg ctgcatgggt agctgggcaa tatgcacaca tcaatttctc agatcagaac 1500

aactttcgta aagcattgca cagtgttgtt tcgggactgc gcgatcctga tctccctgtt 1560

cgtgttgatt cagtttttgc attgcgttca tttgttgagg cgtgcaagga tttaaatgag 1620

atacgtccaa tcctccctca gttacttgat gaatttttca aactcatgaa tgaggtagag 1680

aatgaggacc ttgtttttac tttggagacc atcgtagata agtttggcga agagatggct 1740

ccttttgctt ttggattatg tcaaaatctg gcggctgcgt tttggagatg tctaaacacg 1800

tcagaagcca atgacgactc tgatgacatg ggagctttag ctgccgttgg ttgtttgcgt 1860

gccataagta caatccttga atctgttagc agtctcccgc agctgtttgt tgagatagaa 1920

ccaactatac ttccaataat gcagaaaatg ttgaccactg atggccaaga ggtatttgaa 1980

gaagttttgg agattgcatc atacatgacc ttttattcac ctagcatttc cttggatata 2040

tggagtctct ggcctttaat ggtggaagca ttggttgatt ggggaatcga tttctttcca 2100

aatattttgg ttccaatgga caactttata tcaaggggaa cggctcattt tctcacttgc 2160

aaagagcccg actatcagca aagtctatat aacgttcttt caactcttat gaccgacaga 2220

aacatagaag acagtgaaat tgagtctgct ccaaagctta ttgaagttgt tttccagaat 2280

tgcaaagggc aggtggatca gtgggttgaa ccatatctgc gtctcactgt tgaccggtta 2340

caacgggctg agacttcgta tgttaaatct cttcttatac aagtggtggc aaatatgctt 2400

tactacaatc caggtttgac gcttggtgta ttgcataaca caggacttgc ttccaaagtc 2460

tttgaccttt ggttccagat gttgcagcaa aagagaaaaa gtggcctgcc agcaaacttc 2520

aaaagggaac atgataaaaa ggtttgctgc ttgggtttga cttcattact tgctctccct 2580

gggggtcaat tccccgatga agcgctgcag cgtgttttca gggcaacact tgatcttcta 2640

gttgcatata agaatcagct agctgaagca gctaaggaga cagaagtaga ttatgaagaa 2700

gaaatgaatg gactccagag tagtgatgat gattatgatg atgatgggtc tgatggggag 2760

atggatgata cagaggaagg agatgaagca caaagtgtta aactgcagaa gttagctgca 2820

caggcaaagg ccttccacta tgatgatgat gatgacgacg attctgatga tgactttagt 2880

gatgaggacg agtttcagtc acccatcgat gaggtggatg cctttgtatt ctttgtcgat 2940

gcaatcagag ttatgcaggc atcagatgcg cagaggtttc agaacctgaa ccagtcactg 3000

gacttcactt accaggcgat tgcaaacgga atagcacagc atgcagagct gagaagagtg 3060

gagatcgaga aggagaagca aaagaagtta gcagctgctt ctactcctgt tactgctcta 3120

tga                                                                  3123

<210>  2
<211>  1040
<212>  PRT
<213>  Arabidopsis thaliana

<400>  2

Met Asp Leu His Ser Leu Ala Leu Ile Leu Arg Thr Ala Ala Leu Ser
1               5                   10                  15

Pro Ile Pro Asp Glu Arg Lys Val Ser Glu Gln Gln Leu Asn Gln Leu
            20                  25                  30

Glu His Thr Pro Gln His Leu Val Arg Leu Leu Gln Ile Ala Val Asp
        35                  40                  45

Gly Asn Cys Asp Met Ala Val Arg Gln Ile Ala Ser Ile Gln Phe Lys
    50                  55                  60

Asn Leu Ile Ala Lys Asn Trp Ser Pro Glu Asp Cys Gly Pro Ala Val
65                  70                  75                  80

Arg Gln Gln Gln Ile Phe Glu Ser Asp Lys Glu Leu Val Arg Asp Asn
                85                  90                  95

Ile Leu Val Tyr Val Thr Gln Val Pro Thr Leu Leu Arg Ser Gln Leu
            100                 105                 110

Gly Glu Ser Leu Lys Thr Ile Ile Tyr Ala Asp Tyr Pro Glu Gln Trp
        115                 120                 125

Pro Arg Leu Leu Asp Trp Val Lys Tyr Asn Leu Gln Asn Gln Gln Ile
    130                 135                 140

Tyr Gly Ala Leu Phe Val Leu Arg Ile Leu Ser Arg Lys Tyr Glu Phe
145                 150                 155                 160

Lys Ser Asp Glu Glu Arg Thr Pro Val Ser Arg Ile Val Glu Glu Thr
                165                 170                 175

Phe Pro Gln Leu Leu Thr Ile Phe Asn Gly Leu Ile Gln Ile Pro Asn
            180                 185                 190

Pro Ser Leu Glu Ile Ala Glu Leu Met Lys Leu Ile Cys Lys Ile Phe
        195                 200                 205

Trp Ser Ser Ile Tyr Leu Glu Leu Pro Arg Gln Leu Phe Asp Leu Asn

210                 215                 220

Val Phe Asn Ala Trp Met Val Leu Phe Leu Ser Val Ser Glu Arg Pro
225                 230                 235                 240

Val Pro Val Glu Gly Gln Pro Met Asp Pro Glu Leu Arg Lys Ser Trp
                245                 250                 255

Gly Trp Trp Lys Val Lys Lys Trp Thr Val His Ile Leu Asn Arg Leu
            260                 265                 270

Tyr Ser Arg Phe Gly Asp Pro Lys Leu Gln Ser Pro Glu Asn Lys Pro
        275                 280                 285

Phe Ala Gln Met Phe Gln Lys Asn Tyr Ala Gly Arg Ile Leu Glu Gly
    290                 295                 300

His Leu Asn Phe Leu Asn Thr Ile Arg Val Gly Gly Tyr Leu Pro Asp
305                 310                 315                 320

Arg Val Ile Asn Leu Leu Leu Gln Tyr Leu Ser Asn Ser Ile Ser Lys
                325                 330                 335

Asn Ser Met Tyr Lys Leu Leu Leu Pro Arg Leu Asp Val Leu Leu Phe
            340                 345                 350

Glu Ile Val Phe Pro Leu Met Cys Phe Asn Asp Asn Asp Gln Lys Leu
        355                 360                 365

Trp Glu Glu Asp Pro His Glu Tyr Val Arg Lys Gly Tyr Asn Ile Ile
    370                 375                 380

Glu Asp Leu Tyr Ser Pro Arg Thr Ala Ser Met Asp Phe Val Asn Glu
385                 390                 395                 400

Leu Val Arg Lys Arg Gly Lys Glu Asn Leu Pro Lys Phe Val Lys Phe
                405                 410                 415

Val Val Glu Ile Phe Leu Ser Tyr Glu Lys Ala Thr Val Glu Glu Lys
            420                 425                 430

Pro Tyr Arg Gln Lys Asp Gly Ala Met Leu Ala Val Gly Ala Leu Cys
        435                 440                 445

Asp Lys Leu Lys Gln Thr Asp Pro Tyr Lys Ser Gln Leu Glu Leu Met
    450                 455                 460

Leu Val Gln His Ile Phe Pro Asp Phe Asn Ser Pro Val Gly His Leu
465                 470                 475                 480

Arg Ala Lys Ala Ala Trp Val Ala Gly Gln Tyr Ala His Ile Asn Phe
                485                 490                 495

Ser Asp Gln Asn Asn Phe Arg Lys Ala Leu His Ser Val Val Ser Gly
            500                 505                 510

Leu Arg Asp Pro Asp Leu Pro Val Arg Val Asp Ser Val Phe Ala Leu
        515                 520                 525

Arg Ser Phe Val Glu Ala Cys Lys Asp Leu Asn Glu Ile Arg Pro Ile
    530                 535                 540

Leu Pro Gln Leu Leu Asp Glu Phe Phe Lys Leu Met Asn Glu Val Glu
545                 550                 555                 560

Asn Glu Asp Leu Val Phe Thr Leu Glu Thr Ile Val Asp Lys Phe Gly
                565                 570                 575

Glu Glu Met Ala Pro Phe Ala Phe Gly Leu Cys Gln Asn Leu Ala Ala
            580                 585                 590

Ala Phe Trp Arg Cys Leu Asn Thr Ser Glu Ala Asn Asp Asp Ser Asp
        595                 600                 605

Asp Met Gly Ala Leu Ala Ala Val Gly Cys Leu Arg Ala Ile Ser Thr
    610                 615                 620

Ile Leu Glu Ser Val Ser Ser Leu Pro Gln Leu Phe Val Glu Ile Glu
625                 630                 635                 640

Pro Thr Ile Leu Pro Ile Met Gln Lys Met Leu Thr Thr Asp Gly Gln
                645                 650                 655

Glu Val Phe Glu Glu Val Leu Glu Ile Ala Ser Tyr Met Thr Phe Tyr
            660                 665                 670

Ser Pro Ser Ile Ser Leu Asp Ile Trp Ser Leu Trp Pro Leu Met Val
        675                 680                 685

Glu Ala Leu Val Asp Trp Gly Ile Asp Phe Phe Pro Asn Ile Leu Val
    690                 695                 700

Pro Met Asp Asn Phe Ile Ser Arg Gly Thr Ala His Phe Leu Thr Cys
705                 710                 715                 720

Lys Glu Pro Asp Tyr Gln Gln Ser Leu Tyr Asn Val Leu Ser Thr Leu
                725                 730                 735

Met Thr Asp Arg Asn Ile Glu Asp Ser Glu Ile Glu Ser Ala Pro Lys
            740                 745                 750

Leu Ile Glu Val Val Phe Gln Asn Cys Lys Gly Gln Val Asp Gln Trp
        755                 760                 765

Val Glu Pro Tyr Leu Arg Leu Thr Val Asp Arg Leu Gln Arg Ala Glu
    770                 775                 780

Thr Ser Tyr Val Lys Ser Leu Leu Ile Gln Val Val Ala Asn Met Leu
785                 790                 795                 800

Tyr Tyr Asn Pro Gly Leu Thr Leu Gly Val Leu His Asn Thr Gly Leu
                805                 810                 815

Ala Ser Lys Val Phe Asp Leu Trp Phe Gln Met Leu Gln Gln Lys Arg
            820                 825                 830

Lys Ser Gly Leu Pro Ala Asn Phe Lys Arg Glu His Asp Lys Lys Val
        835                 840                 845

Cys Cys Leu Gly Leu Thr Ser Leu Leu Ala Leu Pro Gly Gly Gln Phe
    850                 855                 860

Pro Asp Glu Ala Leu Gln Arg Val Phe Arg Ala Thr Leu Asp Leu Leu
865                 870                 875                 880

Val Ala Tyr Lys Asn Gln Leu Ala Glu Ala Ala Lys Glu Thr Glu Val
                885                 890                 895

Asp Tyr Glu Glu Glu Met Asn Gly Leu Gln Ser Ser Asp Asp Asp Tyr
            900                 905                 910

Asp Asp Asp Gly Ser Asp Gly Glu Met Asp Asp Thr Glu Glu Gly Asp
        915                 920                 925

Glu Ala Gln Ser Val Lys Leu Gln Lys Leu Ala Ala Gln Ala Lys Ala
    930                 935                 940

Phe His Tyr Asp Asp Asp Asp Asp Asp Asp Ser Asp Asp Asp Phe Ser
945                 950                 955                 960

Asp Glu Asp Glu Phe Gln Ser Pro Ile Asp Glu Val Asp Ala Phe Val
                965                 970                 975

Phe Phe Val Asp Ala Ile Arg Val Met Gln Ala Ser Asp Ala Gln Arg
            980                 985                 990

Phe Gln Asn Leu Asn Gln Ser Leu  Asp Phe Thr Tyr Gln  Ala Ile Ala
        995                 1000                 1005

Asn Gly  Ile Ala Gln His Ala  Glu Leu Arg Arg Val  Glu Ile Glu
    1010                 1015                 1020

Lys Glu  Lys Gln Lys Lys Leu  Ala Ala Ala Ser Thr  Pro Val Thr
    1025                 1030                 1035

Ala Leu
    1040

<210>  3
<211>  3093
<212>  DNA
<213>  Artificial Sequence

<220>
<223>  cDNA SAD1 of Arabidopsis thaliana

<400>  3
atggatctgc ctagcctcgc tttaatcgtt ggcgcggctg ctttcagtcc caatcctgac      60

gagcgcagag ctgctgagca gagccttaat cagttgcagc acactccaca gcatttgata     120

aggatattgc aaataattgt tgatggtggc tctgatcttt ctgtacgcca atctgctagt     180

atccatttca agaatttcat tgctaagcac tgggaacctc attctggtga ccagaacata     240

atcttgccaa gtgataagaa tgttgtcagg aaccagattc ttgtatttgt ctctcaagtg     300

cccccgattt tgagagttca gatgggagag tgcctgaaga ccataattta cgctgactat     360

cctgagcaat ggcccgaact cctggactgg gtgaagcaaa acttgcaaaa accacaagtt     420

tatggagcgc tatttgtctt gaggatacta tcttcaaaat acgagtttaa gtcagatgaa     480

gatagggcac ccattcaccg agtagtcgag gagacttttc cccacctgtt gaacatattt     540

aacaatcttg tccatgttga aaatccttct ctcgaagtgg cagaccatat caagcttatt     600

tgcaagatat tctggtcatg tatttatcta gagcttccga gaccattgtt tgatccaaac     660

ttttttaatg cttggatggg tctgttccta aacattttgg aacgtcctgt tcccgtagaa     720

ggccagcctg aagaccctga gcttagaaaa tcctggggat ggtggaaggc caagaagtgg     780

attgctcata tcttaaacag gctatacact cggtttggag atctgaaact ccagaatcca     840

gataacaaag cctttgctca aatgttccag ataaactatg cagccaagat attagagtgc     900

cacctgaaat tgttgaatgc cattcgtata ggtggatatc ttccggatag agtcatcaac     960

ctaattcttc aatatctaag caacagtatc tcgaagagta gcatgtacaa cttactgcag 1020

ccccacctta acactctcct atttgaaata gttttcccac ttatgtgttt caatgataat 1080

gatcaaatgc tttgggatga agacccgcat gagtatgtaa ggaagggtta tgatataatt 1140

gaagatttat acagtcccag gactgcgtct atggactttg tgactgagct ggtcagaaag 1200

cgtggaaaag aaaacttccc aaaatttata cagttcgttg tggacatttt taagagatat 1260

aatgaagcat ctctggaaaa caagccttat cgccttaagg atggtgctct gcttgctgtt 1320

gggacacttt gtgataaact taggcaaact gaaccctaca aatctgagct ggagaatatg 1380

ttagtgcaac atgtttttcc tgaatttagc agcccagctg gtcatcttag agctaaggct 1440

gcgtgggttg cagggcagta cgccaatatc gatttctcag accagagcaa tttttcaaag 1500

gcattgcatt gtgttatctc cggaatgtgt gatctagaac ttcctgttcg ggtggattca 1560

gtttttgctt tgcgttcatt tattgaagcc tgcaaagatt tagatgaaat ccgcccagtt 1620

cttcctcaac ttcttgatga atttttcaag ctaatgaagg aagttgaaaa cgaagacctt 1680

gcttttacat tagagactat tgtgtataag tttggagagg agatttctcc gtatgctttg 1740

ggtttgtgcc aaaacttggc cagtgctttc tggaggtgta tcgacactga caacggtgat 1800

gatgaaactg atgatgctgg tgcattggct gcagtgggat gtctccgtgc catcagtacc 1860

attcttgaat ctatcagtag tctccctcat ctttatggtc agattgaacc acagttactg 1920

ccaataatgc ggaaaatgct gacaactgat ggtcaagatg tgtttgaaga ggttctggag 1980

atcgtctcat atattactac tttttcacct acaatatcat tggagatgtg gagtctatgg 2040

cctttaatga tggaagcact cgtggattgg gctattgact ttttcccaaa tatattggtc 2100

ccgttgcaca actatatatc cagggggact gggcattatc ttacttgcaa ggaaccagat 2160

taccagcaaa atctttggaa tgtgatatct gtgcttatgg cgaataaaaa tattgatgac 2220

agtgacctcg agccagctcc gaagctatta gggatagtgc ttcagacatg caagggccag 2280

gtggatcaat gggtagaacc ctacctgaga atcacacttg atcggctacg cggtgctgag 2340

aaatcttcct tcaagtgcct tctggttgaa gtggttgcaa atgcctttta ctacaatacc 2400

cctttggcac ttggcatact gcaacgattt ggtatcgcaa cagaaatttt tactctatgg 2460

ttccaaatgc tgcaagaaaa gaagaagagt ggtgcacgtt ctaacttcaa gagagaacat 2520

gataagaaag tttgcatctt ggggttgaca tcacttttca gccttccagc tggtcaattg 2580

cctggagagg tcttgccaca tgtattcagg gctcttctcg agcttttagt ggcatacaag 2640

gatcaacttg ctgaagcggc aaaggcagaa gaggaagaag aagatgaaga tggtgatgat 2700

gacgacatgg atgaattcca aacagatgat gaagatgagg acggcgatga cgagaatcca 2760

gatgaaactg atggaagtac actccgcaag ttagcagcac aggcaaagga cttccgctct 2820

tacagcgatg atgatgactt ttcagacgat gattttagcg atgacgagga attagaatct 2880

ccaattgatg aagtggatcc tttcgtactc ttcatggatg cagtcacagc aatgcaagta 2940

tcagactcgc caaggttcca aagccttacc cagacactag acccgcacta ccatggactc 3000

gcaagcacaa tagctcaaca cacggagctg agaagagcag agattctaaa ggagaaactt 3060

gagaagcaat cttctgcaac ggttgcttcc tga 3093

<210> 4
<211> 1030
<212> PRT
<213> Arabidopsis thaliana

<400> 4

Met Asp Leu Pro Ser Leu Ala Leu Ile Val Gly Ala Ala Ala Phe Ser
1               5                   10                  15

Pro Asn Pro Asp Glu Arg Arg Ala Ala Glu Gln Ser Leu Asn Gln Leu
            20                  25                  30

Gln His Thr Pro Gln His Leu Ile Arg Ile Leu Gln Ile Ile Val Asp
        35                  40                  45

Gly Gly Ser Asp Leu Ser Val Arg Gln Ser Ala Ser Ile His Phe Lys
    50                  55                  60

Asn Phe Ile Ala Lys His Trp Glu Pro His Ser Gly Asp Gln Asn Ile
65                  70                  75                  80

Ile Leu Pro Ser Asp Lys Asn Val Val Arg Asn Gln Ile Leu Val Phe
                85                  90                  95

Val Ser Gln Val Pro Pro Ile Leu Arg Val Gln Met Gly Glu Cys Leu
            100                 105                 110

Lys Thr Ile Ile Tyr Ala Asp Tyr Pro Glu Gln Trp Pro Glu Leu Leu
        115                 120                 125

Asp Trp Val Lys Gln Asn Leu Gln Lys Pro Gln Val Tyr Gly Ala Leu
    130                 135                 140

Phe Val Leu Arg Ile Leu Ser Ser Lys Tyr Glu Phe Lys Ser Asp Glu
145                 150                 155                 160

Asp Arg Ala Pro Ile His Arg Val Val Glu Glu Thr Phe Pro His Leu
                165                 170                 175

Leu Asn Ile Phe Asn Asn Leu Val His Val Glu Asn Pro Ser Leu Glu
            180                 185                 190

Val Ala Asp His Ile Lys Leu Ile Cys Lys Ile Phe Trp Ser Cys Ile
        195                 200                 205

Tyr Leu Glu Leu Pro Arg Pro Leu Phe Asp Pro Asn Phe Phe Asn Ala
    210                 215                 220

Trp Met Gly Leu Phe Leu Asn Ile Leu Glu Arg Pro Val Pro Val Glu
225                 230                 235                 240

Gly Gln Pro Glu Asp Pro Glu Leu Arg Lys Ser Trp Gly Trp Trp Lys
                245                 250                 255

Ala Lys Lys Trp Ile Ala His Ile Leu Asn Arg Leu Tyr Thr Arg Phe
            260                 265                 270

Gly Asp Leu Lys Leu Gln Asn Pro Asp Asn Lys Ala Phe Ala Gln Met
        275                 280                 285

Phe Gln Ile Asn Tyr Ala Ala Lys Ile Leu Glu Cys His Leu Lys Leu
    290                 295                 300

Leu Asn Ala Ile Arg Ile Gly Gly Tyr Leu Pro Asp Arg Val Ile Asn
305                 310                 315                 320

Leu Ile Leu Gln Tyr Leu Ser Asn Ser Ile Ser Lys Ser Ser Met Tyr
                325                 330                 335

Asn Leu Leu Gln Pro His Leu Asn Thr Leu Leu Phe Glu Ile Val Phe
            340                 345                 350

Pro Leu Met Cys Phe Asn Asp Asn Asp Gln Met Leu Trp Asp Glu Asp
        355                 360                 365

Pro His Glu Tyr Val Arg Lys Gly Tyr Asp Ile Ile Glu Asp Leu Tyr
    370                 375                 380

Ser Pro Arg Thr Ala Ser Met Asp Phe Val Thr Glu Leu Val Arg Lys
385                 390                 395                 400

Arg Gly Lys Glu Asn Phe Pro Lys Phe Ile Gln Phe Val Val Asp Ile
                405                 410                 415

Phe Lys Arg Tyr Asn Glu Ala Ser Leu Glu Asn Lys Pro Tyr Arg Leu
            420                 425                 430

Lys Asp Gly Ala Leu Leu Ala Val Gly Thr Leu Cys Asp Lys Leu Arg

435                 440                 445

Gln Thr Glu Pro Tyr Lys Ser Glu Leu Glu Asn Met Leu Val Gln His
    450                 455                 460

Val Phe Pro Glu Phe Ser Ser Pro Ala Gly His Leu Arg Ala Lys Ala
465                 470                 475                 480

Ala Trp Val Ala Gly Gln Tyr Ala Asn Ile Asp Phe Ser Asp Gln Ser
                485                 490                 495

Asn Phe Ser Lys Ala Leu His Cys Val Ile Ser Gly Met Cys Asp Leu
            500                 505                 510

Glu Leu Pro Val Arg Val Asp Ser Val Phe Ala Leu Arg Ser Phe Ile
        515                 520                 525

Glu Ala Cys Lys Asp Leu Asp Glu Ile Arg Pro Val Leu Pro Gln Leu
    530                 535                 540

Leu Asp Glu Phe Phe Lys Leu Met Lys Glu Val Glu Asn Glu Asp Leu
545                 550                 555                 560

Ala Phe Thr Leu Glu Thr Ile Val Tyr Lys Phe Gly Glu Glu Ile Ser
                565                 570                 575

Pro Tyr Ala Leu Gly Leu Cys Gln Asn Leu Ala Ser Ala Phe Trp Arg
            580                 585                 590

Cys Ile Asp Thr Asp Asn Gly Asp Asp Glu Thr Asp Asp Ala Gly Ala
        595                 600                 605

Leu Ala Ala Val Gly Cys Leu Arg Ala Ile Ser Thr Ile Leu Glu Ser
    610                 615                 620

Ile Ser Ser Leu Pro His Leu Tyr Gly Gln Ile Glu Pro Gln Leu Leu
625                 630                 635                 640

Pro Ile Met Arg Lys Met Leu Thr Thr Asp Gly Gln Asp Val Phe Glu
                645                 650                 655

Glu Val Leu Glu Ile Val Ser Tyr Ile Thr Thr Phe Ser Pro Thr Ile
            660                 665                 670

Ser Leu Glu Met Trp Ser Leu Trp Pro Leu Met Met Glu Ala Leu Val
        675                 680                 685

Asp Trp Ala Ile Asp Phe Phe Pro Asn Ile Leu Val Pro Leu His Asn
    690                 695                 700

Tyr Ile Ser Arg Gly Thr Gly His Tyr Leu Thr Cys Lys Glu Pro Asp
705                 710                 715                 720

Tyr Gln Gln Asn Leu Trp Asn Val Ile Ser Val Leu Met Ala Asn Lys
                725                 730                 735

Asn Ile Asp Asp Ser Asp Leu Glu Pro Ala Pro Lys Leu Leu Gly Ile
            740                 745                 750

Val Leu Gln Thr Cys Lys Gly Gln Val Asp Gln Trp Val Glu Pro Tyr
        755                 760                 765

Leu Arg Ile Thr Leu Asp Arg Leu Arg Gly Ala Glu Lys Ser Ser Phe
    770                 775                 780

Lys Cys Leu Leu Val Glu Val Val Ala Asn Ala Phe Tyr Tyr Asn Thr
785                 790                 795                 800

Pro Leu Ala Leu Gly Ile Leu Gln Arg Phe Gly Ile Ala Thr Glu Ile
                805                 810                 815

Phe Thr Leu Trp Phe Gln Met Leu Gln Glu Lys Lys Lys Ser Gly Ala
            820                 825                 830

Arg Ser Asn Phe Lys Arg Glu His Asp Lys Lys Val Cys Ile Leu Gly
        835                 840                 845

Leu Thr Ser Leu Phe Ser Leu Pro Ala Gly Gln Leu Pro Gly Glu Val
    850                 855                 860

Leu Pro His Val Phe Arg Ala Leu Leu Glu Leu Leu Val Ala Tyr Lys
865                 870                 875                 880

Asp Gln Leu Ala Glu Ala Ala Lys Ala Glu Glu Glu Glu Glu Asp Glu
                885                 890                 895

Asp Gly Asp Asp Asp Asp Met Asp Glu Phe Gln Thr Asp Asp Glu Asp
            900                 905                 910

Glu Asp Gly Asp Asp Glu Asn Pro Asp Glu Thr Asp Gly Ser Thr Leu
        915                 920                 925

Arg Lys Leu Ala Ala Gln Ala Lys Asp Phe Arg Ser Tyr Ser Asp Asp
    930                 935                 940

Asp Asp Phe Ser Asp Asp Asp Phe Ser Asp Asp Glu Glu Leu Glu Ser
945                 950                 955                 960

Pro Ile Asp Glu Val Asp Pro Phe Val Leu Phe Met Asp Ala Val Thr
                965                 970                 975

Ala Met Gln Val Ser Asp Ser Pro Arg Phe Gln Ser Leu Thr Gln Thr
            980                 985                 990

Leu Asp Pro His Tyr His Gly Leu  Ala Ser Thr Ile Ala  Gln His Thr
        995                 1000                1005

Glu Leu  Arg Arg Ala Glu Ile  Leu Lys Glu Lys Leu  Glu Lys Gln
    1010                1015                1020

Ser Ser  Ala Thr Val Ala Ser
    1025                1030

<210>  5
<211>  755
<212>  DNA
<213>  Artificial Sequence

<220>
<223>  Insertion Flanking Sequence of WiscDsLox477-480P7

<220>
<221>  misc_feature
<222>  (5)..(5)
<223>  n is a, c, g, or t

<220>
<221>  misc_feature
<222>  (13)..(13)
<223>  n is a, c, g, or t

<220>
<221>  misc_feature
<222>  (40)..(40)
<223>  n is a, c, g, or t

<220>
<221>  misc_feature
<222>  (58)..(58)
<223>  n is a, c, g, or t

<220>
<221>  misc_feature
<222>  (64)..(64)
<223>  n is a, c, g, or t

<220>
<221>  misc_feature
<222>  (106)..(106)
<223>  n is a, c, g, or t

<220>
<221>  misc_feature
<222>  (129)..(129)
<223>  n is a, c, g, or t

<220>
<221>  misc_feature
<222>  (134)..(134)
<223>  n is a, c, g, or t

<220>
<221>  misc_feature
<222>  (136)..(136)
<223>  n is a, c, g, or t

<220>
<221>  misc_feature
<222>  (154)..(154)
<223>  n is a, c, g, or t

<220>
<221>  misc_feature
<222>  (157)..(157)
<223>  n is a, c, g, or t

<220>
<221>  misc_feature
<222>  (159)..(161)
<223>  n is a, c, g, or t

<220>
<221>  misc_feature
<222>  (165)..(165)
<223>  n is a, c, g, or t

<220>
<221>  misc_feature
<222>  (175)..(175)
<223>  n is a, c, g, or t

<220>
<221>  misc_feature
<222>  (184)..(184)
<223>  n is a, c, g, or t

<220>
<221>  misc_feature
<222>  (187)..(187)
<223>  n is a, c, g, or t

<220>
<221>  misc_feature
<222>  (189)..(189)
<223>  n is a, c, g, or t

<220>
<221>  misc_feature
<222>  (194)..(194)
<223>  n is a, c, g, or t

<220>
<221>  misc_feature

<222>  (208)..(208)
<223>  n is a, c, g, or t

<220>
<221>  misc_feature
<222>  (212)..(212)
<223>  n is a, c, g, or t

<220>
<221>  misc_feature
<222>  (222)..(222)
<223>  n is a, c, g, or t

<400>  5
cttgngttgg cgnaaaggaa cacgaaactc gacatatccn aatgccgaca accaaatncc      60

gacnagcaaa tgccacatgc gaatgccgaa cacgatactc gagtancgtc cgcaatgtgt      120

tatgtaagnt gtcntnacct ttacttgagt tggnatntnn ngaantccgg atccntaagc      180

tganctncna ctgnaacagg acgttccnaa anactcaaga anagaaccat ccaggcatta      240

aagacgttta gatcaaatag ttgccttgga agctccagct gcccacagag aacagtaaaa      300

aagacattaa acagcttaac tggtttgaat tgtgttttaa ggatatgcat attatgttaa      360

gaaagataaa atgtgaaatt ccgcagaggc atatgtaact gatctctcta gcaacttaca      420

tatatggatg accaaaatat tttgcatatc aacttcataa gttctgctat ctccagagac      480

ggatttggta tctggataag cccattaaaa atagtcaaaa gttgaggaaa tgtctcctcc      540

acaatgcggg aaaccggtgt cctctcctca tctgacttga acctagaaaa taaaatcatg      600

aaacatcatc aacgggatat actacttagg tgtcatcata taaagagcaa cttgcatttc      660

tagctggaag tcgtcacata cttgacagga catcatgaat tgacagagac acgaaaacca      720

ttgtgttcta acaatacgtt ttaaacactc tcatt                                 755


<210>  6
<211>  1032
<212>  PRT
<213>  Beta vulgaris

<400>  6

Met Asp Leu Pro Ser Leu Ala Val Ile Leu Arg Ala Ala Leu Ser Pro
1               5                   10                  15


Asn Pro Asp Glu Arg Lys Ala Gly Glu Glu Ser Leu Asn Gln Phe Gln
            20                  25                  30


Tyr Thr Pro Gln His Thr Val Arg Leu Leu Gln Ile Ile Val Asp Gly
        35                  40                  45


Asn Cys Asp Met Ala Val Arg Gln Val Ala Ser Ile His Phe Lys Asn
    50                  55                  60

Phe Ile Ala Lys Asn Trp Ser Pro His Asp Pro Glu Glu Gln Ser Lys
65                  70                  75                  80

Ile Ala Pro Ala Asp Lys Asp Met Val Arg Glu Asn Ile Leu Val Phe
                85                  90                  95

Val Ala Gln Val Pro Thr Leu Leu Arg Val Gln Leu Gly Glu Cys Leu
            100                 105                 110

Lys Thr Ile Ile His Thr Asp Tyr Pro Glu Gln Trp Pro Gly Leu Leu
        115                 120                 125

His Trp Val Lys His Asn Leu Gln Asp Gln Gln Val Tyr Gly Ala Leu
    130                 135                 140

Phe Val Leu Arg Ile Leu Ala Arg Lys Tyr Glu Phe Lys Ser Asp Asp
145                 150                 155                 160

Glu Arg Thr Pro Val His Leu Ile Val Glu Glu Thr Phe Pro Pro Leu
                165                 170                 175

Leu Asn Ile Phe Asn Lys Leu Val Gln Ile Pro Asn Pro Ser Ile Asp
            180                 185                 190

Val Ala Glu Leu Ile Lys Leu Ile Cys Lys Ile Phe Trp Ser Ser Ile
        195                 200                 205

Tyr Leu Glu Ile Pro Lys Gln Leu Phe Glu Pro Asn Leu Phe Asn Ala
    210                 215                 220

Trp Met Val Leu Phe Leu Asn Ile Leu Glu Arg Pro Val Pro Leu Glu
225                 230                 235                 240

Gly Gln Pro Ala Asp Leu Glu Gln Arg Lys Ser Trp Gly Trp Trp Lys
                245                 250                 255

Val Lys Lys Trp Thr Val His Ile Leu Asn Arg Leu Tyr Thr Arg Phe
            260                 265                 270

Gly Asp Met Arg Leu Gln Asn Pro Glu Asn Lys Ala Phe Ala Leu Met
        275                 280                 285

Phe Gln Lys Ser Tyr Ala Gly Lys Ile Leu Asp Cys His Leu Asn Leu
    290                 295                 300

Leu Asn Val Ile Arg Ala Gly Gly Tyr Leu Pro Asp Arg Val Ile Asn
305                 310                 315                 320

Leu Ile Leu Gln Tyr Leu Ser Asn Ser Ile Ser Lys Ser Ser Met Tyr
                325                 330                 335

Gln Leu Leu Gln Pro Arg Leu Asp Val Leu Leu Phe Glu Ile Ile Phe
            340                 345                 350

Pro Leu Met Cys Phe Asn Asp Asn Asp Gln Arg Leu Trp Asp Glu Asp
        355                 360                 365

Pro His Glu Tyr Val Arg Lys Gly Tyr Asp Ile Ile Glu Asp Leu Tyr
    370                 375                 380

Ser Pro Arg Thr Ala Ala Met Asp Phe Val Ser Glu Leu Val Arg Lys
385                 390                 395                 400

Arg Gly Lys Glu Asn Leu Gln Lys Phe Ile Gln Phe Val Val Glu Ile
                405                 410                 415

Leu Lys Arg Tyr Asp Glu Ile Pro Val Glu His Lys Pro Tyr Arg Gln
            420                 425                 430

Lys Asp Gly Ala Leu Leu Ala Ile Gly Ala Leu Cys Asp Lys Leu Lys
        435                 440                 445

Gln Thr Ala Pro Tyr Lys Ser Glu Leu Glu Arg Met Leu Val Gln His
    450                 455                 460

Val Phe Ser Glu Phe Asn Ser Pro Ala Gly His Leu Arg Ala Lys Ala
465                 470                 475                 480

Ala Trp Val Ala Gly Gln Tyr Ala His Ile Asn Phe Ser Asp Gln Asp
                485                 490                 495

Asn Phe Arg Lys Ala Leu His Ser Val Val Ala Gly Leu Arg Asp Pro
            500                 505                 510

Glu Leu Pro Val Arg Val Asp Ser Val Phe Ala Leu Arg Ser Phe Val
        515                 520                 525

Glu Ala Cys Lys Asp Leu Gly Glu Ile Arg Pro Ile Leu Pro Gln Leu
    530                 535                 540

Leu Asp Glu Phe Phe Lys Leu Met Asn Glu Val Glu Asn Glu Asp Leu
545                 550                 555                 560

Val Phe Thr Leu Glu Thr Ile Val Asp Lys Phe Gly Glu Glu Met Ala

                565                 570                 575

Pro Tyr Ala Phe Gly Leu Cys Gln Asn Leu Ala Ala Ala Phe Trp Lys
            580                 585                 590

Cys Met Ser Thr Ala Glu Ala Asp Glu Glu Gly Asp Asp Pro Gly Gly
        595                 600                 605

Ala Leu Ala Ala Val Gly Cys Leu Arg Ala Ile Ser Thr Ile Leu Glu
    610                 615                 620

Ser Val Ser Arg Leu Pro His Leu Phe Ala Gln Ile Glu Pro Thr Leu
625                 630                 635                 640

Leu Pro Ile Met Arg Arg Met Leu Thr Thr Asp Gly Gln Glu Val Phe
                645                 650                 655

Glu Glu Val Leu Glu Ile Val Ser Tyr Met Thr Phe Phe Ser Pro Thr
            660                 665                 670

Ile Ser Leu Asp Met Trp Ser Leu Trp Pro Leu Met Met Asp Ala Leu
        675                 680                 685

Ser Asp Trp Ala Ile Asp Phe Phe Pro Asn Ile Leu Val Pro Leu Asp
    690                 695                 700

Asn Tyr Val Ser Arg Ser Thr Ala His Phe Leu Thr Cys Lys Asp Pro
705                 710                 715                 720

Asp Tyr Gln Gln Ser Leu Trp Lys Met Ile Ser Ser Ile Met Ser Asp
                725                 730                 735

Asn Asn Leu Glu Asp Gly Asp Ile Glu Pro Ala Pro Lys Leu Ile Glu
            740                 745                 750

Val Val Phe Gln Asn Cys Lys Gly Gln Val Asp His Trp Val Glu Pro
        755                 760                 765

Tyr Leu Arg Ile Thr Val Glu Arg Leu Arg Arg Ala Glu Lys Pro Tyr
    770                 775                 780

Leu Lys Cys Leu Leu Ile Gln Val Ile Ala Asn Ala Leu Tyr Tyr Asn
785                 790                 795                 800

Pro Ser Leu Ala Leu Ser Ile Leu His Gln Leu Gly Val Ala Thr Glu
                805                 810                 815

Ile Phe Asn Leu Trp Phe Gln Met Ile Gln Gln Val Lys Arg Ser Gly
                820                 825                 830

Ala Arg Ala Asn Phe Lys Arg Glu His Asp Lys Lys Val Cys Cys Leu
        835                 840                 845

Gly Leu Thr Ser Leu Leu Ser Leu Pro Ala Asp Gln Leu Pro Val Glu
    850                 855                 860

Ala Leu Gly Arg Ile Phe Arg Val Thr Leu Glu Leu Leu Val Ala Tyr
865                 870                 875                 880

Lys Glu Gln Val Ala Glu Thr Ala Lys Glu Asp Glu Ala Glu Asp Asp
                885                 890                 895

Asp Glu Met Asp Gly Phe Glu Ser Asp Gly Glu Asp Glu Gly Asp Gly
                900                 905                 910

Ser Asp Lys Glu Met Gly Val Asp Glu Asp Glu Gly Asp Asp Ala Asp
        915                 920                 925

Ser Asn His Leu Asn Arg Leu Ala Ala Arg Ala Lys Ala Phe Arg Ser
    930                 935                 940

Thr Asp Glu Asp Asp Asp Asp Ser Glu Asp Asp Tyr Ser Asp Asp Glu
945                 950                 955                 960

Glu Leu Gln Ser Pro Ile Asp Glu Val Asp Pro Phe Ile Leu Phe Val
                965                 970                 975

Asp Thr Val Lys Val Met Gln Gly Ser Asp Pro Leu Arg Phe Gln Asn
                980                 985                 990

Leu Thr Gln Thr Leu Glu Phe Gln  Tyr Gln Ala Leu Ala  Asn Gly Val
        995                 1000                 1005

Ala Gln  His Ala Asp Gln Arg  Arg Val Glu Ile Glu  Lys Glu Lys
    1010                 1015                 1020

Leu Glu  Lys Ala Ala Ala Ala  Pro Ser
    1025                 1030

<210>  7
<211>  1039
<212>  PRT
<213>  Brassica napus

<400>  7

Met Asp Leu Pro Ser Leu Ala Leu Ile Leu Arg Ala Ala Ala Leu Ser
1               5                   10                  15

Pro Asn Pro Asp Glu Arg Lys Ala Ser Glu Gln Gln Leu Asn Gln Leu
            20                  25                  30

Gln His Thr Pro Gln His Leu Val Arg Leu Leu Gln Ile Val Val Asp
        35                  40                  45

Gly Asn Cys Asp Met Ala Val Arg Gln Ile Ala Ser Ile Gln Phe Lys
    50                  55                  60

Asn Phe Ile Ala Lys Asn Trp Ser Pro Val Asp Ser Gly Glu Gln Pro
65                  70                  75                  80

Arg Ile Leu Gln Ser Asp Lys Glu Leu Val Arg Asp Asn Ile Leu Val
                85                  90                  95

Tyr Val Thr His Val Pro Thr Leu Leu Arg Ser Gln Leu Gly Glu Cys
            100                 105                 110

Leu Lys Thr Ile Ile Tyr Ser Asp Tyr Pro Glu Gln Trp Pro Arg Leu
        115                 120                 125

Leu Asp Trp Val Lys Tyr Asn Leu Gln Asn Gln Gln Ile Tyr Gly Ala
    130                 135                 140

Leu Phe Val Leu Arg Ile Leu Ser Arg Lys Tyr Glu Phe Lys Ser Asp
145                 150                 155                 160

Glu Glu Arg Thr Pro Val Ser Arg Ile Val Glu Glu Thr Phe Pro Val
                165                 170                 175

Leu Leu Thr Ile Phe Asn Gly Leu Ile Gln Ile Glu Asn Pro Ser Leu
            180                 185                 190

Glu Ile Ala Glu Phe Met Lys Leu Ile Cys Lys Ile Phe Trp Ser Ser
        195                 200                 205

Ile Tyr Leu Glu Leu Pro Lys Gln Leu Ala Asp Pro Asn Val Phe Asn
    210                 215                 220

Ala Trp Met Leu Leu Phe Leu Thr Val Ser Glu Arg Pro Val Pro Val
225                 230                 235                 240

Glu Gly Gln Pro Met Asp Pro Glu Leu Arg Lys Ser Trp Gly Trp Trp
                245                 250                 255

Lys Val Lys Lys Trp Thr Val His Ile Leu Asn Arg Leu Tyr Ser Arg
            260                 265                 270

Phe Gly Asp Pro Lys Leu Gln Thr Leu Glu Asn Lys Pro Phe Ala Gln
        275                 280                 285

Met Phe Gln Lys Thr Tyr Ala Gly Arg Ile Leu Glu Gly His Leu Asn
    290                 295                 300

Phe Leu Asn Thr Ile Arg Leu Gly Gly Tyr Ile Pro Asp Arg Val Thr
305                 310                 315                 320

Asn Leu Leu Leu Gln Tyr Leu Ser Asn Ser Ile Ser Lys Asn Ser Met
                325                 330                 335

Tyr Ser Leu Leu Leu Pro Arg Leu Asp Val Leu Leu Phe Glu Ile Val
            340                 345                 350

Phe Pro Leu Met Cys Phe Asn Asp Ser Asp Gln Lys Leu Trp Glu Glu
        355                 360                 365

Asp Pro His Glu Tyr Val Arg Lys Gly Tyr Asn Ile Ile Glu Asp Leu
    370                 375                 380

Tyr Ser Pro Arg Thr Ala Ser Met Asp Phe Val Asn Glu Leu Val Gln
385                 390                 395                 400

Lys Arg Gly Ile Glu Asn Leu Pro Lys Phe Val Gln Phe Val Val Gly
                405                 410                 415

Ile Phe Arg Ser Tyr Asp Glu Ala Pro Ala Glu His Lys Pro Tyr Arg
            420                 425                 430

Gln Lys Asp Gly Ala Met Leu Ala Val Gly Ala Leu Cys Asp Lys Leu
        435                 440                 445

Lys Lys Thr Glu Pro Tyr Lys Ser Glu Leu Glu His Met Leu Val Gln
    450                 455                 460

His Ile Phe Pro Glu Phe Ser Ser Pro Ala Gly His Leu Arg Ala Lys
465                 470                 475                 480

Ala Ala Trp Val Ala Gly Gln Tyr Ala His Ile Asn Phe Ser Asp Gln
                485                 490                 495

Asn Asn Phe Arg Lys Ala Leu His Ser Val Val Ser Gly Met Arg Asp
            500                 505                 510

Pro Asp Leu Pro Val Arg Ile Asp Ser Val Phe Ala Leu Arg Ser Phe
        515                 520                 525

Val Glu Ala Cys Lys Asp Leu Asn Glu Ile Arg Pro Ile Leu Pro Gln
    530                 535                 540

Leu Leu Asp Glu Phe Phe Lys Leu Met Asn Glu Val Glu Asn Glu Asp
545                 550                 555                 560

Leu Val Phe Thr Leu Glu Thr Ile Val Asp Lys Phe Gly Glu Glu Met
                565                 570                 575

Ala Pro Phe Ala Phe Gly Leu Cys Gln Asn Leu Ala Ala Ala Phe Trp
            580                 585                 590

Arg Cys Leu Asn Thr Ser Glu Ala Gly Asp Asp Ser Asp Asp Met Gly
        595                 600                 605

Ala Leu Ala Ala Val Gly Cys Leu Arg Ala Ile Ser Thr Ile Leu Glu
    610                 615                 620

Ser Val Ser Ser Leu Pro Gln Leu Phe Val Glu Ile Glu Pro Thr Ile
625                 630                 635                 640

Leu Pro Ile Met Gln Lys Met Leu Thr Thr Asp Gly His Asp Val Phe
                645                 650                 655

Glu Glu Val Leu Glu Ile Ala Ser Tyr Met Thr Phe Tyr Ser Pro Thr
            660                 665                 670

Ile Ser Leu Asp Ile Trp Ser Leu Trp Pro Leu Leu Val Glu Ala Leu
        675                 680                 685

Val Asp Trp Ala Ile Asp Phe Phe Pro Asn Ile Leu Val Pro Met Asp
    690                 695                 700

Asn Phe Ile Ser Arg Gly Thr Ala His Phe Leu Thr Cys Lys Glu Pro
705                 710                 715                 720

Asp Tyr Gln Gln Ser Leu Phe Asn Val Leu Ser Thr Leu Met Thr Asp
                725                 730                 735

Arg Asn Ile Glu Asp Ser Asp Val Glu Ser Ala Pro Lys Leu Ile Glu
            740                 745                 750

Val Val Phe Gln Asn Cys Lys Gly Gln Val Asp Gln Trp Val Glu Pro

755                 760                 765

Tyr Leu Arg Leu Thr Val Asp Arg Leu Gln Arg Ala Glu Thr Ser Tyr
    770                 775                 780

Leu Lys Ser Leu Leu Ile Gln Val Val Ala Asn Met Leu Tyr Tyr Asn
785                 790                 795                 800

Pro Gly Leu Thr Leu Gly Val Leu His Asn Thr Gly Leu Gly Ser Thr
                805                 810                 815

Val Phe Asp Leu Trp Phe Gln Met Leu Gln Gln Lys Lys Lys Ser Gly
            820                 825                 830

Leu Pro Ala Asn Phe Lys Arg Glu His Asp Lys Lys Val Cys Cys Leu
        835                 840                 845

Gly Leu Thr Ser Leu Leu Ala Leu Pro Gly Gly Gln Phe Pro Asp Glu
    850                 855                 860

Ala Leu Gln Arg Val Phe Arg Ala Thr Leu Asp Leu Leu Val Ala Tyr
865                 870                 875                 880

Lys Asn Gln Ile Ala Glu Ala Lys Glu Ala Glu Val Asp Tyr Glu Asn
                885                 890                 895

Glu Met Asn Gly Leu Asp Ser Asp Asp Glu Asp Asp Asp Gly Ser Asp
            900                 905                 910

Gly Glu Met Gly Met Asp Asp Thr Glu Asp Gly Asp Glu Ala Glu Ser
        915                 920                 925

Met Arg Leu Lys Lys Leu Ala Ala Gln Ala Lys Ser Tyr Gly Tyr Asp
    930                 935                 940

Asp Glu Asp Glu Asp Asp Asp Asp Ser Asp Asp Gly Ser Asp Asp Asp
945                 950                 955                 960

Asp Glu Phe Gln Ser Pro Ile Asp Glu Val Asp Ala Phe Val Phe Phe
                965                 970                 975

Val Asp Ala Ile Arg Val Met Gln Ala Ser Asp Ala Gln Arg Phe Gln
            980                 985                 990

Asn Leu Asn Gln Ser Leu Asp Phe  Thr Tyr Gln Ala Ile  Ala Asn Gly
        995                 1000                1005

Ile Ala Gln His Ala Glu Val Arg Arg Val Glu Ile Glu Lys Glu
    1010                1015                1020

Lys Gln Lys Lys Ala Ala Glu Ala Ala Gly Thr Pro Val Pro Ala
    1025                1030                1035

Ile

<210> 8
<211> 1037
<212> PRT
<213> Brassica napus

<400> 8

Met Asp Leu Pro Ser Leu Ala Leu Ile Leu Arg Ala Ala Ala Leu Ser
1               5                   10                  15

Pro Asn Pro Asp Glu Arg Lys Ala Ser Glu Gln Gln Leu Asn Gln Leu
            20                  25                  30

Gln His Thr Pro Gln His Leu Val Arg Leu Leu Gln Ile Val Val Asp
        35                  40                  45

Gly Asn Cys Asp Met Ala Val Arg Gln Ile Ala Ser Ile Gln Phe Lys
    50                  55                  60

Asn Phe Ile Ala Lys Asn Trp Ala Pro Val Asp Ser Gly Glu Gln Pro
65                  70                  75                  80

Arg Ile Leu Gln Ser Asp Lys Glu Leu Val Arg Asp Asn Ile Leu Val
                85                  90                  95

Tyr Val Thr Gln Val Pro Thr Leu Leu Arg Ser Gln Leu Gly Glu Cys
            100                 105                 110

Leu Lys Thr Ile Ile Tyr Ser Asp Tyr Pro Glu Gln Trp Pro Arg Leu
        115                 120                 125

Leu Asp Trp Val Lys Tyr Asn Phe Gln Asn Gln Gln Ile Tyr Gly Ala
    130                 135                 140

Leu Phe Val Leu Arg Ile Leu Ser Arg Lys Tyr Glu Phe Lys Ser Asp
145                 150                 155                 160

Glu Glu Arg Thr Pro Val Ser Arg Ile Val Glu Glu Thr Phe Pro Val
                165                 170                 175

Leu Leu Thr Ile Phe Asn Gly Leu Ile Gln Ile Glu Asn Pro Ser Leu
            180                 185                 190

Glu Ile Ala Glu Phe Met Lys Leu Ile Cys Lys Ile Phe Trp Ser Ser
        195                 200                 205

Ile Tyr Leu Glu Leu Pro Lys Gln Leu Thr Asp Pro Asn Val Phe Asn
    210                 215                 220

Ala Trp Met Leu Leu Phe Leu Thr Val Ser Glu Arg Pro Val Pro Val
225                 230                 235                 240

Glu Gly Gln Pro Met Asp Pro Glu Leu Arg Lys Ser Trp Gly Trp Trp
                245                 250                 255

Lys Val Lys Lys Trp Thr Val His Ile Leu Asn Arg Leu Tyr Ser Arg
            260                 265                 270

Phe Gly Asp Pro Lys Leu Gln Thr Leu Glu Asn Lys Pro Phe Ala Gln
        275                 280                 285

Met Phe Gln Lys Thr Tyr Ala Gly Arg Ile Leu Glu Gly His Leu Asn
    290                 295                 300

Phe Leu Thr Thr Ile Arg Leu Gly Gly Tyr Ile Pro Asp Arg Val Thr
305                 310                 315                 320

Asn Leu Leu Leu Gln Tyr Leu Ser Asn Ser Ile Ser Lys Asn Ser Met
                325                 330                 335

Tyr Ser Leu Leu Leu Pro Arg Leu Asp Val Leu Leu Phe Glu Ile Val
            340                 345                 350

Phe Pro Leu Met Cys Phe Asn Asp Ser Asp Gln Lys Leu Trp Glu Glu
        355                 360                 365

Asp Pro His Glu Tyr Val Arg Lys Gly Tyr Asn Ile Ile Glu Asp Leu
    370                 375                 380

Tyr Ser Pro Arg Thr Ala Ser Ile Asp Phe Val Asn Glu Leu Val Gln
385                 390                 395                 400

Lys Arg Gly Lys Glu Asn Leu Pro Lys Phe Val Gln Phe Val Val Gly
                405                 410                 415

Ile Phe Arg Ser Tyr Asp Glu Ala Pro Ala Glu His Lys Pro Tyr Arg
            420                 425                 430

Gln Lys Asp Gly Ala Met Leu Ala Val Gly Ala Leu Cys Asp Lys Leu
        435                 440                 445

Lys Lys Thr Glu Pro Tyr Lys Ser Glu Leu Glu His Met Leu Val Gln
    450                 455                 460

His Ile Phe Pro Glu Phe Ser Ser Pro Ala Gly His Leu Arg Ala Lys
465                 470                 475                 480

Ala Ala Trp Val Ala Gly Gln Tyr Ala Arg Ile Asn Phe Ser Asp Gln
                485                 490                 495

Asn Asn Phe Arg Lys Ser Leu His Ser Val Val Ser Gly Met Arg Asp
            500                 505                 510

Pro Asp Leu Pro Val Arg Ile Asp Ser Val Phe Ala Leu Arg Ser Phe
        515                 520                 525

Val Glu Ala Cys Lys Asp Leu Asn Glu Ile Arg Pro Ile Leu Pro Gln
    530                 535                 540

Leu Leu Asp Glu Phe Phe Lys Leu Met Asn Glu Val Glu Asn Glu Asp
545                 550                 555                 560

Leu Val Phe Thr Leu Glu Thr Ile Val Asp Lys Phe Gly Glu Glu Met
                565                 570                 575

Ala Pro Phe Ala Phe Gly Leu Cys Gln Asn Leu Ala Ala Ala Phe Trp
            580                 585                 590

Arg Cys Leu Asn Thr Ser Glu Ala Gly Asp Asp Ser Asp Asp Met Gly
        595                 600                 605

Ala Leu Ala Ala Val Gly Cys Leu Arg Ala Ile Ser Thr Ile Leu Glu
    610                 615                 620

Ser Val Ser Ser Leu Pro Gln Leu Phe Val Glu Ile Glu Pro Thr Ile
625                 630                 635                 640

Leu Pro Ile Met Gln Lys Met Leu Thr Thr Asp Gly His Asp Val Phe
                645                 650                 655

Glu Glu Val Leu Glu Ile Ala Ser Tyr Met Thr Phe Tyr Ser Pro Thr
            660                 665                 670

Ile Ser Leu Asp Ile Trp Ser Leu Trp Pro Leu Leu Val Glu Ala Leu
        675                 680                 685

Val Asp Trp Ala Ile Asp Phe Phe Pro Asn Ile Leu Val Pro Met Asp
    690                 695                 700

Asn Phe Ile Ser Arg Gly Thr Ala His Phe Leu Thr Cys Lys Glu Pro
705                 710                 715                 720

Asp Tyr Gln Gln Ser Leu Phe Asn Val Leu Ser Thr Leu Met Thr Asp
                725                 730                 735

Arg Asn Ile Glu Asp Ser Asp Val Glu Ser Ala Pro Lys Leu Ile Glu
            740                 745                 750

Val Val Phe Gln Asn Cys Lys Gly Gln Val Asp Gln Trp Val Glu Pro
        755                 760                 765

Tyr Leu Arg Leu Thr Val Asp Arg Leu Gln Arg Ala Glu Thr Ser Tyr
    770                 775                 780

Leu Lys Ser Leu Leu Ile Gln Val Val Ala Asn Met Leu Tyr Tyr Asn
785                 790                 795                 800

Pro Ala Leu Thr Leu Gly Val Leu His Asn Thr Gly Leu Gly Ser Thr
                805                 810                 815

Val Phe Asp Leu Trp Phe Gln Met Leu Gln Gln Lys Lys Lys Ser Gly
            820                 825                 830

Leu Ala Ala Asn Phe Lys Arg Glu His Asp Lys Lys Val Cys Cys Leu
        835                 840                 845

Gly Leu Thr Ser Leu Leu Ala Leu Pro Gly Gly Gln Phe Pro Asp Glu
    850                 855                 860

Ala Leu Gln Arg Val Phe Arg Ala Thr Leu Asp Leu Leu Val Ala Tyr
865                 870                 875                 880

Lys Asn Gln Ile Ala Ala Ala Lys Glu Ala Glu Val Asp Tyr Glu Asn
                885                 890                 895

Glu Met Asn Gly Leu Asp Ser Asp Asp Glu Asp Asp Asp Gly Ser Asp
            900                 905                 910

Gly Glu Met Gly Met Asp Asp Thr Glu Asp Gly Asp Glu Ala Glu Ser
        915                 920                 925

Met Arg Leu Lys Lys Leu Ala Ala Gln Ala Lys Ser Tyr Gly Tyr Asp

930                 935                 940

Asp Glu Asp Asp Asp Asp Ser Asp Asp Gly Ser Asp Asp Asp Asp Glu
945                 950                 955                 960

Phe Gln Ser Pro Ile Asp Glu Val Asp Ala Phe Val Phe Phe Val Asp
                965                 970                 975

Ala Ile Arg Val Met Gln Ala Ser Asp Ala Gln Arg Phe Gln Asn Leu
            980                 985                 990

Asn Gln Ser Leu Asp Phe Thr Tyr  Gln Ala Ile Ala Asn  Gly Ile Ala
        995                 1000                 1005

Gln His  Ala Glu Val Arg Arg  Val Glu Ile Glu Lys  Glu Lys Gln
    1010                 1015                 1020

Lys Lys  Ala Ala Glu Ala Ala  Gly Ala Pro Val Pro  Ala Ile
    1025                 1030                 1035

<210>  9
<211>  1040
<212>  PRT
<213>  Brassica oleracea

<400>  9

Met Asp Leu Pro Ser Leu Ala Leu Ile Leu Arg Ala Ala Ala Leu Ser
1               5                   10                  15

Pro Asn Pro Asp Glu Arg Lys Ala Ser Glu Gln Gln Leu Asn Gln Leu
            20                  25                  30

Gln His Thr Pro Gln His Leu Val Arg Leu Leu Gln Ile Val Val Asp
        35                  40                  45

Gly Asn Cys Asp Met Ala Val Arg Gln Ile Ala Ser Ile Gln Phe Lys
    50                  55                  60

Asn Phe Ile Ala Lys Asn Trp Ala Pro Val Asp Ser Gly Glu Gln Pro
65                  70                  75                  80

Arg Ile Leu Gln Ser Asp Lys Glu Leu Val Arg Asp Asn Ile Leu Val
                85                  90                  95

Tyr Val Thr Gln Val Pro Thr Leu Leu Arg Ser Gln Leu Gly Glu Cys
            100                 105                 110

Leu Lys Thr Ile Ile Tyr Ala Asp Tyr Pro Glu Gln Trp Pro Arg Leu

        115                 120                 125

Leu Asp Trp Val Lys Tyr Asn Leu Gln Asn Gln Gln Ile Tyr Gly Ala
    130                 135                 140

Leu Phe Val Leu Arg Ile Leu Ser Arg Lys Tyr Glu Phe Lys Ser Asp
145                 150                 155                 160

Glu Glu Arg Thr Pro Val Ser Arg Ile Val Glu Glu Thr Phe Pro Val
                165                 170                 175

Leu Leu Thr Ile Phe Asn Gly Leu Ile Gln Ile Glu Asn Pro Ser Leu
            180                 185                 190

Glu Ile Ala Glu Phe Met Lys Leu Ile Cys Lys Ile Phe Trp Ser Ser
        195                 200                 205

Ile Tyr Leu Glu Leu Pro Lys Gln Leu Ala Asp Pro Asn Val Phe Asn
    210                 215                 220

Ala Trp Met Leu Leu Phe Leu Thr Val Ser Glu Arg Pro Val Pro Val
225                 230                 235                 240

Glu Gly Gln Pro Met Asp Pro Glu Leu Arg Lys Ser Trp Gly Trp Trp
                245                 250                 255

Lys Val Lys Lys Trp Thr Val His Ile Leu Asn Arg Leu Tyr Ser Arg
            260                 265                 270

Phe Gly Asp Pro Lys Leu Gln Thr Leu Glu Asn Lys Pro Phe Ala Gln
        275                 280                 285

Met Phe Gln Lys Thr Tyr Ala Ser Arg Ile Leu Glu Gly His Leu Asn
    290                 295                 300

Phe Leu Asn Thr Ile Arg Leu Gly Gly Tyr Ile Pro Asp Arg Val Thr
305                 310                 315                 320

Asn Leu Leu Leu Gln Tyr Leu Ser Asn Ser Ile Ser Lys Asn Ser Met
                325                 330                 335

Tyr Ser Leu Leu Leu Pro Arg Leu Asp Val Leu Leu Phe Glu Ile Val
            340                 345                 350

Phe Pro Leu Met Cys Phe Asn Asp Ser Asp Gln Lys Leu Trp Glu Glu
        355                 360                 365

Asp Pro His Glu Tyr Val Arg Lys Gly Tyr Asn Ile Ile Glu Asp Leu
    370                 375                 380

Tyr Ser Pro Arg Thr Ala Ser Met Asp Phe Val Asn Glu Leu Val Gln
385                 390                 395                 400

Lys Arg Gly Lys Glu Asn Leu Pro Lys Phe Val Gln Phe Val Val Gly
                405                 410                 415

Ile Phe Arg Ser Tyr Asp Glu Ala Pro Ala Glu His Lys Pro Tyr Arg
            420                 425                 430

Gln Lys Asp Gly Ala Met Leu Ala Val Gly Ala Leu Cys Asp Lys Leu
        435                 440                 445

Lys Lys Thr Glu Pro Tyr Lys Ser Glu Leu Glu His Met Leu Val Gln
    450                 455                 460

His Ile Phe Pro Glu Phe Ser Ser Pro Ala Gly His Leu Arg Ala Lys
465                 470                 475                 480

Ala Ala Trp Val Ala Gly Gln Tyr Ala His Ile Asn Phe Ser Asp Gln
                485                 490                 495

Asn Asn Phe Arg Lys Ser Leu His Ser Val Val Ser Gly Met Arg Asp
            500                 505                 510

Pro Asp Leu Pro Val Arg Ile Asp Ser Val Phe Ala Leu Arg Ser Phe
        515                 520                 525

Val Glu Ala Cys Lys Asp Leu Asn Glu Ile Arg Pro Ile Leu Pro Gln
    530                 535                 540

Leu Leu Asp Glu Phe Phe Lys Leu Met Asn Glu Val Glu Asn Glu Asp
545                 550                 555                 560

Leu Val Phe Thr Leu Glu Thr Ile Val Asp Lys Phe Gly Glu Glu Met
                565                 570                 575

Ala Pro Phe Ala Phe Gly Leu Cys Gln Asn Leu Ala Ala Ala Phe Trp
            580                 585                 590

Arg Cys Leu Asn Thr Ser Glu Ala Gly Asp Asp Ser Asp Asp Met Gly
        595                 600                 605

Ala Leu Ala Ala Val Gly Cys Leu Arg Ala Ile Ser Thr Ile Leu Glu
    610                 615                 620

Ser Val Ser Ser Leu Pro Gln Leu Phe Val Glu Ile Glu Pro Thr Ile
625                 630                 635                 640

Leu Pro Ile Met Gln Lys Met Leu Thr Asn Asp Gly His Asp Val Phe
                645                 650                 655

Glu Glu Val Leu Glu Ile Ala Ser Tyr Met Thr Phe Tyr Ser Pro Thr
            660                 665                 670

Ile Ser Leu Asp Ile Trp Ser Leu Trp Pro Leu Leu Val Glu Ala Leu
        675                 680                 685

Val Asp Trp Ala Ile Asp Phe Phe Pro Asn Ile Leu Val Pro Met Asp
    690                 695                 700

Asn Phe Ile Ser Arg Gly Thr Ala His Phe Leu Thr Cys Lys Glu Pro
705                 710                 715                 720

Asp Tyr Gln Gln Ser Leu Phe Asn Val Leu Ser Thr Leu Met Thr Asp
                725                 730                 735

Arg Asn Ile Glu Asp Ser Asp Val Glu Ser Ala Pro Lys Leu Ile Glu
            740                 745                 750

Val Val Phe Gln Asn Cys Lys Gly Gln Val Asp Gln Trp Val Glu Pro
        755                 760                 765

Tyr Leu Arg Leu Thr Val Asp Arg Leu Gln Arg Ala Glu Thr Ser Tyr
    770                 775                 780

Leu Lys Ser Leu Leu Ile Gln Val Val Ala Asn Met Leu Tyr Tyr Asn
785                 790                 795                 800

Pro Ala Leu Thr Leu Gly Val Leu His Asn Thr Gly Leu Gly Ser Thr
                805                 810                 815

Val Phe Asp Leu Trp Phe Gln Met Leu Gln Gln Lys Lys Lys Ser Gly
            820                 825                 830

Leu Ala Ala Asn Phe Lys Arg Glu His Asp Lys Lys Val Cys Cys Leu
        835                 840                 845

Gly Leu Thr Ser Leu Leu Ala Leu Pro Gly Gly Gln Phe Pro Asp Glu
    850                 855                 860

Ala Leu Gln Arg Val Phe Arg Ala Thr Leu Asp Leu Leu Val Ala Tyr
865                 870                 875                 880

Lys Asn Gln Ile Ala Glu Ala Ala Lys Glu Ala Glu Val Asp Tyr Glu
                885                 890                 895

Asn Glu Met Asn Gly Leu Asp Ser Asp Asp Glu Asp Asp Asp Gly Ser
            900                 905                 910

Asp Gly Glu Met Gly Met Asp Asp Thr Glu Asp Gly Asp Glu Ala Glu
        915                 920                 925

Ser Met Arg Leu Lys Lys Leu Val Ala Gln Ala Lys Ser Tyr Gly Tyr
    930                 935                 940

Asp Asp Glu Asp Glu Asp Asp Asp Asp Ser Asp Asp Gly Ser Asp Asp
945                 950                 955                 960

Asp Asp Glu Phe Gln Ser Pro Ile Asp Glu Val Asp Ala Phe Val Phe
                965                 970                 975

Phe Val Asp Ala Ile Arg Val Met Gln Ala Ser Asp Ala Gln Arg Phe
            980                 985                 990

Gln Asn Leu Asn Gln Ser Leu Asp  Phe Thr Tyr Gln Ala  Ile Ala Asn
        995                 1000                1005

Gly Ile  Ala Gln His Ala Glu  Ala Arg Arg Val Glu  Ile Glu Lys
    1010                1015                1020

Glu Lys  Gln Lys Lys Ala Ala  Glu Ala Ala Gly Thr  Pro Val Pro
    1025                1030                1035

Ala Ile
    1040

<210>  10
<211>  1039
<212>  PRT
<213>  Capsicum annuum

<400>  10

Met Asp Leu Gln Asn Leu Ala Ile Ile Leu Arg Gly Ala Leu Ser Pro
1               5                   10                  15

Asn Pro Asp Glu Arg Lys Ala Ala Glu Asn Ser Leu Asn Gln Phe Gln
            20                  25                  30

His Thr Pro Gln His Leu Val Arg Leu Leu Gln Ile Ile Val Asp Gly
        35                  40                  45

Ser Cys Asp Met Ala Val Arg Gln Val Ala Ser Ile His Phe Lys Asn
    50                  55                  60

Phe Val Ala Lys Asn Trp Ser Pro His Asp Pro Ala Glu Gln Ser Lys
65                  70                  75                  80

Ile Met Pro Ser Asp Lys Glu Leu Val Arg Gln Asn Ile Leu Ile Phe
                85                  90                  95

Val Ala Gln Val Pro Ser Leu Leu Arg Val Gln Leu Gly Glu Cys Ile
            100                 105                 110

Lys Thr Met Ile His Ala Asp Tyr Pro Glu Gln Trp Pro Thr Leu Leu
        115                 120                 125

Pro Trp Val Lys His Asn Leu Gln Asp Gln Gln Val Tyr Gly Ala Leu
    130                 135                 140

Phe Val Leu Arg Ile Leu Ser Arg Lys Tyr Glu Phe Lys Ser Asp Glu
145                 150                 155                 160

Glu Arg Ala Pro Val Tyr His Val Val Glu Glu Thr Phe Pro His Leu
                165                 170                 175

Leu Asn Ile Phe Asn Arg Leu Val Gln Ile Ala Asn Pro Ser Ile Glu
            180                 185                 190

Val Ala Asp Leu Ile Lys Leu Ile Cys Lys Ile Phe Trp Ser Ser Ile
        195                 200                 205

Tyr Leu Glu Ile Pro Lys Gln Leu Phe Asp Pro Asn Val Phe Asn Ala
    210                 215                 220

Trp Met Val Leu Phe Leu Asn Met Leu Glu Arg Pro Val Pro Val Glu
225                 230                 235                 240

Gly Gln Pro Ala Asp Pro Glu Leu Arg Lys Ser Trp Gly Trp Trp Lys
                245                 250                 255

Val Lys Lys Trp Thr Ile His Ile Leu Asn Arg Leu Tyr Thr Arg Phe
            260                 265                 270

Gly Asp Leu Lys Leu Gln Asn Pro Asp Asn Lys Ala Phe Ala Gln Met
        275                 280                 285

Phe Gln Lys Gly Tyr Ala Gly Lys Ile Leu Glu Cys His Leu Asn Leu

290                 295                 300

Leu Asn Val Ile Arg Ala Gly Gly Tyr Leu Pro Asp Arg Val Ile Asn
305                 310                 315                 320

Leu Ile Leu Gln Tyr Leu Ser Asn Ser Ile Ser Lys Ser Asn Met Tyr
                325                 330                 335

Ser Leu Leu Gln Pro Arg Leu Asp Thr Val Leu Phe Glu Ile Ile Phe
            340                 345                 350

Pro Leu Met Cys Phe Ser Asp Asn Asp Gln Lys Leu Trp Glu Glu Asp
        355                 360                 365

Pro His Glu Tyr Val Arg Lys Gly Tyr Asp Ile Ile Glu Asp Leu Tyr
    370                 375                 380

Ser Pro Arg Thr Ala Ala Met Asp Phe Val Ser Glu Leu Val Arg Lys
385                 390                 395                 400

Arg Gly Lys Glu Asn Leu Gln Lys Phe Ile Leu Phe Ile Val Glu Ile
                405                 410                 415

Phe Lys Arg Tyr Glu Glu Ala Ala Pro Glu Tyr Lys Pro Tyr Arg Gln
            420                 425                 430

Lys Asp Gly Ala Leu Leu Ala Ile Gly Ala Leu Cys Asp Lys Leu Lys
        435                 440                 445

Gln Thr Glu Pro Tyr Lys Ser Glu Leu Glu Arg Met Leu Val Gln His
    450                 455                 460

Val Phe Pro Glu Phe Ser Ser Pro Val Gly His Leu Arg Ala Lys Ala
465                 470                 475                 480

Ala Trp Val Ala Gly Gln Tyr Ala His Ile Asn Phe Ser Asp Pro Asn
                485                 490                 495

Asn Phe Arg Lys Ala Leu His Ser Val Val Asn Gly Met Arg Asp Pro
            500                 505                 510

Asp Leu Pro Val Arg Val Asp Ser Val Phe Ala Leu Arg Ser Phe Val
        515                 520                 525

Glu Ala Cys Lys Asp Leu Asn Glu Val Arg Pro Ile Leu Pro Gln Leu
    530                 535                 540

Leu Asp Glu Phe Phe Lys Leu Met Asn Glu Val Glu Asn Glu Asp Leu
545                 550                 555                 560

Val Phe Thr Leu Glu Thr Ile Val Asp Lys Phe Gly Glu Glu Met Ala
                565                 570                 575

Pro Tyr Ala Ile Gly Leu Cys Gln Asn Leu Ala Ala Ala Phe Trp Lys
            580                 585                 590

Cys Ile Asn Ser Ser Glu Ala Glu Glu Glu Ala Asp Asp Ala Gly Ala
        595                 600                 605

Leu Ala Ala Val Gly Cys Leu Arg Ala Ile Ser Thr Ile Leu Glu Ser
    610                 615                 620

Val Asn Arg Leu Pro His Leu Phe Met His Ile Glu Pro Thr Leu Leu
625                 630                 635                 640

Pro Ile Met Arg Arg Met Leu Thr Thr Asp Gly Gln Glu Val Phe Glu
                645                 650                 655

Glu Val Leu Glu Ile Val Ser Tyr Met Thr Phe Phe Ser Pro Thr Ile
            660                 665                 670

Ser Val Asp Met Trp Ser Leu Trp Pro Leu Met Met Glu Ala Leu Ala
        675                 680                 685

Asp Trp Ala Ile Asp Phe Phe Pro Asn Ile Leu Val Pro Leu Asp Asn
    690                 695                 700

Tyr Ile Ser Arg Ser Thr Ala His Phe Leu Thr Cys Lys Asp Pro Asp
705                 710                 715                 720

Tyr Gln Gln Ser Leu Trp Asn Met Ile Ser Ser Val Met Gly Asp Lys
                725                 730                 735

Asn Leu Glu Asp Ser Asp Ile Glu Ser Ala Pro Lys Leu Ile Gln Val
            740                 745                 750

Val Phe Gln His Cys Lys Gly Gln Val Asp Gln Trp Val Glu Pro Tyr
        755                 760                 765

Ile Arg Ile Thr Ile Glu Arg Leu Arg Arg Thr Glu Lys Pro Tyr Leu
    770                 775                 780

Lys Cys Leu Leu Val Gln Val Ile Ala Asp Ala Leu Phe Tyr Asn Ala
785                 790                 795                 800

Pro Leu Thr Leu Asn Ile Leu Gln Lys Leu Gly Val Ala Thr Glu Val
                805                 810                 815

Phe Asn Leu Trp Phe Gln Met Leu Gly Glu Thr Lys Lys Ser Gly Thr
            820                 825                 830

Arg Ala Asn Phe Lys Arg Glu Gln Asp Lys Lys Val Cys Cys Leu Gly
        835                 840                 845

Leu Thr Ser Leu Leu Pro Leu Pro Val Asp Gln Leu Pro Gly Glu Ala
    850                 855                 860

Leu Glu Arg Val Phe Lys Ala Thr Leu Asp Leu Leu Val Ala Tyr Lys
865                 870                 875                 880

Asp Gln Val Ala Glu Ala Ala Lys Glu Asp Glu Ala Glu Asp Asp Asp
                885                 890                 895

Asp Met Asn Gly Leu Gln Thr Asp Glu Asp Asp Asp Glu Asp Asp Gly
            900                 905                 910

Ser Asp Lys Glu Met Gly Asp Asp Ala Glu Glu Gly Asp Glu Ala Asp
        915                 920                 925

Ser Thr Arg Leu Gln Lys Leu Ala Ala Gln Ala Lys Ala Phe Arg Ser
    930                 935                 940

His Asp Glu Asp Glu Asp Asp Asp Ser Asp Asp Asp Phe Ser Asp Asp
945                 950                 955                 960

Glu Glu Glu Leu Gln Ser Pro Leu Asp Glu Val Asp Pro Phe Ile Phe
                965                 970                 975

Phe Val Glu Thr Ile Lys Ala Leu Gln Ala Ser Asp Pro Leu Arg Phe
            980                 985                 990

Gln Ser Leu Thr Gln Thr Leu Asp  Phe His Tyr Gln Ala  Leu Ala Asn
        995                 1000                1005

Gly Val  Ala Gln His Ala Glu  Gln Arg Lys Val Glu  Ile Glu Lys
    1010                1015                1020

Glu Lys  Met Glu Lys Ala Ser  Ala Ala Ala Ala Ala  Ala Ala Ala
    1025                1030                1035

Ser

<210>  11
<211>  1029
<212>  PRT
<213>  Cucumis sativus

<400>  11

Met Asp Leu Pro Ser Leu Ala Val Val Leu Gln Ala Val Leu Ser Pro
1               5                   10                  15

Asn Pro Asp Glu Arg Lys Ala Ala Glu Gln Ser Leu Asn Gln Ile Gln
            20                  25                  30

His Thr Pro Gln His Leu Val Arg Met Leu Gln Ile Ile Val Asp Asn
        35                  40                  45

Asn Cys Asp Leu Ala Val Arg Gln Val Ala Ser Ile His Phe Lys Asn
    50                  55                  60

Tyr Ile Ala Lys Asn Trp Ser Pro Val Asp Pro Asp Glu His Gln Lys
65                  70                  75                  80

Ile Ser Glu Ser Asp Lys Asp Ala Val Arg Lys Asn Ile Leu Pro Phe
                85                  90                  95

Leu Ser Gln Val Pro Ser Leu Leu Arg Val Gln Leu Gly Glu Cys Leu
            100                 105                 110

Lys Thr Ile Ile His Ala Asp Tyr Pro Glu Gln Trp Pro Ser Leu Leu
        115                 120                 125

Glu Trp Val Lys Glu Asn Leu Leu Ala Ser Asn Val Tyr Gly Ala Leu
    130                 135                 140

Phe Val Leu Arg Ile Leu Ala Arg Lys Tyr Glu Phe Lys Ser Asp Asp
145                 150                 155                 160

Asp Arg Thr Pro Val Tyr Arg Ile Val Asp Glu Thr Phe Pro Leu Leu
                165                 170                 175

Leu Asn Ile Phe Ser Arg Leu Val Gln Ile Gly Asp Pro Ser Leu Glu
            180                 185                 190

Val Ala Glu Leu Ile Lys Phe Ile Cys Lys Ile Phe Trp Ser Ser Ile
        195                 200                 205

Tyr Met Glu Ile Pro Lys His Leu Phe Asp Thr His Val Phe Asn Ala
    210                 215                 220

Trp Met Met Leu Phe Leu Asn Ile Leu Glu Arg Pro Val Pro Leu Glu
225                 230                 235                 240

Gly Gln Pro Ala Asp Pro Glu Leu Arg Lys Ser Trp Gly Trp Trp Lys
                245                 250                 255

Val Lys Lys Trp Thr Val His Ile Leu Asn Arg Leu Tyr Thr Arg Phe
            260                 265                 270

Gly Asp Leu Lys Leu Lys Asn Pro Glu Ser Arg Ala Phe Ala Gln Ala
        275                 280                 285

Phe Gln Lys Asn Tyr Ala Gly Lys Val Met Glu Cys His Leu Asn Leu
    290                 295                 300

Leu Asn Val Ile Arg Ser Gly Gly Tyr Leu Pro Asp Arg Val Thr Asn
305                 310                 315                 320

Leu Ile Leu Gln Tyr Leu Ser Asn Ser Ile Ser Lys Asn Ser Met Tyr
                325                 330                 335

Ser Leu Leu Gln Pro Arg Leu Asp Ser Leu Leu Phe Glu Ile Ile Phe
            340                 345                 350

Pro Leu Met Cys Phe Asn Asp Asn Asp Gln Lys Leu Trp Asp Glu Asp
        355                 360                 365

Pro His Glu Tyr Val Arg Lys Gly Tyr Asp Ile Ile Glu Asp Leu Tyr
    370                 375                 380

Ser Pro Arg Thr Ala Ser Met Asp Phe Val Ser Glu Leu Val Arg Lys
385                 390                 395                 400

Arg Gly Lys Glu Asn Leu Gln Lys Phe Ile Gln Phe Ile Val Gly Ile
                405                 410                 415

Phe Asn Arg Tyr Asp Glu Ala Thr Ile Glu Phe Lys Pro Tyr Arg Gln
            420                 425                 430

Lys Asp Gly Ala Leu Leu Ala Ile Gly Ala Leu Cys Asp Lys Leu Lys
        435                 440                 445

Gln Thr Glu Pro Tyr Lys Ser Glu Leu Glu Arg Met Leu Val Gln His
    450                 455                 460

Val Phe Pro Glu Phe Asn Ser Pro Val Gly His Leu Arg Ala Lys Ala

465                 470                 475                 480

Ala Trp Val Ala Gly Gln Tyr Ala His Ile Asn Phe Ala Asp Gln Asn
                485                 490                 495

Asn Phe Arg Lys Ala Leu His Ser Val Val Ala Gly Met Arg Asp Pro
            500                 505                 510

Glu Leu Pro Val Arg Val Asp Ser Val Phe Ala Leu Arg Ser Phe Val
        515                 520                 525

Glu Ala Cys Arg Asp Leu Asn Glu Ile Arg Pro Ile Leu Pro Gln Leu
    530                 535                 540

Leu Asp Glu Phe Phe Lys Leu Met Asn Glu Val Glu Asn Glu Asp Leu
545                 550                 555                 560

Val Phe Thr Leu Glu Thr Ile Val Asp Lys Phe Gly Glu Glu Met Ala
                565                 570                 575

Pro Tyr Ala Leu Gly Leu Cys His Asn Leu Ala Ala Ala Phe Trp Arg
            580                 585                 590

Cys Met Asn Thr Ala Glu Ala Asp Glu Glu Ala Asp Asp Pro Gly Ala
        595                 600                 605

Leu Ala Ala Val Gly Cys Leu Arg Ala Ile Ser Thr Ile Leu Glu Ser
    610                 615                 620

Val Ser Arg Ile Pro Gln Leu Phe Val Gln Ile Glu Pro Thr Leu Leu
625                 630                 635                 640

Pro Ile Met Arg Arg Met Leu Thr Thr Asp Gly Gln Glu Val Phe Glu
                645                 650                 655

Glu Val Leu Glu Ile Val Ser Phe Met Thr Phe Phe Ser Pro Thr Ile
            660                 665                 670

Ser Met Asp Met Trp Ser Leu Trp Pro Leu Met Met Glu Ala Leu Ser
        675                 680                 685

Glu Trp Ala Ile Asp Phe Phe Pro Asn Ile Leu Val Pro Leu Asp Asn
    690                 695                 700

Tyr Val Ser Arg Gly Thr Ala His Phe Leu Thr Cys Lys Ala Pro Asp
705                 710                 715                 720

Tyr Gln Gln Ser Leu Trp Asn Met Ile Ser Ser Ile Met Thr Asp Lys
                725                 730                 735

Asn Leu Glu Asp Gly Asp Ile Glu Pro Ala Pro Lys Leu Ile Gln Val
            740                 745                 750

Val Phe Gln Asn Cys Lys Gly Gln Val Asp Gln Trp Ile Glu Pro Tyr
        755                 760                 765

Leu Arg Ile Thr Ile Asp Arg Leu Gln Arg Thr Glu Lys Ser Tyr Leu
    770                 775                 780

Lys Cys Leu Leu Met Gln Val Ile Ser Asp Ala Leu Tyr Tyr Asn Ala
785                 790                 795                 800

Ser Leu Ser Leu Asn Ile Leu Gln Lys Leu Gly Val Ala Ala Asp Val
                805                 810                 815

Phe Asn Leu Trp Phe Gln Met Leu Gln Gln Val Lys Lys Ser Gly Ile
            820                 825                 830

Arg Val Asn Phe Arg Arg Glu Gln Asp Lys Lys Val Cys Cys Leu Gly
        835                 840                 845

Leu Thr Ser Leu Leu Ala Leu Pro Ala Asp Gln Leu Pro Gly Glu Ala
    850                 855                 860

Leu Gly Arg Val Phe Arg Ala Thr Leu Asp Leu Leu Val Ala Tyr Lys
865                 870                 875                 880

Asp Gln Val Ala Glu Ala Ala Lys Glu Glu Glu Val Glu Glu Asp Asp
                885                 890                 895

Glu Met Asp Gly Tyr Pro Ser Asp Glu Asp Asp Asp Asp Gly Asp Gly
            900                 905                 910

Ser Asp Lys Glu Met Gly Phe Asp Gly Glu Asp Gly Asp Glu Val Asp
        915                 920                 925

Ser Ile Lys Leu Gln Lys Leu Ala Ala Gln Ala Lys Ser Phe Arg Pro
    930                 935                 940

Asp Asp Asp Asp Phe Asp Ser Asp Asp Asp Tyr Ser Asp Asp Glu Glu
945                 950                 955                 960

Met Gln Ser Pro Leu Asp Asp Val Asp Pro Phe Ile Tyr Phe Val Asp
                965                 970                 975

Thr Ile Lys Ala Met Gln Gly Leu Asp Pro Met Arg Phe Gln Ser Leu
            980                 985                 990

Ser Gln Ser Leu Glu Phe Gln Tyr  Gln Ala Leu Ala His  Gly Val Ala
        995                 1000                 1005

Gln His  Ala Glu Gln Arg Arg  Val Glu Ile Glu Lys  Glu Lys Leu
    1010                 1015                 1020

Glu Arg  Ala Ala Ser Ser
    1025

<210>  12
<211>  1026
<212>  PRT
<213>  Glycine max

<400>  12

Met Asp Leu Ala Met Ile Leu Glu Ala Ala Leu Ser Pro Gln Pro Asp
1               5                   10                  15

Glu Arg Lys Gly Ala Glu Gln Arg Leu Asp Glu Met Gln Gln Ala Pro
            20                  25                  30

Gln His Pro Val Ser Leu Phe Gln Ile Ile Val Asp Ser Ser Asn Cys
        35                  40                  45

Asn Met Pro Ile Arg Gln Val Ala Ala Ile His Phe Lys Asn Leu Ile
    50                  55                  60

Ala Lys Asn Trp Thr Lys Ile Ser Leu Asp His Lys Glu Leu Leu Arg
65                  70                  75                  80

Asn His Ile Leu Leu Phe Leu Pro Gln Leu Pro Pro Leu Leu Arg Ser
                85                  90                  95

Gln Leu Gly Glu Cys Leu Lys Thr Ile Ile His Ser Asp Tyr Pro His
            100                 105                 110

His Phe Pro His Leu Leu Asp Trp Ile Ile Leu Asn Leu Gln Asp His
        115                 120                 125

His His Val His Ser Ser Leu Phe Val Leu Arg Ile Leu Ser Arg Lys
    130                 135                 140

Tyr Glu Phe Lys Ser Asp Asp Glu Arg Thr Pro Ile Tyr Arg Val Val
145                 150                 155                 160

Glu Asp Thr Phe Pro Leu Leu Leu Asn Ile Phe Asn Ser Phe Leu Gln
                165                 170                 175

Ile Pro Asn Pro Ser Ile Glu Leu Ala Asp Leu Ile Lys Leu Ile Ser
            180                 185                 190

Lys Ile Phe Trp Ser Ser Ile Tyr Leu Glu Val Pro Lys Val Leu Phe
        195                 200                 205

Asp Gln Asn Val Phe Asn Ala Trp Met Val Leu Phe Leu Asn Val Leu
    210                 215                 220

Glu Arg Pro Val Pro Leu Glu Gly Gln Pro Ala Asp Pro Glu Leu Arg
225                 230                 235                 240

Lys Ser Trp Gly Trp Trp Lys Val Lys Lys Trp Thr Val His Ile Leu
                245                 250                 255

Asn Arg Leu Tyr Thr Arg Phe Gly Asp Leu Lys Leu His Asn Leu Glu
            260                 265                 270

Asn Lys Ala Phe Ala Gln Met Phe Gln Lys His Tyr Ala Gly Lys Ile
        275                 280                 285

Leu Asp Cys Tyr Leu Asn Leu Leu Asn Val Ile Arg Val Gly Gly Tyr
    290                 295                 300

Leu Pro Asp Arg Val Ile Asn Leu Val Leu Gln Tyr Leu Ser Asn Ser
305                 310                 315                 320

Ile Ser Lys Asn Ser Met Tyr Thr Ala Leu Gln Pro Arg Leu Asp Val
                325                 330                 335

Leu Leu Phe Glu Ile Val Phe Pro Leu Met Cys Phe Asn Asp Asn Asp
            340                 345                 350

Gln Lys Leu Trp Asp Glu Asp Pro His Glu Tyr Val Arg Lys Gly Tyr
        355                 360                 365

Asp Ile Ile Glu Asp Leu Tyr Ser Pro Lys Thr Ala Ser Leu Asp Phe
    370                 375                 380

Val Ser Glu Leu Ile Arg Lys Arg Gly Lys Asp Asn Leu His Asn Phe
385                 390                 395                 400

Ile Gln Phe Thr Val Glu Ile Phe Lys Arg Tyr Asp Glu Ala Pro Ala
                405                 410                 415

Glu Tyr Lys Pro Tyr Arg Gln Lys Asp Gly Ala Leu Leu Ala Ile Gly
            420                 425                 430

Ala Leu Cys Asp Lys Leu Lys Gln Thr Glu Pro Tyr Lys Ser Glu Leu
        435                 440                 445

Glu His Met Leu Val Gln His Val Phe Pro Glu Phe Ser Ser Pro Val
    450                 455                 460

Gly His Leu Arg Ala Lys Ala Ala Trp Val Ala Gly Gln Tyr Ala His
465                 470                 475                 480

Ile Asn Phe Ser Asp Gln Asn Asn Phe Arg Lys Ala Leu Gln Cys Val
                485                 490                 495

Val Cys Gly Met Arg Asp Pro Glu Leu Pro Val Arg Ile Asp Ser Val
            500                 505                 510

Phe Ala Leu Arg Ser Phe Val Glu Ala Cys Lys Asp Leu Asn Glu Ile
        515                 520                 525

Arg Pro Ile Leu Pro Gln Leu Leu Asp Glu Phe Phe Lys Leu Met Asn
    530                 535                 540

Glu Ile Glu Asn Glu Asp Leu Val Phe Thr Leu Glu Thr Ile Val Asp
545                 550                 555                 560

Lys Phe Gly Glu Glu Met Ala Pro Tyr Ala Leu Gly Leu Cys Gln Asn
                565                 570                 575

Leu Ala Ala Thr Phe Trp Arg Cys Met Asn Thr Thr Glu Ala Asp Asp
            580                 585                 590

Glu Ala Asp Asp Pro Gly Ala Leu Ala Ala Val Gly Cys Leu Arg Ala
        595                 600                 605

Ile Ser Thr Ile Leu Glu Ser Val Ser Ser Leu Pro Gln Leu Phe Val
    610                 615                 620

Gln Ile Glu Pro Ala Leu Leu Pro Ile Met Arg Arg Met Leu Thr Thr
625                 630                 635                 640

Asp Gly Gln Glu Val Phe Glu Glu Val Leu Glu Ile Val Ser Tyr Met
                645                 650                 655

Thr Phe Phe Ser Pro Thr Ile Ser Leu Asp Met Trp Ser Leu Trp Pro

            660                 665                 670

Leu Met Met Glu Ala Leu Ala Asp Trp Ala Ile Asp Phe Phe Ser Asn
        675                 680                 685

Ile Leu Val Pro Leu Asp Asn Tyr Ile Ser Arg Gly Thr Ala His Phe
    690                 695                 700

Leu Ile Cys Lys Glu Pro Asp Tyr Gln Gln Ser Leu Trp Asn Met Ile
705                 710                 715                 720

Ser Ser Val Met Gly Asp Lys Asn Met Glu Asp Asn Asp Ile Glu Pro
                725                 730                 735

Ala Pro Lys Leu Ile Glu Val Val Phe Leu Asn Cys Lys Gly Gln Val
            740                 745                 750

Asp Gln Trp Val Glu Pro Tyr Leu Arg Ile Thr Val Glu Arg Leu His
        755                 760                 765

Arg Thr Glu Lys Ser His Leu Lys Cys Leu Leu Met Gln Val Ile Ala
    770                 775                 780

Asp Ala Leu Tyr Tyr Asn Ala Pro Leu Thr Leu Ser Ile Leu Gln Lys
785                 790                 795                 800

Leu Gly Val Thr Ser Glu Ile Phe Thr Leu Trp Phe Asn Met Leu Gln
                805                 810                 815

Gly Val Lys Lys Asn Gly Val Arg Ala Asn Phe Lys Arg Glu His Asp
            820                 825                 830

Lys Lys Val Cys Cys Leu Gly Leu Thr Ser Leu Leu Ala Leu Pro Ala
        835                 840                 845

Gly Gln Leu Pro Gly Asp Ala Leu Gly Gln Val Phe Arg Ala Thr Leu
    850                 855                 860

Asp Leu Leu Val Ala Tyr Lys Asp Gln Val Ala Glu Ala Ala Lys Glu
865                 870                 875                 880

Glu Glu Ala Glu Asp Asp Asp Asp Asp Asp Met Asp Asp Phe Gln Thr
                885                 890                 895

Asp Asp Glu Asp Glu Asp Asp Asn Gly Ser Asp Lys Glu Met Gly Ile
            900                 905                 910

Asp Ala Glu Asp Gly Asp Glu Ala Asp Ser Ser Lys Phe Thr Lys Leu
        915                 920                 925

Ala Glu Gln Thr Lys Thr Phe Arg Pro Asn Asp Glu Asp Asp Asp Asp
    930                 935                 940

Ser Asp Asp Asp Phe Ser Asp Asp Glu Glu Leu His Ser Pro Ile Asp
945                 950                 955                 960

Glu Val Asp Pro Phe Val Phe Phe Val Asp Thr Met Lys Val Met Gln
                965                 970                 975

Ser Ser Asp Pro Leu Gly Phe Gln Asn Leu Thr Gln Thr Leu Glu Phe
            980                 985                 990

Ser Tyr Gln Ala Leu Ala Asn Gly  Val Ala Gln His Ala  Glu Leu Arg
        995                 1000                 1005

Arg Gly  Glu Thr Glu Lys Glu  Lys Leu Glu Lys Ser  Ser Ala Thr
    1010                 1015                 1020

Ile Asp  Ser
    1025

<210>  13
<211>  1027
<212>  PRT
<213>  Helianthus annuus

<400>  13

Met Asp Leu Ser Asn Leu Val Ile Val Leu Lys Ala Ala Leu Ser Pro
1               5                   10                  15

Asn Pro Ala Glu Arg Lys Ala Ala Glu Asp Ser Leu Asn Gln Tyr Gln
            20                  25                  30

Tyr Thr Pro Gln His Leu Val Arg Leu Leu Gln Ile Val Val Asp Gly
        35                  40                  45

Asn Cys Asp Met Ala Val Arg Gln Ala Ala Ser Ile His Phe Lys Asn
    50                  55                  60

Phe Ile Ala Lys Asn Trp Ala Pro Leu Asp Pro Glu Glu Gln Cys Lys
65                  70                  75                  80

Ile Leu Pro Ser Asp Lys Asp Val Val Arg Gln Asn Ile Leu Val Phe
                85                  90                  95

Val Ala Gln Leu Pro Pro Leu Leu Arg Ala Gln Leu Gly Glu Cys Leu
            100                 105                 110

Lys Thr Ile Ile His Ala Asp Tyr Pro Glu Gln Trp Pro Gly Leu Leu
        115                 120                 125

His Trp Val Thr His Asn Leu Gln Asp Gln Gln Val Tyr Gly Ala Leu
    130                 135                 140

Phe Val Leu Arg Ile Leu Ala Arg Lys Tyr Glu Phe Lys Ser Asp Glu
145                 150                 155                 160

Glu Arg Ile Pro Val Tyr His Val Val Glu Glu Thr Phe Pro His Leu
                165                 170                 175

Leu Asn Ile Phe Ser Arg Leu Val Gln Ile Ala Asn Pro Ser Ile Glu
            180                 185                 190

Val Ala Asp Leu Ile Lys Leu Ile Cys Lys Ile Phe Trp Ser Ser Ile
        195                 200                 205

Tyr Leu Glu Ile Pro Lys Lys Leu Phe Asp Pro Asn Val Phe Asn Ala
    210                 215                 220

Trp Met Ala Leu Phe Leu Asn Met Leu Glu Arg Pro Val Pro Leu Glu
225                 230                 235                 240

Gly Gln Pro Ile Asp Pro Glu Leu Arg Lys Ser Trp Gly Trp Trp Lys
                245                 250                 255

Val Lys Lys Trp Thr Val His Ile Leu Asn Arg Leu Tyr Thr Arg Phe
            260                 265                 270

Gly Asp Leu Lys Leu Gln Ser Pro Glu Asn Lys Ala Phe Ala Gln His
        275                 280                 285

Phe Gln Lys Asn Tyr Ala Gly Lys Ile Leu Glu Cys His Leu Asn Leu
    290                 295                 300

Leu Asn Ala Ile Arg Thr Gly Asp Tyr Leu Pro Asp Arg Val Thr Asn
305                 310                 315                 320

Leu Ile Leu Gln Tyr Leu Ser Asn Ser Ile Ser Lys Thr Ala Leu Tyr
                325                 330                 335

Asn Leu Leu Gln Pro Arg Leu Asp Val Val Leu Phe Glu Ile Ile Phe
            340                 345                 350

Pro Leu Met Cys Phe Asn Asp Asn Asp Gln Thr Leu Trp Glu Glu Asp
        355                 360                 365

Pro His Glu Tyr Val Arg Lys Gly Tyr Asp Ile Ile Glu Asp Leu Tyr
    370                 375                 380

Ser Pro Arg Thr Ala Ala Met Asp Phe Val Ser Glu Leu Val Arg Lys
385                 390                 395                 400

Arg Gly Lys Glu Asn Leu Gln Lys Phe Ile Leu Phe Ile Val Glu Ile
                405                 410                 415

Phe Lys Arg Tyr Glu Ala Ala Ser Ile Glu Phe Lys Pro Tyr Arg Gln
            420                 425                 430

Lys Asp Gly Ala Leu Leu Ala Ile Gly Ala Leu Cys Asp Lys Leu Lys
        435                 440                 445

His Thr Glu Pro Tyr Lys Ser Glu Leu Glu Pro Met Leu Val Gln His
    450                 455                 460

Val Phe Pro Glu Phe Ala Ser Pro Val Gly His Leu Arg Ala Lys Ala
465                 470                 475                 480

Ala Trp Val Ala Gly Gln Tyr Ala His Ile Asn Phe Ser Asp Ser Asn
                485                 490                 495

Asn Phe Arg Lys Ala Leu Gln Ser Val Val Ala Gly Met Arg Asp Pro
            500                 505                 510

Glu Leu Pro Val Arg Val Asp Ser Val Phe Ala Leu Arg Ser Phe Val
        515                 520                 525

Glu Ser Cys Lys Asp Leu Ser Glu Ile Arg Pro Ile Leu Pro Gln Leu
    530                 535                 540

Leu Asp Asp Phe Phe Lys Leu Met Asn Glu Val Glu Asn Glu Asp Leu
545                 550                 555                 560

Val Phe Thr Leu Glu Thr Ile Val Asp Lys Phe Gly Glu Glu Met Ala
                565                 570                 575

Pro Tyr Ala Leu Gly Leu Cys Gln Asn Leu Ala Ala Ala Phe Trp Lys
            580                 585                 590

Cys Ile Asn Thr Ser Glu Gly Asp Glu Glu Ala Asp Asp Pro Gly Ala
        595                 600                 605

Leu Ala Ala Ala Gly Cys Leu Arg Ala Ile Ser Thr Ile Leu Glu Ser
    610                 615                 620

Val Ser Arg Leu Pro His Leu Phe Ala His Thr Glu Pro Thr Leu Leu
625                 630                 635                 640

Pro Ile Met Arg Arg Met Leu Thr Thr Asp Gly Gln Asp Val Phe Glu
                645                 650                 655

Glu Val Leu Glu Ile Val Ser Tyr Leu Thr Phe Phe Ser Pro Thr Ile
            660                 665                 670

Ser Met Glu Met Trp Ser Leu Trp Pro Leu Leu Leu Glu Ala Leu Ala
        675                 680                 685

Asp Trp Ala Ile Asp Phe Phe Pro Asn Ile Leu Val Pro Leu Asp Asn
    690                 695                 700

Tyr Ile Ser Arg Gly Thr Val His Tyr Leu Thr Cys Lys Glu Pro Asp
705                 710                 715                 720

Tyr Gln Gln Ser Leu Trp Asn Val Leu Ser Asn Ile Met Ser Asp Lys
                725                 730                 735

Asn Leu Glu Asp Asn Asp Ile Glu Pro Ala Pro Lys Leu Ile Glu Val
            740                 745                 750

Val Phe Gln Asn Cys Lys Gly Gln Val Asp His Trp Val Glu Pro Tyr
        755                 760                 765

Ile Arg Leu Thr Val Glu Arg Leu Arg Phe Thr Glu Arg Pro Tyr Leu
    770                 775                 780

Lys Cys Leu Leu Val Gln Val Ile Ala Asn Ala Leu Tyr Tyr Asn Pro
785                 790                 795                 800

Ser Leu Thr Leu Asn Ile Leu Gln Lys Leu Gly Val Ala Thr Glu Val
                805                 810                 815

Phe Asn Leu Trp Phe Gln Met Leu Gln Gln Thr Lys Lys Ser Gly Ala
            820                 825                 830

Arg Ala Asn Phe Lys Arg Glu His Asp Lys Lys Val Cys Cys Leu Gly
        835                 840                 845

Leu Thr Ser Leu Leu Thr Leu Pro Ser Asp Gln Leu Pro Gly Glu Ala

850                 855                 860

Leu Glu Arg Val Phe Lys Ala Thr Leu Glu Leu Leu Val Ala Tyr Lys
865                 870                 875                 880

Asp Gln Val Ala Glu Ala Ala Lys Asp Glu Pro Glu Asp Asp Asp Asp
                885                 890                 895

Met Asp Asp Gly Leu Glu Thr Asp Asp Asp Glu Asp Asp Met Gly Asp
            900                 905                 910

Asp Ala Glu Asp Gly Asp Asp Ala Asp Ser Leu Arg Leu Gln Arg Leu
        915                 920                 925

Ala Ala Gln Ala Lys Ser Phe Arg Pro Ala Asp Asp Asp Asp Asp Asp
    930                 935                 940

Ser Asp Asp Glu Phe Ser Asp Asp Asp Glu Leu Gln Ser Pro Ile Asp
945                 950                 955                 960

Glu Val Asp Pro Phe Ile Phe Phe Met Asp Ile Ala Lys Ala Met Gln
                965                 970                 975

Ala Ser Asp Gln Val Arg Phe Gln Asn Leu Ser Gln Thr Leu Asp Phe
            980                 985                 990

Arg Tyr Gln Ala Leu Ala Thr Gly  Val Ala Gln His Ala  Asp Gln Arg
        995                 1000                1005

Arg Val  Glu Ile Glu Lys Glu  Lys Leu Asp Lys Ala  Asn Ala Ala
    1010                1015                1020

Ala Ala  Ala Ser
    1025

<210>  14
<211>  1033
<212>  PRT
<213>  Helianthus annuus

<400>  14

Met Asp Leu Pro Asn Leu Ala Ile Val Leu Gln Ala Ala Leu Ser Pro
1               5                   10                  15

Asn Pro Ala Glu Arg Lys Ala Ala Glu Asp Ser Leu Asn Gln Tyr Gln
            20                  25                  30

Tyr Thr Pro Gln His Leu Val Arg Leu Leu Gln Ile Thr Val Asp Gly

35                  40                  45

Asn Cys Asp Ile Ala Val Arg Gln Ala Ala Ser Ile His Phe Lys Asn
    50                  55                  60

Phe Ile Ala Lys Asn Trp Ser Pro Leu Asp Pro Asp Glu Gln Ser Lys
65                  70                  75                  80

Ile Leu Pro Ser Asp Lys Asp Leu Val Arg Gln Asn Ile Leu Leu Phe
                85                  90                  95

Val Ala Gln Val Pro Pro Leu Leu Arg Ala Gln Leu Gly Glu Cys Leu
            100                 105                 110

Lys Thr Ile Ile His Ala Asp Tyr Pro Glu Gln Trp Pro Gly Leu Leu
        115                 120                 125

His Trp Val Thr His Asn Leu Gln Asp Gln Gln Gln Val Tyr Gly Ala
    130                 135                 140

Leu Phe Val Leu Arg Ile Leu Ala Arg Lys Tyr Glu Phe Lys Ser Asp
145                 150                 155                 160

Glu Glu Arg Ile Pro Val Tyr Leu Val Val Glu Gln Thr Phe Pro His
                165                 170                 175

Leu Leu Asn Ile Phe Gly Asn Leu Val Gln Ile Gly Asn Pro Ser Ile
            180                 185                 190

Glu Val Ala Asp Leu Val Lys Leu Ile Cys Lys Ile Phe Trp Ser Ser
        195                 200                 205

Ile Tyr Leu Glu Ile Pro Lys Lys Leu Phe Asp Pro Asn Val Phe Asn
    210                 215                 220

Ala Trp Met Val Leu Phe Leu Asn Met Leu Glu Arg Pro Val Pro Leu
225                 230                 235                 240

Glu Gly Gln Pro Ala Asp Pro Glu Leu Arg Lys Ser Trp Gly Trp Trp
                245                 250                 255

Lys Val Lys Lys Trp Thr Val His Ile Leu Asn Arg Leu Tyr Thr Arg
            260                 265                 270

Phe Gly Asp Leu Lys Leu Gln Asn Pro Glu Asn Lys Ala Phe Ala Gln
        275                 280                 285

His Phe Gln Lys Asn Tyr Ala Gly Lys Ile Leu Glu Cys His Leu Asn
    290                 295                 300

Leu Leu Asn Ala Val Arg Thr Gly Asp Tyr Leu Pro Asp Arg Val Thr
305                 310                 315                 320

Asn Leu Ile Leu Gln Tyr Leu Ser Asn Ser Ile Ser Lys Thr Thr Met
                325                 330                 335

Tyr Asn Gln Leu Gln Pro Arg Leu Asp Val Val Leu Phe Glu Ile Ile
            340                 345                 350

Phe Pro Leu Met Cys Phe Asn Asp Asn Asp Gln Thr Leu Trp Glu Glu
        355                 360                 365

Asp Pro His Glu Tyr Val Arg Lys Gly Tyr Asp Ile Ile Glu Asp Leu
    370                 375                 380

Tyr Ser Pro Arg Thr Ala Ala Met Asp Phe Val Ser Glu Leu Val Arg
385                 390                 395                 400

Lys Arg Gly Lys Glu Asn Leu Gln Lys Phe Ile Leu Phe Ile Val Glu
                405                 410                 415

Ile Phe Lys Arg Tyr Glu Ala Ala Pro Val Glu Leu Lys Pro Tyr Arg
            420                 425                 430

Gln Lys Asp Gly Ala Leu Leu Ala Ile Gly Ala Leu Cys Asp Lys Leu
        435                 440                 445

Lys Gln Thr Glu Pro Tyr Lys Ser Glu Leu Glu Pro Met Leu Val Gln
    450                 455                 460

His Val Phe Pro Glu Phe Val Ser Pro Val Gly His Leu Arg Ala Lys
465                 470                 475                 480

Ala Ala Trp Val Ala Gly Gln Tyr Ala His Val Asn Phe Ser Asp Pro
                485                 490                 495

Asn Asn Phe Arg Lys Ala Leu Gln Cys Val Val Ala Gly Met Arg Asp
            500                 505                 510

Pro Glu Leu Pro Val Arg Val Asp Ser Val Phe Ala Leu Arg Ser Phe
        515                 520                 525

Val Glu Ser Cys Lys Asp Leu Gly Glu Ile Arg Pro Ile Leu Pro Gln
    530                 535                 540

Leu Leu Asp Asp Phe Phe Lys Leu Met Asp Glu Val Glu Asn Glu Asp
545                 550                 555                 560

Leu Val Phe Thr Leu Glu Thr Ile Val Asp Lys Phe Gly Glu Glu Met
                565                 570                 575

Ala Pro Tyr Ala Leu Gly Leu Cys Gln Ser Leu Ala Ala Ala Phe Trp
            580                 585                 590

Lys Cys Ile Asn Thr Cys Glu Gly Asp Glu Glu Ala Asp Asp Pro Gly
        595                 600                 605

Ala Leu Ala Ala Ala Gly Cys Leu Arg Ala Ile Ser Thr Ile Leu Glu
    610                 615                 620

Ser Val Ser Arg Leu Pro His Ile Phe Ala His Ile Glu Pro Thr Leu
625                 630                 635                 640

Leu Pro Ile Met Arg Arg Met Leu Thr Thr Asp Gly Gln Asp Val Phe
                645                 650                 655

Glu Glu Val Leu Glu Ile Val Ser Tyr Met Thr Phe Phe Ser Pro Thr
            660                 665                 670

Ile Ser Met Asp Met Trp Ser Leu Trp Pro Leu Leu Ile Glu Ala Leu
        675                 680                 685

Ala Asp Trp Ala Ile Asp Phe Phe Pro Asn Ile Leu Val Pro Leu Asp
    690                 695                 700

Asn Tyr Ile Ser Arg Ser Thr Val His Tyr Leu Thr Cys Lys Glu Pro
705                 710                 715                 720

Asp Tyr Gln Gln Ser Leu Trp Asn Met Leu Ser Asn Ile Met Ser Asp
                725                 730                 735

Lys Asn Leu Glu Asp Asn Asp Ile Glu Pro Ala Pro Lys Leu Ile Glu
            740                 745                 750

Val Val Phe Gln Asn Cys Lys Gly Gln Val Asp His Trp Val Glu Pro
        755                 760                 765

Tyr Ile Arg Leu Thr Val Glu Arg Leu Arg Val Ala Glu Arg Pro Tyr
    770                 775                 780

Leu Lys Cys Leu Leu Val Gln Val Ile Ala Asp Ala Leu Tyr Tyr Asn
785                 790                 795                 800

Ala Ser Leu Thr Leu Asn Val Leu Gln Lys Leu Gly Val Ala Thr Glu
                805                 810                 815

Val Phe Asn Leu Trp Phe Gln Met Leu Gln Gln Thr Lys Arg Ser Gly
            820                 825                 830

Gly Arg Ala Asn Phe Lys Arg Glu His Asp Lys Lys Val Cys Cys Leu
        835                 840                 845

Gly Leu Thr Ser Leu Leu Thr Leu Ser Ala Asp Gln Leu Pro Gly Glu
    850                 855                 860

Ala Leu Glu Arg Val Phe Lys Ala Thr Leu Glu Leu Leu Val Ala Tyr
865                 870                 875                 880

Lys Asp Gln Val Ala Glu Ala Ala Lys Glu Gly Ser Glu Asp Asp Asp
                885                 890                 895

Gly Met Asn Asp Asp Leu Glu Thr Asp Asp Asp Glu Asp Asp Gly Ser
            900                 905                 910

Asp Lys Asp Met Gly Val Asp Ala Glu Asp Gly Asp Glu Ala Asp Ser
        915                 920                 925

Leu Arg Leu Gln Arg Leu Ala Ala Gln Ala Lys Ser Phe Arg Pro Ala
    930                 935                 940

Asp Asp Asp Asp Asp Asp Asp Asp Asp Asp Phe Ser Asp Asp Glu Asp
945                 950                 955                 960

Leu Gln Ser Pro Ile Asp Glu Val Asp Pro Phe Val Phe Phe Met Asp
                965                 970                 975

Thr Ala Lys Ala Leu Gln Ala Ser Asp Gln Ile Arg Phe Gln Asn Leu
            980                 985                 990

Ser Gln Thr Leu Asp Phe His Tyr  Gln Ala Leu Ala Ser  Gly Ile Ser
        995                 1000                1005

Gln His  Ala Asp Gln Arg Arg  Val Glu Ile Glu Lys  Glu Lys Leu
    1010                1015                1020

Glu Lys  Ala Ser Ala Ser Ala  Pro Ala Ser
    1025                1030

<210>  15

<211>  1026
<212>  PRT
<213>  Helianthus annuus

<400>  15

Met Asp Leu Gln Ser Leu Ala Ile Val Leu Gln Ala Ala Leu Thr Pro
1               5                   10                  15

Asn Pro Ala Glu Arg Lys Ala Ala Glu Asp Thr Leu Thr Lys Phe Gln
            20                  25                  30

Tyr Thr Pro Gln His Leu Val Arg Leu Leu Gln Ile Ile Val Asp Gly
        35                  40                  45

Asn Cys Asp Leu Ala Val Arg Gln Val Ala Ser Ile His Phe Lys Asn
    50                  55                  60

Phe Ile Ala Glu Asn Trp Ser Pro His Asp Pro Asp Glu Gln Ser Lys
65                  70                  75                  80

Ile Leu Pro Ser Asp Lys Asp Leu Val Arg Gln Asn Ile Leu Val Phe
                85                  90                  95

Val Ala Gln Val Pro Ala Leu Leu Arg Ala Gln Leu Gly Glu Cys Leu
            100                 105                 110

Lys Thr Ile Ile His Ala Asp Tyr Pro Glu Gln Trp Pro Ala Leu Leu
        115                 120                 125

Gln Trp Val Thr His Asn Leu Gln Asp Gln Gln Val Tyr Ala Ala Leu
    130                 135                 140

Phe Val Leu Arg Ile Leu Ser Arg Lys Tyr Glu Phe Lys Ser Asp Glu
145                 150                 155                 160

Glu Arg Thr Pro Ile His His Val Val Glu Glu Thr Phe Pro His Leu
                165                 170                 175

Leu Asn Ile Phe Ser Arg Leu Val Gln Ile Gly Asn Pro Ser Ile Glu
            180                 185                 190

Ile Ala Gly Leu Ile Lys Leu Ile Cys Lys Ile Tyr Trp Ser Ser Ile
        195                 200                 205

Tyr Leu Glu Ile Pro Lys Lys Leu Tyr Asp Pro Asn Val Phe Asp Ala
    210                 215                 220

Trp Met Val Leu Phe Leu Asn Ile Leu Glu Arg Pro Val Pro Leu Glu

225                 230                 235                 240

Gly Gln Pro Thr Asp Pro Glu Leu Arg Lys Ser Trp Gly Trp Trp Lys
                245                 250                 255

Val Lys Lys Trp Thr Ala His Ile Leu Asn Arg Leu Tyr Thr Arg Phe
            260                 265                 270

Gly Asp Leu Lys Leu Gln Asn Pro Glu Ser Lys Ala Phe Ala Gln His
        275                 280                 285

Phe Gln Lys Asn Tyr Ala Ala Lys Ile Leu Glu Cys His Leu Asn Leu
    290                 295                 300

Leu Asn Ala Val Arg Val Gly Gly Tyr Leu Pro Glu Arg Val Ala Asn
305                 310                 315                 320

Leu Ser Leu Gln Tyr Leu Ser Asn Ser Leu Ala Lys Ser Thr Thr Tyr
                325                 330                 335

Ser Leu Leu Gln Thr Arg Leu Asp Val Val Leu Phe Glu Ile Ile Phe
            340                 345                 350

Pro Leu Met Cys Phe Asn Asp Asp Asp Gln Lys Leu Trp Glu Glu Asp
        355                 360                 365

Pro His Glu Tyr Val Arg Lys Gly Tyr Asp Ile Ile Glu Asp Leu Tyr
    370                 375                 380

Ser Ser Arg Thr Ala Ala Met Asp Phe Val Thr Glu Leu Val Arg Lys
385                 390                 395                 400

Arg Gly Lys Glu Asn Leu Gln Lys Phe Ile Leu Phe Ile Val Glu Ile
                405                 410                 415

Phe Lys Arg Tyr Glu Gln Ala Pro Ile Glu Ile Lys Pro Tyr Arg Gln
            420                 425                 430

Lys Asp Gly Ala Leu Leu Ala Ile Gly Thr Leu Cys Asp Lys Leu Lys
        435                 440                 445

Gln Thr Glu Pro Tyr Lys Ser Glu Leu Glu Pro Met Leu Val Gln His
    450                 455                 460

Val Phe Pro Glu Phe Thr Ser Pro Met Gly His Ile Arg Ala Lys Ala
465                 470                 475                 480

Ala Trp Val Ala Gly Gln Tyr Ala His Ile Asn Phe Ser Asp Pro Asn
                485                 490                 495

Asn Phe Arg Lys Ala Leu Gln Ser Val Val Ala Gly Met Arg Asp Pro
            500                 505                 510

Glu Leu Pro Val Arg Val Asp Ser Val Phe Ala Leu Arg Ser Phe Val
        515                 520                 525

Glu Ala Cys Thr Asp Leu Gly Glu Ile Arg Pro Ile Leu Pro Gln Leu
    530                 535                 540

Leu Asp Glu Phe Phe Lys Leu Met Asn Glu Val Glu Asn Glu Asp Leu
545                 550                 555                 560

Val Phe Thr Leu Glu Thr Ile Val Asp Lys Phe Gly Glu Glu Met Ala
                565                 570                 575

Pro Tyr Ala Val Gly Leu Cys Gln Asn Leu Ala Ala Thr Phe Trp Lys
            580                 585                 590

Cys Met Asn Thr Glu Ala Asp Asp Glu Ala Asp Asp Pro Gly Ala Leu
        595                 600                 605

Ala Ala Val Gly Cys Leu Arg Ala Ile Ser Thr Ile Leu Glu Ser Val
    610                 615                 620

Ser Lys Leu Pro His Leu Phe Ala His Val Glu Pro Thr Leu Leu Pro
625                 630                 635                 640

Ile Met Arg Arg Met Leu Thr Thr Asp Gly Gln Glu Val Phe Glu Glu
                645                 650                 655

Val Leu Glu Ile Ala Ser Tyr Met Thr Phe Phe Ser Pro Thr Ile Ser
            660                 665                 670

Met Asp Met Trp Ser Leu Trp Pro Leu Leu Met Glu Ala Leu Ala Glu
        675                 680                 685

Trp Ala Ile Asp Phe Phe Ser Asn Ile Leu Val Pro Leu Asp Asn Tyr
    690                 695                 700

Ile Ser Arg Ser Thr Val His Tyr Leu Thr Cys Lys Glu Pro Asp Tyr
705                 710                 715                 720

Gln Gln Ser Leu Trp Asn Met Leu Ser Asn Ile Met Asn Asp Lys Asn
                725                 730                 735

Leu Glu Asp Asn Asp Ile Asp Pro Ala Pro Lys Leu Ile Ala Val Val
            740                 745                 750

Leu Gln Asn Cys Arg Gly Gln Val Asp Gln Trp Val Glu Pro Tyr Ile
        755                 760                 765

Arg Ile Thr Val Glu Arg Leu His Arg Thr Glu Arg Pro Phe Leu Lys
    770                 775                 780

Cys Leu Leu Met Gln Val Ile Ala Asp Ala Leu Tyr Tyr Asn Pro Ser
785                 790                 795                 800

Leu Thr Leu Asn Ile Leu Gln Lys Leu Gly Val Ala Thr Glu Ile Phe
                805                 810                 815

Asn Leu Trp Phe Gln Met Leu Gln Gln Thr Lys Lys Asn Gly Val Arg
            820                 825                 830

Val Asn Phe Lys Arg Glu Asn Asp Lys Lys Ile Cys Cys Leu Gly Leu
        835                 840                 845

Thr Ser Leu Leu Ser Leu Ser Ala Asp Gln Leu Pro Leu Glu Ala Leu
    850                 855                 860

Glu Arg Val Phe Lys Ala Thr Leu Asp Leu Leu Val Ala Tyr Lys Asp
865                 870                 875                 880

Gln Leu Thr Glu Ala Glu Lys Glu Ala Pro Glu Asp Asp Asp Asp Asp
                885                 890                 895

Asp Asp Asp Asp Met Asn Asp Ser Leu Gln Thr Asp Asp Asp Asp Tyr
            900                 905                 910

Asp Asp Asp Asp Glu Asp Gly Asp Glu Ala Asn Ser Ser Arg Leu Gln
        915                 920                 925

Lys Leu Gly Ala Gln Glu Arg Val Phe Arg Ser Thr Asp Asp Tyr Asp
    930                 935                 940

Asp Asp Ser Asp Asp Asp Phe Ser Asp Asp Glu Asp Leu Gln Ser Pro
945                 950                 955                 960

Ile Asp Asp Val Asp Pro Phe Ile Leu Phe Val Asp Thr Val Lys Val
                965                 970                 975

Met Gln Ala Ser Asp Pro Thr Arg Phe Gln Asn Leu Ser Gln Thr Leu
            980                 985                 990

Asp Phe Arg Tyr Gln Ala Leu Ala Asn Gly Val Ala Gln His Ala Asp
        995                 1000                1005

Gln Arg Arg Ala Val Ile Glu Lys Glu Lys Leu Glu Lys Ala Ser
    1010                1015                1020

Val Ala Ser
    1025

<210> 16
<211> 1036
<212> PRT
<213> Solanum lycopersicum

<400> 16

Met Asp Phe Gln Asn Leu Ala Val Ile Leu Ala Gly Ala Leu Ser Pro
1               5                   10                  15

Asn Pro Asp Glu Arg Lys Ala Ala Glu Asn Ser Leu Asn Gln Phe Gln
            20                  25                  30

His Thr Pro Gln His Leu Val Arg Leu Leu Gln Ile Ile Val Asp Gly
        35                  40                  45

Ser Cys Asp Met Ala Val Arg Gln Val Ala Ser Ile His Phe Lys Asn
    50                  55                  60

Phe Val Ala Lys Asn Trp Cys Pro His Asp Pro Ala Glu Gln Ser Lys
65                  70                  75                  80

Ile Met Pro Ser Asp Lys Glu Leu Val Arg Gln Asn Ile Leu Ile Phe
                85                  90                  95

Ile Ala Gln Val Pro Ser Leu Leu Arg Val Gln Leu Gly Glu Cys Ile
            100                 105                 110

Lys Thr Met Ile His Ala Asp Tyr Pro Glu Gln Trp Pro Thr Leu Leu
        115                 120                 125

Pro Trp Ile Lys His Asn Leu Gln Asp Gln Gln Val Tyr Gly Ala Leu
    130                 135                 140

Phe Val Leu Arg Ile Leu Ser Arg Lys Tyr Glu Phe Lys Ser Asp Glu
145                 150                 155                 160

Glu Arg Thr Pro Val Tyr His Val Val Glu Glu Thr Phe Pro His Leu
                165                 170                 175

Leu Asn Ile Phe Asn Lys Leu Val Gln Ile Thr Asn Pro Ser Ile Glu
            180                 185                 190

Val Ala Asp Leu Ile Lys Leu Ile Cys Lys Ile Phe Trp Ser Ser Ile
        195                 200                 205

Tyr Leu Glu Ile Pro Lys Gln Leu Phe Asp Pro Asn Val Phe Asn Ala
    210                 215                 220

Trp Met Val Leu Phe Leu Asn Met Leu Glu Arg Pro Val Pro Val Glu
225                 230                 235                 240

Gly Gln Pro Ala Asp Pro Glu Leu Arg Lys Ser Trp Gly Trp Trp Lys
                245                 250                 255

Val Lys Lys Trp Thr Val His Ile Leu Asn Arg Leu Tyr Thr Arg Phe
            260                 265                 270

Gly Asp Leu Lys Leu Gln Asn Pro Asp Asn Lys Ala Phe Ala Gln Met
        275                 280                 285

Phe Gln Lys Gly Tyr Ala Gly Lys Ile Leu Glu Cys His Leu Asn Leu
    290                 295                 300

Leu Asn Val Ile Arg Ala Gly Gly Tyr Leu Pro Asp Arg Val Ile Asn
305                 310                 315                 320

Leu Ile Leu Gln Tyr Leu Ser Asn Ser Ile Ser Lys Ser Asn Met Tyr
                325                 330                 335

Ser Leu Leu Gln Pro Arg Leu Asp Ile Val Leu Phe Glu Ile Ile Phe
            340                 345                 350

Pro Leu Met Cys Phe Ser Asp Asn Asp Gln Lys Leu Trp Glu Glu Asp
        355                 360                 365

Pro His Glu Tyr Val Arg Lys Gly Tyr Asp Ile Ile Glu Asp Leu Tyr
    370                 375                 380

Ser Pro Arg Thr Ala Ala Met Asp Phe Val Ser Glu Leu Val Arg Lys
385                 390                 395                 400

Arg Gly Lys Glu Asn Leu His Lys Phe Leu Phe Phe Ile Val Glu Ile
                405                 410                 415

Phe Lys Arg Tyr Gln Glu Ala Ala Pro Glu Tyr Lys Pro Tyr Arg Gln

420                 425                 430

Lys Asp Gly Ala Leu Leu Ala Ile Gly Ala Leu Cys Asp Lys Leu Lys
        435                 440                 445

Gln Thr Glu Pro Tyr Lys Ser Glu Leu Glu His Met Leu Val Gln His
    450                 455                 460

Val Phe Pro Glu Phe Ser Ser Pro Val Gly His Leu Arg Ala Lys Ala
465                 470                 475                 480

Ala Trp Val Ala Gly Gln Tyr Ala His Ile Asn Phe Ala Asp Pro Asn
                485                 490                 495

Asn Phe Arg Asn Ala Leu His Ser Val Val Thr Gly Met Arg Asp Pro
            500                 505                 510

Asp Leu Pro Val Arg Val Asp Ser Val Phe Ala Leu Arg Ser Phe Ile
        515                 520                 525

Glu Ala Cys Lys Asp Leu Asn Glu Ile Arg Pro Ile Leu Pro Gln Leu
    530                 535                 540

Leu Asp Glu Phe Phe Lys Leu Met Asn Glu Val Glu Asn Glu Asp Leu
545                 550                 555                 560

Val Phe Thr Leu Glu Thr Ile Val Asp Lys Phe Gly Glu Glu Met Ala
                565                 570                 575

Pro Tyr Ala Leu Gly Leu Cys Gln Asn Leu Ala Ala Ala Phe Trp Lys
            580                 585                 590

Cys Met Asn Ser Ala Glu Ala Glu Glu Glu Ala Asp Asp Pro Gly Ala
        595                 600                 605

Leu Ala Ala Val Gly Cys Leu Arg Ala Ile Ser Thr Ile Leu Glu Ser
    610                 615                 620

Val Ser Arg Leu Pro His Leu Phe Ile His Ile Glu Pro Thr Leu Leu
625                 630                 635                 640

Pro Ile Met Arg Arg Met Leu Thr Thr Asp Gly Gln Glu Val Phe Glu
                645                 650                 655

Glu Val Leu Glu Ile Val Ser Tyr Met Thr Phe Phe Ser Pro Thr Ile
            660                 665                 670

Ser Met Asp Met Trp Thr Leu Trp Pro Leu Met Met Glu Ala Leu Ala
        675                 680                 685

Asp Trp Ala Ile Asp Phe Phe Pro Asn Ile Leu Val Pro Leu Asp Asn
    690                 695                 700

Tyr Ile Ser Lys Ser Thr Ala His Phe Leu Thr Cys Lys Asp Pro Asp
705                 710                 715                 720

Tyr Gln Gln Ser Leu Trp Asn Met Ile Ser Ser Val Met Gly Asp Lys
                725                 730                 735

Asn Leu Glu Asp Gly Asp Ile Glu Ser Ala Pro Lys Leu Ile Gln Val
            740                 745                 750

Val Phe Glu His Cys Lys Gly Gln Val Asp His Trp Val Glu Pro Tyr
        755                 760                 765

Ile Arg Leu Thr Val Glu Arg Leu Arg Arg Ala Glu Lys Ser His Leu
    770                 775                 780

Lys Cys Leu Leu Val Gln Val Ile Ala Asp Ala Leu Tyr Tyr Asn Ala
785                 790                 795                 800

Pro Leu Thr Trp Asn Ile Leu Gln Lys Leu Gly Leu Ala Leu Glu Val
                805                 810                 815

Phe Asn Leu Trp Phe Leu Met Leu Gly Gln Thr Lys Lys Ser Gly Lys
            820                 825                 830

Arg Val Asn Phe Lys Arg Glu His Asp Lys Lys Val Cys Cys Leu Gly
        835                 840                 845

Leu Thr Ser Leu Leu Pro Leu Pro Val Asp Gln Phe Pro Arg Glu Ala
    850                 855                 860

Leu Asp Arg Val Phe Lys Ala Thr Leu Asp Leu Leu Ile Ala Tyr Lys
865                 870                 875                 880

Asp Gln Val Ala Glu Ala Ala Lys Glu Asp Glu Ala Glu Asp Asp Asp
                885                 890                 895

Asp Met Asn Gly Leu Gln Thr Asp Glu Asp Asp Asp Glu Asp Asp Glu
            900                 905                 910

Ser Asp Lys Glu Met Gly Asp Asp Ala Glu Glu Gly Asp Glu Ala Asp
        915                 920                 925

Ser Thr Arg Leu Gln Lys Leu Ala Ala Gln Ala Lys Ala Phe Arg Ser
    930                 935                 940

His Asp Glu Asp Asp Asp Asp Asp Asp Ser Asp Asp Asp Phe Ser Asp
945                 950                 955                 960

Asp Asp Glu Glu Met Gln Ser Pro Leu Asp Glu Val Asp Pro Phe Ile
                965                 970                 975

Phe Phe Val Glu Thr Ile Lys Ala Met Gln Ala Ser Asp Pro Val Lys
            980                 985                 990

Phe Gln Ser Leu Thr Gln Thr Leu  Asp Phe Arg Tyr Gln  Ala Leu Ala
        995                 1000                1005

Asn Gly  Val Ala Val His Ala  Glu Glu Arg Arg Ala  Glu Ile Glu
    1010                1015                1020

Lys Glu  Lys Leu Glu Lys Ala  Ser Ala Val Val Ala  Ser
    1025                1030                1035

<210>  17
<211>  1037
<212>  PRT
<213>  Sorghum bicolor

<400>  17

Met Asp Leu Pro Ser Leu Ala Val Val Leu Arg Ala Ala Leu Ser His
1               5                   10                  15

Val Pro Glu Glu Arg Lys Ala Ala Glu Glu Ser Leu Asn Gln Phe Gln
            20                  25                  30

Tyr Thr Pro Gln His Leu Val Arg Leu Leu Gln Ile Ile Val Asp Gly
        35                  40                  45

Ser Cys Asp Met Ala Val Arg Gln Val Ala Ser Ile His Phe Lys Asn
    50                  55                  60

Phe Ile Ala Lys Asn Trp Ser Pro Asn Asp Pro Asp Glu Ser Pro Lys
65                  70                  75                  80

Val Leu Glu Ser Asp Lys Ala Met Val Arg Glu Asn Ile Leu Gly Phe
                85                  90                  95

Ile Val Gln Val Pro Pro Leu Leu Arg Ala Gln Leu Gly Glu Ser Ile
            100                 105                 110

Lys Thr Ile Ile His Ser Asp Tyr Pro Glu Gln Trp Pro Ser Leu Leu
        115                 120                 125

His Trp Val Ser His Asn Leu Asp Leu Gln Asn Gln Ile Phe Gly Ala
    130                 135                 140

Leu Tyr Val Leu Arg Val Leu Ala Arg Lys Tyr Glu Phe Lys Ser Glu
145                 150                 155                 160

Asp Glu Arg Ile Pro Leu Tyr Arg Ile Val Glu Glu Thr Phe Pro Arg
                165                 170                 175

Leu Leu Ser Ile Phe Ser Lys Leu Val Gln Ile Val Asn Pro Pro Ile
            180                 185                 190

Glu Val Ala Asp Leu Ile Lys Leu Ile Cys Lys Ile Phe Trp Ser Ser
        195                 200                 205

Ile Tyr Leu Glu Ile Pro Lys Gln Leu Phe Asp Pro Asn Val Phe Asn
    210                 215                 220

Ala Trp Met Val Leu Phe Ile Asn Leu Leu Glu Arg Pro Val Pro Val
225                 230                 235                 240

Glu Gly Gln Pro Ile Asp Pro Glu Ile Arg Lys Ser Trp Ala Trp Trp
                245                 250                 255

Lys Val Lys Lys Trp Thr Ile His Ile Leu Asn Arg Leu Tyr Thr Arg
            260                 265                 270

Phe Gly Asp Leu Lys Leu Gln Lys Pro Glu Ser Lys Ala Phe Ala Gln
        275                 280                 285

Met Phe Gln Lys Thr Tyr Ala Gly Lys Ile Leu Ala Cys His Met Gln
    290                 295                 300

Leu Leu Asn Ala Ile Arg Ser Gly Asp Tyr Leu Pro Asp Arg Val Val
305                 310                 315                 320

Asn Leu Val Leu Gln Tyr Leu Thr Asn Ser Val Thr Lys Asn Ser Met
                325                 330                 335

Tyr Gln Met Met Gln Pro Gln Ile Asp Ile Leu Leu Phe Glu Ile Ile
            340                 345                 350

Phe Pro Leu Met Cys Phe Asn Asp Asn Asp Gln Lys Leu Trp Glu Glu
        355                 360                 365

Asp Pro His Glu Tyr Val Arg Lys Gly Tyr Asp Ile Ile Glu Asp Leu
    370                 375                 380

Tyr Ser Pro Arg Thr Ala Ala Met Asp Phe Val Ser Glu Leu Val Arg
385                 390                 395                 400

Lys Arg Gly Lys Ser Asn Leu Gln Lys Phe Ile His Phe Ile Val Asp
                405                 410                 415

Ile Phe Arg Arg Tyr Asp Glu Ala Ser Ala Asp Leu Lys Pro Tyr Arg
            420                 425                 430

Gln Lys Asp Gly Ala Leu Leu Ala Ile Gly Thr Leu Cys Asp Lys Leu
        435                 440                 445

Lys Gln Thr Asp Pro Tyr Lys Ser Glu Leu Glu Arg Met Leu Val Gln
    450                 455                 460

His Val Phe Pro Glu Phe Thr Ser Arg Val Gly His Leu Arg Ala Lys
465                 470                 475                 480

Ala Ala Trp Val Ala Gly Gln Tyr Ala His Ile Asn Phe Ser Asp Pro
                485                 490                 495

Asn Asn Phe Arg Gln Ala Met His Cys Ile Val Ser Gly Met Arg Asp
            500                 505                 510

Pro Asp Leu Pro Val Arg Val Asp Ser Val Phe Ala Leu Arg Ser Phe
        515                 520                 525

Val Glu Ala Cys Lys Asp Leu Asn Glu Ile Arg Pro Ile Leu Pro Gln
    530                 535                 540

Leu Leu Asp Glu Phe Phe Lys Leu Met Asn Glu Val Glu Asn Glu Asp
545                 550                 555                 560

Leu Val Phe Thr Leu Glu Thr Ile Val Asp Lys Phe Gly Glu Glu Met
                565                 570                 575

Ala Pro Tyr Ala Leu Gly Leu Cys Gln Asn Leu Ala Ala Ala Phe Trp
            580                 585                 590

Arg Cys Met Ala Ser Ser Glu Thr Asp Asp Glu Ala Asp Asp Ser Gly
        595                 600                 605

Ala Leu Ala Ala Val Gly Cys Leu Arg Ala Ile Ser Thr Ile Leu Glu

610                 615                 620

Ser Ile Ser Ser Leu Pro His Leu Phe Met Gln Ile Glu Pro Thr Leu
625                 630                 635                 640

Leu Pro Ile Met Arg Arg Met Leu Thr Ser Asp Gly Gln Asp Val Tyr
                645                 650                 655

Glu Glu Val Leu Glu Ile Val Ser Tyr Met Thr Phe Phe Ser Pro Thr
            660                 665                 670

Ile Ser Leu Asp Met Trp Ser Leu Trp Pro Leu Met Met Glu Ala Leu
        675                 680                 685

Asn Asp Trp Ala Ile Asp Phe Phe Glu Asn Ile Leu Val Pro Leu Asp
    690                 695                 700

Asn Tyr Ile Ser Arg Gly Thr Asp His Phe Leu Glu Cys Lys Asp Pro
705                 710                 715                 720

Asp Tyr Gln Gln Ser Leu Trp Asn Ala Leu Gln Ser Ile Met Met Asp
                725                 730                 735

Glu Asn Met Glu Asp Ser Asp Ile Glu Pro Ala Pro Lys Leu Ile Glu
            740                 745                 750

Val Val Phe Gln Asn Cys Lys Gly Asn Val Asp Gln Trp Val Glu His
        755                 760                 765

Tyr Leu Arg Ile Thr Ile Glu Arg Leu Arg Arg Ala Lys Lys Pro Tyr
    770                 775                 780

Leu Lys Cys Leu Leu Val Gln Val Ile Ala Asn Ala Leu Tyr Tyr Asn
785                 790                 795                 800

Pro Ala Leu Thr Leu Glu Thr Leu Asn Lys Leu Gly Val Ala Ala Asp
                805                 810                 815

Ile Phe Asn His Trp Phe Ala Met Leu Gln Gln Val Lys Lys Ser Gly
            820                 825                 830

Ala Arg Val Asn Phe Lys Arg Glu His Asp Lys Lys Val Cys Cys Leu
        835                 840                 845

Gly Leu Thr Ser Leu Ile Ala Leu Pro Ala Asp Lys Ile Pro Ala Glu
    850                 855                 860

Ala Leu Asp Arg Ile Phe Lys Ala Thr Leu Gly Leu Phe Phe Pro Ser
865                 870                 875                 880

Lys Leu Cys Leu Cys Phe Phe Ser Pro Gly Glu Tyr Thr Glu Ala Lys
                885                 890                 895

Lys Gln Asn Glu Glu Gly Ala Asp Asp Met Asp Gly Phe Asp Ala Asp
            900                 905                 910

Glu Glu Asp Asp Asp Glu Val Asp Ser Asp Lys Glu Met Gly Leu Asp
        915                 920                 925

Asp Glu Asp Gly Asp Glu Val Ser Ser Leu Gln Leu Gln Lys Leu Ala
    930                 935                 940

Ala Glu Ala Arg Gly Phe Gln Pro Ala Asp Glu Asp Asp Asp Ser Asp
945                 950                 955                 960

Asp Asp Phe Ser Asp Asp Glu Glu Leu His Ser Pro Ile Asp Glu Val
                965                 970                 975

Asp Pro Phe Ile Phe Phe Val Glu Thr Val Gln Gly Leu Gln Ala Ser
            980                 985                 990

Asp Pro Ala Arg Phe Gln Asn Leu  Met Gln Thr Leu Asp  Phe Arg Tyr
        995                 1000                1005

Gln Ala  Leu Ala Ser Gly Ile  Ala Gln His Ala Glu  Glu Arg Lys
    1010                1015                1020

Ile Glu  Ile Glu Lys Glu Lys  Ser Glu Lys Ala Asn  Ala Gln
    1025                1030                1035

<210>  18
<211>  1027
<212>  PRT
<213>  Zea mays

<400>  18

Met Asp Leu Pro Ser Leu Ala Val Val Leu Arg Ala Ala Leu Ser His
1               5                   10                  15

Val Pro Glu Glu Arg Lys Ala Ala Glu Glu Ser Leu Asn Gln Phe Gln
            20                  25                  30

Tyr Thr Pro Gln His Leu Val Arg Leu Leu Gln Ile Ile Val Asp Gly
        35                  40                  45

Ser Cys Asp Met Ala Val Arg Gln Val Ala Ser Ile His Phe Lys Asn
    50                  55                  60

Phe Ile Ala Lys Asn Trp Ser Pro Asn Asp Pro Asp Glu Ser Pro Lys
65                  70                  75                  80

Val Leu Glu Ser Asp Lys Ala Met Val Arg Glu Asn Ile Leu Gly Phe
                85                  90                  95

Ile Val Gln Val Pro Pro Leu Leu Arg Ala Gln Leu Gly Glu Ser Ile
            100                 105                 110

Lys Thr Ile Val His Ser Asp Tyr Pro Glu Gln Trp Pro Ser Leu Leu
        115                 120                 125

His Trp Val Ser His Asn Leu Asp Leu Gln Asn Gln Ile Phe Gly Ala
    130                 135                 140

Leu Tyr Val Leu Arg Val Leu Ala Arg Lys Tyr Glu Phe Lys Ser Glu
145                 150                 155                 160

Asp Glu Arg Ile Pro Leu Phe His Ile Val Glu Glu Thr Phe Pro Arg
                165                 170                 175

Leu Leu Ser Ile Phe Ser Lys Leu Val Gln Ile Val Asn Pro Pro Ile
            180                 185                 190

Glu Val Ala Asp Leu Ile Lys Leu Ile Cys Lys Ile Phe Trp Ser Ser
        195                 200                 205

Ile Tyr Leu Glu Ile Pro Lys Gln Leu Phe Asp Pro Asn Val Phe Asn
    210                 215                 220

Ala Trp Met Val Leu Phe Ile Asn Leu Leu Glu Arg Pro Val Pro Val
225                 230                 235                 240

Glu Gly Gln Pro Ile Asp Pro Glu Ile Arg Lys Ser Trp Ala Trp Trp
                245                 250                 255

Lys Val Lys Lys Trp Thr Ile His Ile Leu Asn Arg Leu Tyr Thr Arg
            260                 265                 270

Phe Gly Asp Leu Lys Leu Gln Lys Pro Glu Ser Lys Ala Phe Ala Gln
        275                 280                 285

Met Phe Gln Lys Thr Tyr Ser Gly Lys Ile Leu Ala Cys His Met Gln
    290                 295                 300

Leu Leu Asn Ala Ile Arg Gly Gly Asp Tyr Leu Pro Asp Arg Val Ile
305                 310                 315                 320

Asn Leu Val Leu Gln Tyr Leu Thr Asn Ser Val Thr Lys Asn Ser Met
                325                 330                 335

Tyr Gln Met Met Gln Pro Gln Ile Asp Ile Leu Leu Phe Glu Ile Ile
            340                 345                 350

Phe Pro Leu Met Cys Phe Asn Asp Asn Asp Gln Lys Leu Trp Asp Glu
        355                 360                 365

Asp Pro His Glu Tyr Val Arg Lys Gly Tyr Asp Ile Ile Glu Asp Leu
    370                 375                 380

Tyr Ser Pro Arg Thr Ala Ala Met Asp Phe Val Ser Glu Leu Val Arg
385                 390                 395                 400

Lys Arg Gly Lys Asn Asn Leu Gln Lys Phe Ile His Phe Ile Val Asp
                405                 410                 415

Ile Phe Arg Arg Tyr Asp Glu Ala Ser Ala Asp Leu Lys Pro Tyr Arg
            420                 425                 430

Gln Lys Asp Gly Ala Leu Leu Ser Ile Gly Thr Leu Cys Asp Lys Leu
        435                 440                 445

Lys Gln Thr Asp Pro Tyr Lys Ser Glu Leu Glu Thr Met Leu Val Arg
    450                 455                 460

His Val Phe Pro Glu Phe Ser Ser Arg Val Gly His Leu Arg Ala Lys
465                 470                 475                 480

Ala Ala Trp Val Ala Gly Gln Tyr Ala His Ile Asn Phe Ser Asp Pro
                485                 490                 495

Asn Asn Phe Arg Gln Ala Met His Cys Ile Val Ser Gly Met His Asp
            500                 505                 510

Pro Asp Leu Pro Val Arg Val Asp Ser Val Phe Ala Leu Arg Ser Phe
        515                 520                 525

Val Glu Ala Cys Lys Asp Leu Asn Glu Ile Arg Pro Ile Leu Pro Gln
    530                 535                 540

Leu Leu Asp Glu Phe Phe Lys Leu Met Asn Glu Val Glu Asn Glu Asp
545                 550                 555                 560

Leu Val Phe Thr Leu Glu Thr Ile Val Asp Lys Phe Gly Glu Glu Met
                565                 570                 575

Ala Pro Tyr Ala Leu Gly Leu Cys Gln Asn Leu Ala Ala Ala Phe Trp
            580                 585                 590

Arg Cys Met Ala Ser Ser Glu Ala Asp Asp Glu Ala Asp Asp Ser Gly
        595                 600                 605

Ala Leu Ala Ala Val Gly Cys Leu Arg Ala Ile Ser Thr Ile Leu Glu
    610                 615                 620

Ser Ile Ser Ser Leu Pro His Leu Phe Thr Gln Ile Glu Pro Thr Leu
625                 630                 635                 640

Leu Pro Ile Met Arg Arg Met Leu Thr Ser Asp Gly Gln Asp Val Tyr
                645                 650                 655

Glu Glu Val Leu Glu Ile Val Ser Tyr Met Ser Phe Tyr Ser Pro Thr
            660                 665                 670

Ile Ser Leu Asp Met Trp Thr Leu Trp Pro Val Met Met Glu Ala Leu
        675                 680                 685

Asn Asp Trp Ala Ile Asp Phe Phe Glu Asn Ile Leu Val Pro Leu Asp
    690                 695                 700

Asn Tyr Ile Ser Arg Gly Thr Asp His Phe Leu Ala Cys Lys Asp Pro
705                 710                 715                 720

Asp Tyr Gln Gln Ser Leu Trp Lys Ala Leu Gln Ser Ile Met Met Asp
                725                 730                 735

Glu Asn Met Glu Asp Ser Asp Ile Glu Pro Ala Pro Lys Leu Ile Glu
            740                 745                 750

Val Val Phe Gln Asn Cys Lys Gly Asn Val Asp His Trp Val Glu His
        755                 760                 765

Tyr Leu Arg Ile Thr Ile Glu Arg Leu Arg Arg Ala His Lys Pro Tyr
    770                 775                 780

Leu Lys Cys Leu Leu Val Gln Val Ile Ala Asn Ala Leu Tyr Tyr Asn
785                 790                 795                 800

Pro Ala Leu Thr Leu Glu Ile Leu Asn Lys Leu Gly Val Ala Ala Asp

805                 810                 815

Ile Phe Asn His Trp Phe Ala Met Leu Arg Gln Val Lys Lys Ser Gly
            820                 825                 830

Ala Arg Val Asn Phe Lys Arg Glu His Asp Lys Lys Val Cys Cys Leu
        835                 840                 845

Gly Leu Thr Ser Leu Ile Ala Leu Pro Ala Ala Lys Ile Pro Ala Asp
    850                 855                 860

Ala Leu Asp Arg Ile Phe Lys Ala Thr Leu Glu Leu Leu Val Ala Tyr
865                 870                 875                 880

Lys Asp Gln Val Ala Glu Ala Lys Lys Gln Asn Glu Glu Ala Ala Asp
                885                 890                 895

Asp Met Asp Gly Phe Asp Ala Asp Glu Glu Asp Asp Asp Glu Val Asp
            900                 905                 910

Ser Asp Lys Glu Met Gly Leu Asp Asp Glu Asp Gly Asp Glu Val Ser
        915                 920                 925

Ser Leu Gln Leu Gln Lys Leu Ala Ala Glu Ala Arg Gly Phe Gln Pro
    930                 935                 940

Ala Asp Asp Asp Asp Ser Asp Asp Asp Phe Ser Asp Asp Glu Leu His
945                 950                 955                 960

Ser Pro Ile Asp Glu Val Asp Pro Phe Ile Phe Phe Val Glu Thr Val
                965                 970                 975

Gln Gly Leu Gln Ala Ser Asp Pro Ala Arg Phe Gln Asn Leu Met His
            980                 985                 990

Thr Leu Asp Phe Ser Tyr Gln Ala  Leu Ala Ser Gly Ile  Ala Gln His
        995                 1000                1005

Ala Glu  Gln Arg Lys Asn Glu  Ile Glu Lys Glu Lys  Ser Glu Lys
    1010                1015                1020

Ala Asn  Ala Gln
    1025

<210>  19
<211>  39
<212>  PRT
<213>  Artificial Sequence

<220>
<223>  consensus sequence

<220>
<221>  misc_feature
<222>  (5)..(5)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (14)..(14)
<223>  Xaa can be any naturally occurring amino acid

<220>
<221>  misc_feature
<222>  (17)..(17)
<223>  Xaa can be any naturally occurring amino acid

<220>
<221>  misc_feature
<222>  (20)..(20)
<223>  Xaa can be any naturally occurring amino acid

<220>
<221>  misc_feature
<222>  (32)..(32)
<223>  Xaa can be any naturally occurring amino acid

<400>  19

Leu Phe Glu Ile Xaa Phe Pro Leu Met Cys Phe Xaa Asp Xaa Asp Gln
1               5                   10                  15

Xaa Leu Trp Xaa Glu Asp Pro His Glu Tyr Val Arg Lys Gly Tyr Xaa
            20                  25                  30

Ile Ile Glu Asp Leu Tyr Ser
        35

<210>  20
<211>  1035
<212>  PRT
<213>  Nicotiana tabacum

<400>  20

Met Asp Leu Gln Asn Leu Ala Ile Val Leu Arg Gly Ala Leu Ser Pro
1               5                   10                  15

Asn Pro Asp Glu Arg Lys Ala Ala Glu Glu Ser Leu Asn Gln Phe Gln
            20                  25                  30

Tyr Thr Pro Gln His Leu Val Arg Leu Leu Gln Ile Ile Val Asp Gly
        35                  40                  45

Ser Cys Asp Met Ala Val Arg Gln Val Ala Ser Ile His Phe Lys Asn
    50                  55                  60

Phe Val Ala Lys Asn Trp Ser Pro His Asp Pro Ala Glu Gln Ser Lys
65                  70                  75                  80

Ile Leu Leu Ser Asp Lys Glu Leu Val Arg Gln Asn Ile Leu Ile Phe
                85                  90                  95

Ile Ala Gln Val Pro Ser Leu Leu Arg Val Gln Leu Gly Glu Cys Leu
            100                 105                 110

Lys Thr Met Ile His Ala Asp Tyr Pro Glu Gln Trp Pro Thr Leu Leu
        115                 120                 125

Pro Trp Val Lys His Asn Leu Gln Asp Gln Gln Val Tyr Gly Ala Leu
    130                 135                 140

Phe Val Leu Arg Ile Leu Ser Arg Lys Tyr Glu Phe Lys Ser Asp Glu
145                 150                 155                 160

Glu Arg Thr Pro Val Tyr His Ile Val Glu Glu Thr Phe Pro Gln Leu
                165                 170                 175

Leu Asn Ile Phe Asn Arg Leu Ala Gln Ile Ala Asn Pro Ser Ile Glu
            180                 185                 190

Val Ala Asp Leu Ile Lys Leu Ile Cys Lys Ile Phe Trp Ser Ser Ile
        195                 200                 205

Tyr Leu Glu Ile Pro Lys Gln Leu Phe Asp Pro Asn Met Phe Asn Ala
    210                 215                 220

Trp Met Val Leu Phe Leu Asn Met Leu Glu Arg Pro Val Pro Leu Glu
225                 230                 235                 240

Gly Gln Pro Ala Asp Pro Glu Leu Arg Lys Ser Trp Gly Trp Trp Lys
                245                 250                 255

Val Lys Lys Trp Thr Val His Ile Leu Asn Arg Leu Tyr Thr Arg Phe
            260                 265                 270

Gly Asp Leu Lys Leu Gln Asn Pro Asp Asn Lys Ala Phe Ala Gln Met
        275                 280                 285

Phe Gln Lys Gly Tyr Ala Gly Lys Ile Leu Glu Cys His Leu Asn Leu
    290                 295                 300

Leu Asn Val Ile Arg Ala Gly Gly Tyr Leu Pro Asp Arg Val Ile Asn
305                 310                 315                 320

Leu Ile Leu Gln Tyr Leu Ser Asn Ser Ile Ser Lys Ser Asn Met Tyr
                325                 330                 335

Ser Leu Leu Gln Pro Arg Leu Asp Ile Val Leu Phe Glu Ile Ile Phe
            340                 345                 350

Pro Leu Met Cys Phe Ser Asp Asn Asp Gln Lys Leu Trp Glu Glu Asp
        355                 360                 365

Pro His Glu Tyr Val Arg Lys Gly Tyr Asp Ile Ile Glu Asp Leu Tyr
    370                 375                 380

Ser Pro Arg Thr Ala Ala Met Asp Phe Val Ser Glu Leu Val Arg Lys
385                 390                 395                 400

Arg Gly Lys Glu Asn Leu Gln Lys Phe Ile Leu Phe Ile Val Glu Ile
                405                 410                 415

Phe Lys Arg Tyr Glu Glu Ala Ala Pro Glu Tyr Lys Pro Tyr Arg Gln
            420                 425                 430

Lys Asp Gly Ala Leu Leu Ala Ile Gly Ala Leu Cys Asp Lys Leu Lys
        435                 440                 445

Gln Thr Glu Pro Tyr Lys Ser Glu Leu Glu Arg Met Leu Val Gln His
    450                 455                 460

Val Phe Pro Glu Phe Ser Ser Pro Val Gly His Leu Arg Ala Lys Ala
465                 470                 475                 480

Ala Trp Val Ala Gly Gln Tyr Ala His Val Asn Phe Ser Asp Pro Asn
                485                 490                 495

Asn Phe Arg Lys Ala Leu His Ser Val Val Ala Gly Met Arg Asp Pro
            500                 505                 510

Asp Leu Pro Val Arg Val Asp Ser Val Phe Ala Leu Arg Ser Phe Val
        515                 520                 525

Glu Ala Cys Arg Asp Leu Asp Glu Ile Arg Pro Ile Ile Pro Gln Leu

530                 535                 540

Leu Asp Glu Phe Phe Lys Leu Met Asn Glu Val Glu Asn Glu Asp Leu
545                 550                 555                 560

Val Phe Thr Leu Glu Thr Ile Val Asp Lys Phe Gly Glu Glu Met Ala
                565                 570                 575

Pro Tyr Ala Leu Gly Leu Cys Gln Asn Leu Ala Ala Ala Phe Trp Lys
            580                 585                 590

Cys Ile Asn Thr Ser Glu Ala Asp Glu Glu Gly Asp Asp Pro Gly Ala
        595                 600                 605

Leu Ala Ala Val Gly Cys Leu Arg Ala Ile Ser Thr Ile Leu Glu Ser
    610                 615                 620

Val Ser Arg Leu Pro His Leu Phe Ile His Ile Glu Pro Thr Leu Leu
625                 630                 635                 640

Pro Ile Met Arg Arg Met Leu Thr Thr Asp Gly Gln Glu Val Phe Glu
                645                 650                 655

Glu Val Leu Glu Ile Val Ser Tyr Met Thr Phe Phe Ser Pro Thr Ile
            660                 665                 670

Ser Met Glu Met Trp Ser Leu Trp Pro Leu Met Met Glu Ala Leu Ala
        675                 680                 685

Asp Trp Ala Ile Asp Phe Phe Pro Asn Ile Leu Val Pro Leu Asp Asn
    690                 695                 700

Tyr Ile Ser Arg Ser Thr Ala His Phe Leu Thr Cys Lys Asp Pro Asp
705                 710                 715                 720

Tyr Gln Lys Ser Leu Trp Asn Met Ile Ser Thr Val Met Gly Asp Lys
                725                 730                 735

Asn Leu Glu Asp Gly Asp Ile Glu Pro Ala Pro Lys Leu Ile Gln Val
            740                 745                 750

Val Phe Gln His Cys Lys Gly Gln Val Asp His Trp Val Glu Pro Tyr
        755                 760                 765

Ile Arg Ile Thr Ile Glu Arg Leu Arg Arg Ala Glu Lys Pro Tyr Leu
    770                 775                 780

Lys Cys Leu Leu Met Gln Val Ile Ala Asp Ala Leu Tyr Tyr Asn Ala
785                 790                 795                 800

Ser Leu Thr Leu Asn Ile Leu Gln Lys Leu Gly Ile Ala Thr Glu Val
                805                 810                 815

Phe Asn Leu Trp Phe Gln Met Leu Gly Gln Lys Lys Lys Ser Gly Ala
            820                 825                 830

Arg Val Asn Phe Lys Arg Glu His Asp Lys Lys Val Cys Cys Leu Gly
        835                 840                 845

Leu Thr Ser Leu Leu Pro Leu Pro Val Asp Gln Leu Pro Gly Glu Ala
    850                 855                 860

Leu Glu Arg Val Phe Lys Ala Ser Leu Glu Leu Leu Val Ala Tyr Lys
865                 870                 875                 880

Asp Gln Val Ala Glu Ala Ala Lys Glu Glu Glu Val Glu Asp Asp Asp
                885                 890                 895

Asp Met His Gly Leu Gln Thr Asp Glu Asp Asp Asp Glu Asp Asp Gly
            900                 905                 910

Ser Asp Lys Glu Met Gly Val Asp Ala Glu Glu Gly Asp Glu Ala Asp
        915                 920                 925

Ser Thr Arg Leu Gln Lys Leu Ala Ala Gln Ala Lys Ala Phe Arg Ser
    930                 935                 940

His Asp Ser Asp Glu Asp Asp Asp Ser Asp Asp Asp Phe Ser Asp Asp
945                 950                 955                 960

Asp Glu Glu Leu Gln Ser Pro Leu Asp Glu Val Asp Pro Phe Ile Phe
                965                 970                 975

Phe Val Glu Thr Ile Lys Ala Met Gln Ala Ser Asp Pro Leu Arg Phe
            980                 985                 990

Gln Ser Leu Thr Gln Thr Leu Asp  Phe His Tyr Gln Ala  Leu Ala Asn
        995                 1000                1005

Gly Val  Ala Gln His Ala Glu  Gln Arg Arg Val Glu  Ile Glu Lys
    1010                1015                1020

Glu Lys  Met Glu Lys Ala Ser  Ala Thr Ala Ala Ser
    1025                1030                1035

<210>  21
<211>  1030
<212>  PRT
<213>  Oryza sativa

<400>  21

Met Asp Leu Gln Ser Leu Ala Val Val Leu Arg Ala Ala Leu Ser His
1               5                   10                  15

Val Pro Glu Glu Arg Lys Ala Ala Glu Glu Ser Leu Asn Gln Phe Gln
            20                  25                  30

Tyr Ala Pro Gln His Leu Val Arg Leu Leu Gln Ile Ile Val Asp Gly
        35                  40                  45

Ser Cys Asp Met Ala Val Arg Gln Val Ala Ser Ile His Phe Lys Asn
    50                  55                  60

Phe Val Ala Lys Asn Trp Ser Pro Asn Asp Pro Glu Glu Ser Gln Lys
65                  70                  75                  80

Ile Ser Glu Ser Asp Lys Leu Met Val Arg Glu Asn Ile Leu Gly Phe
                85                  90                  95

Ile Val Gln Val Pro Pro Leu Leu Arg Ala Gln Leu Gly Glu Ser Ile
            100                 105                 110

Lys Thr Ile Ile His Ser Asp Tyr Pro Glu Gln Trp Pro Val Leu Leu
        115                 120                 125

His Trp Val Thr His Asn Leu Glu Ser Glu Asn Gln Ile Phe Gly Ala
    130                 135                 140

Leu Tyr Val Leu Arg Val Leu Ser Arg Lys Tyr Glu Phe Lys Ser Glu
145                 150                 155                 160

Glu Glu Arg Ile Pro Leu Tyr His Ile Val Glu Glu Thr Phe Pro Arg
                165                 170                 175

Leu Leu Ser Ile Phe Ser Lys Leu Val Gln Ile Val Asn Pro Pro Ile
            180                 185                 190

Glu Val Ala Asp Leu Ile Lys Leu Ile Cys Lys Ile Phe Trp Ser Ser
        195                 200                 205

Ile Tyr Leu Glu Ile Pro Lys Gln Leu Phe Asp Pro Asn Val Phe Asn
    210                 215                 220

Ala Trp Met Ser Leu Phe Ile Asn Leu Leu Glu Arg Pro Val Pro Val
225                 230                 235                 240

Glu Gly Gln Pro Leu Asp Pro Asp Val Arg Lys Ser Trp Gly Trp Trp
                245                 250                 255

Lys Val Lys Lys Trp Thr Ile His Ile Leu Asn Arg Leu Tyr Thr Arg
            260                 265                 270

Phe Gly Asp Met Lys Leu Gln Lys Pro Glu Ser Lys Ala Phe Ala Gln
        275                 280                 285

Met Phe Gln Lys Asn Tyr Ala Gly Arg Ile Leu Gly Cys His Leu Gln
    290                 295                 300

Ile Leu Asn Ala Val Arg Thr Gly Asp Tyr Leu Pro Asp Arg Val Ile
305                 310                 315                 320

Asn Leu Val Leu Gln Tyr Leu Thr Asn Ser Val Thr Lys Asn Ser Met
                325                 330                 335

Tyr Gln Leu Met Gln Pro Gln Ile Asp Ile Ile Leu Phe Glu Ile Ile
            340                 345                 350

Phe Pro Leu Met Cys Phe Asn Asp Asn Asp Gln Lys Leu Trp Asp Glu
        355                 360                 365

Asp Pro His Glu Tyr Val Arg Lys Gly Tyr Asp Ile Ile Glu Asp Leu
    370                 375                 380

Tyr Ser Pro Arg Thr Ala Ala Met Asp Phe Val Ser Glu Leu Val Arg
385                 390                 395                 400

Lys Arg Gly Lys Ser Asn Leu Gln Lys Phe Ile His Phe Ile Val Asp
                405                 410                 415

Ile Phe Arg Arg Tyr Asp Glu Ala Ser Ile Glu Ile Lys Pro Tyr Arg
            420                 425                 430

Gln Lys Asp Gly Ala Leu Leu Ala Ile Gly Thr Leu Cys Asp Lys Leu
        435                 440                 445

Lys Gln Thr Asp Pro Tyr Lys Ala Glu Leu Glu Arg Met Leu Val Gln
    450                 455                 460

His Val Phe Pro Glu Phe Asn Ser His Val Gly His Leu Arg Ala Lys
465                 470                 475                 480

Ala Ala Trp Val Ala Gly Gln Tyr Ala His Ile Ser Phe Ser Asp Gln
                485                 490                 495

Asp Asn Phe Arg Lys Ala Met His Cys Ile Val Ser Gly Met Arg Asp
            500                 505                 510

Pro Asp Leu Pro Val Arg Val Asp Ser Val Phe Ala Leu Arg Ser Phe
        515                 520                 525

Val Glu Ala Cys Lys Asp Leu Asn Glu Ile Arg Pro Ile Leu Pro Gln
    530                 535                 540

Leu Leu Asp Glu Phe Phe Lys Leu Met Asn Glu Val Glu Asn Glu Asp
545                 550                 555                 560

Leu Val Phe Thr Leu Glu Thr Ile Val Asp Lys Phe Gly Glu Glu Met
                565                 570                 575

Ala Pro Tyr Ala Leu Gly Leu Cys Gln Asn Leu Ala Ala Ala Phe Trp
            580                 585                 590

Arg Cys Met Ala Ser Gln Glu Ala Asp Asp Glu Ala Asp Asp Ser Gly
        595                 600                 605

Ala Leu Ala Ala Val Gly Cys Leu Arg Ala Ile Ser Thr Ile Leu Glu
    610                 615                 620

Ser Val Ser Ser Leu Pro His Leu Phe Ile Gln Ile Glu Pro Thr Leu
625                 630                 635                 640

Leu Pro Ile Met Arg Arg Met Leu Thr Ser Asp Gly Gln Asp Val Tyr
                645                 650                 655

Glu Glu Val Leu Glu Ile Val Ser Tyr Met Thr Phe Phe Ser Pro Ser
            660                 665                 670

Ile Ser Leu Asp Met Trp Ser Leu Trp Pro Leu Met Met Glu Ala Leu
        675                 680                 685

Asn Asp Trp Ala Ile Asp Phe Phe Glu Asn Ile Leu Val Pro Leu Asp
    690                 695                 700

Asn Tyr Val Ser Arg Gly Ser Asp His Phe Leu Ala Cys Lys Asn Pro
705                 710                 715                 720

Asp Tyr Gln Gln Ser Leu Trp Ser Ala Leu Ser Ser Ile Met Met Asp

                725                 730                 735

Gln Asn Met Glu Asp Ser Asp Ile Glu Pro Ala Pro Lys Leu Ile Glu
            740                 745                 750

Val Val Phe Gln Asn Cys Lys Gly Asn Val Asp Gln Trp Val Glu Pro
        755                 760                 765

Tyr Leu Ser Leu Thr Ile Asp Arg Leu Arg Arg Ala His Lys Pro Tyr
    770                 775                 780

Leu Lys Cys Leu Leu Val Gln Val Ile Ala Asn Ala Phe Tyr Tyr Asn
785                 790                 795                 800

Pro Ser Leu Thr Leu Ala Thr Leu His Lys Leu Gly Ala Val Thr Glu
                805                 810                 815

Ile Phe Asn Ile Trp Phe Gly Met Leu Glu Gln Val Lys Lys Ser Gly
            820                 825                 830

Val Arg Ala Asn Phe Lys Arg Glu His Asp Lys Lys Val Cys Cys Leu
        835                 840                 845

Gly Leu Thr Ser Leu Ile Ser Leu Pro Ala Asp His Ile Pro Gly Glu
    850                 855                 860

Ala Leu Asn Arg Ile Phe Lys Ala Thr Leu Asp Leu Leu Val Ala Tyr
865                 870                 875                 880

Lys Glu Gln Val Ala Glu Ser Lys Lys Gln Asp Asp Ala Asp Gly Asp
                885                 890                 895

Asp Met Asp Gly Phe Asp Ala Asp Glu Asp Glu Asp Asp Asp Glu Val
            900                 905                 910

Glu Ser Asp Lys Glu Met Gly Leu Asp Glu Glu Asp Ala Asp Glu Val
        915                 920                 925

Asn Ser Leu His Leu Gln Lys Leu Ala Ala Glu Ala Arg Gly Phe Gln
    930                 935                 940

Pro Ala Asp Glu Tyr Asp Asp Ser Asp Asp Asp Phe Ser Asp Asp Glu
945                 950                 955                 960

Glu Leu Gln Ser Pro Ile Asp Glu Val Asp Pro Phe Ile Leu Phe Val
                965                 970                 975

Glu Thr Val Gln Gly Leu Gln Ala Ser Asp Pro Ile Arg Phe Gln Ser
            980                 985                 990

Leu Met Gln Thr Leu Asp Phe Arg Tyr Gln Ala Leu Ala Ser Gly Ile
        995                 1000                1005

Ala Gln His Ala Glu Glu Arg Arg Val Glu Ile Glu Lys Glu Lys
    1010                1015                1020

Leu Glu Lys Ala Asn Ala Gln
    1025                1030

<210> 22
<211> 1030
<212> PRT
<213> Oryza sativa

<400> 22

Met Asp Leu Gln Ser Leu Ala Val Val Leu Arg Ala Ala Leu Ser His
1               5                   10                  15

Val Pro Glu Glu Arg Lys Ala Ala Glu Glu Ser Leu Asn Gln Phe Gln
            20                  25                  30

Tyr Ala Pro Gln His Leu Val Arg Leu Leu Gln Ile Ile Val Asp Gly
        35                  40                  45

Ser Cys Asp Met Ala Val Arg Gln Val Ala Ser Ile His Phe Lys Asn
    50                  55                  60

Phe Val Ala Lys Asn Trp Ser Pro Asn Asp Pro Glu Glu Ser Gln Lys
65                  70                  75                  80

Ile Ser Glu Ser Asp Lys Leu Met Val Arg Glu Asn Ile Leu Gly Phe
                85                  90                  95

Ile Val Gln Val Pro Pro Leu Leu Arg Ala Gln Leu Gly Glu Ser Ile
            100                 105                 110

Lys Thr Ile Ile His Ser Asp Tyr Pro Glu Gln Trp Pro Gly Leu Leu
        115                 120                 125

His Trp Val Thr His Asn Leu Glu Ser Glu Asn Gln Ile Phe Gly Ala
    130                 135                 140

Leu Tyr Val Leu Arg Val Leu Ser Arg Lys Tyr Glu Phe Lys Ser Glu
145                 150                 155                 160

Asp Glu Arg Ile Pro Leu Tyr His Ile Val Glu Glu Thr Phe Pro Arg
                165                 170                 175

Leu Leu Ser Ile Phe Ser Lys Leu Val Gln Ile Val Asn Pro Pro Ile
            180                 185                 190

Glu Val Ala Asp Leu Ile Lys Leu Ile Cys Lys Ile Phe Trp Ser Ser
        195                 200                 205

Ile Tyr Leu Glu Ile Pro Lys Gln Leu Phe Asp Pro Asn Val Phe Asn
    210                 215                 220

Ala Trp Met Ser Leu Phe Ile Asn Leu Leu Glu Arg Pro Val Pro Val
225                 230                 235                 240

Glu Gly Gln Pro Leu Asp Pro Asp Val Arg Lys Ser Trp Gly Trp Trp
                245                 250                 255

Lys Val Lys Lys Trp Thr Ile His Ile Leu Asn Arg Leu Tyr Thr Arg
            260                 265                 270

Phe Gly Asp Met Lys Leu Gln Lys Pro Glu Ser Lys Ala Phe Ala Gln
        275                 280                 285

Met Phe Gln Lys Asn Tyr Ala Gly Arg Ile Leu Gly Cys His Leu Gln
    290                 295                 300

Ile Leu Asn Ala Val Arg Thr Gly Asp Tyr Leu Pro Asp Arg Val Ile
305                 310                 315                 320

Asn Leu Val Leu Gln Tyr Leu Thr Asn Ser Val Thr Lys Asn Ser Met
                325                 330                 335

Tyr Gln Leu Met Gln Pro Gln Ile Asp Ile Ile Leu Phe Glu Ile Ile
            340                 345                 350

Phe Pro Leu Met Cys Phe Asn Asp Asn Asp Gln Lys Leu Trp Asp Glu
        355                 360                 365

Asp Pro His Glu Tyr Val Arg Lys Gly Tyr Asp Ile Ile Glu Asp Leu
    370                 375                 380

Tyr Ser Pro Arg Thr Ala Ala Met Asp Phe Val Ser Glu Leu Val Arg
385                 390                 395                 400

Lys Arg Gly Lys Ser Asn Leu Gln Lys Phe Ile His Phe Ile Val Asp
                405                 410                 415

Ile Phe Arg Arg Tyr Asp Glu Ala Ser Ile Glu Ile Lys Pro Tyr Arg
            420                 425                 430

Gln Lys Asp Gly Ala Leu Leu Ala Ile Gly Thr Leu Cys Asp Lys Leu
        435                 440                 445

Lys Gln Thr Asp Pro Tyr Lys Ala Glu Leu Glu Arg Met Leu Val Gln
    450                 455                 460

His Val Phe Pro Glu Phe Asn Ser His Val Gly His Leu Arg Ala Lys
465                 470                 475                 480

Ala Ala Trp Val Ala Gly Gln Tyr Ala His Ile Ser Phe Ser Asp Gln
                485                 490                 495

Asp Asn Phe Arg Lys Ala Met His Cys Ile Val Ser Gly Met Arg Asp
            500                 505                 510

Pro Asp Leu Pro Val Arg Val Asp Ser Val Phe Ala Leu Arg Ser Phe
        515                 520                 525

Val Glu Ala Cys Lys Asp Leu Asn Glu Ile Arg Pro Ile Leu Pro Gln
    530                 535                 540

Leu Leu Asp Glu Phe Phe Lys Leu Met Asn Glu Val Glu Asn Glu Asp
545                 550                 555                 560

Leu Val Phe Thr Leu Glu Thr Ile Val Asp Lys Phe Gly Glu Glu Met
                565                 570                 575

Ala Pro Tyr Ala Leu Gly Leu Cys Gln Asn Leu Ala Ala Ala Phe Trp
            580                 585                 590

Arg Cys Met Ala Ser Gln Glu Ala Asp Asp Glu Ala Asp Asp Ser Gly
        595                 600                 605

Ala Leu Ala Ala Val Gly Cys Leu Arg Ala Ile Ser Thr Ile Leu Glu
    610                 615                 620

Ser Val Ser Ser Leu Pro His Leu Phe Ile Gln Ile Glu Pro Thr Leu
625                 630                 635                 640

Leu Pro Ile Met Arg Arg Met Leu Thr Ser Asp Gly Gln Asp Val Tyr
                645                 650                 655

Glu Glu Val Leu Glu Ile Val Ser Tyr Met Thr Phe Phe Ser Pro Ser
            660                 665                 670

Ile Ser Leu Asp Met Trp Ser Leu Trp Pro Leu Met Met Glu Ala Leu
        675                 680                 685

Asn Asp Trp Ala Ile Asp Phe Phe Glu Asn Ile Leu Val Pro Leu Asp
    690                 695                 700

Asn Tyr Val Ser Arg Gly Ser Asp His Phe Leu Ala Cys Lys Asn Pro
705                 710                 715                 720

Asp Tyr Gln Gln Ser Leu Trp Ser Ala Leu Ser Ser Ile Met Met Asp
                725                 730                 735

Gln Asn Met Glu Asp Ser Asp Ile Glu Pro Ala Pro Lys Leu Ile Glu
            740                 745                 750

Val Val Phe Gln Asn Cys Lys Gly Asn Val Asp Gln Trp Val Glu Pro
        755                 760                 765

Tyr Leu Ser Leu Thr Ile Asp Arg Leu Arg Arg Ala His Lys Pro Tyr
    770                 775                 780

Leu Lys Cys Leu Leu Val Gln Val Ile Ala Asn Ala Phe Tyr Tyr Asn
785                 790                 795                 800

Pro Ser Leu Thr Leu Ala Thr Leu His Lys Leu Gly Ala Val Thr Glu
                805                 810                 815

Ile Phe Asn Ile Trp Phe Gly Met Leu Glu Gln Val Lys Lys Ser Gly
            820                 825                 830

Val Arg Ala Asn Phe Lys Arg Glu His Asp Lys Lys Val Cys Cys Leu
        835                 840                 845

Gly Leu Thr Ser Leu Ile Ser Leu Pro Ala Asp His Ile Pro Gly Glu
    850                 855                 860

Ala Leu Asn Arg Ile Phe Lys Ala Thr Leu Asp Leu Leu Val Ala Tyr
865                 870                 875                 880

Lys Glu Gln Val Ala Glu Ser Lys Lys Gln Asp Asp Ala Asp Gly Asp
                885                 890                 895

Asp Met Asp Gly Phe Asp Ala Asp Glu Asp Glu Asp Asp Asp Glu Val
            900                 905                 910

Glu Ser Asp Lys Glu Met Gly Leu Asp Glu Glu Asp Ala Asp Glu Val

915                 920                 925

Asn Ser Leu His Leu Gln Lys Leu Ala Ala Glu Ala Arg Gly Phe Gln
    930                 935                 940

Pro Ala Asp Glu Tyr Asp Glu Ser Asp Asp Asp Phe Ser Asp Asp Glu
945                 950                 955                 960

Glu Leu Gln Ser Pro Ile Asp Glu Val Asp Pro Phe Ile Leu Phe Val
                965                 970                 975

Glu Thr Val Gln Gly Leu Gln Ala Ser Asp Pro Ile Arg Phe Gln Ser
            980                 985                 990

Leu Met Gln Thr Leu Asp Phe Arg  Tyr Gln Ala Leu Ala  Ser Gly Ile
        995                 1000                 1005

Ala Gln  His Ala Glu Glu Arg  Arg Val Glu Ile Glu  Lys Glu Lys
    1010                 1015                 1020

Leu Glu  Lys Ala Asn Ala Gln
    1025                 1030

<210>  23
<211>  1034
<212>  PRT
<213>  Coffea arabica

<400>  23

Met Asp Leu Gln Asn Leu Ala Ile Ile Leu Arg Gly Ala Leu Ser Pro
1               5                   10                  15

Asn Pro Asp Glu Arg Lys Ala Ala Glu Glu Ser Leu Asn Gln Phe Gln
            20                  25                  30

Tyr Thr Pro Gln His Leu Val Arg Leu Leu Gln Ile Ile Val Asp Gly
        35                  40                  45

Asn Cys Asp Met Ala Val Arg Gln Val Ala Ser Ile His Phe Lys Asn
    50                  55                  60

Phe Ile Ala Arg Asn Trp Ala Pro His Asp Pro Gly Glu Gln Ser Lys
65                  70                  75                  80

Ile Leu Pro Ser Asp Lys Asp Val Val Arg Gln Asn Ile Leu Asn Phe
                85                  90                  95

Ile Ala Gln Val Pro Gln Leu Leu Arg Val Gln Leu Gly Glu Cys Leu

            100                 105                 110

Lys Thr Ile Ile His Ala Asp Tyr Pro Glu Gln Trp Pro Ala Leu Leu
        115                 120                 125

His Trp Val Lys Leu Asn Leu Gln Asp Gln Gln Val Phe Gly Ala Leu
    130                 135                 140

Phe Val Leu Arg Ile Leu Ala Arg Lys Tyr Glu Phe Lys Ser Asp Glu
145                 150                 155                 160

Glu Arg Thr Pro Val Tyr His Ile Val Asp Glu Thr Phe Pro Pro Leu
                165                 170                 175

Leu Asn Ile Phe Asn Arg Leu Val Gln Ile Thr Asn Pro Ser Ile Glu
            180                 185                 190

Val Ala Asp Leu Ile Lys Leu Ile Cys Lys Ile Phe Trp Ser Cys Ile
        195                 200                 205

Tyr Leu Glu Ile Pro Lys Gln Leu Phe Asp Pro Asn Val Phe Asn Ala
    210                 215                 220

Trp Met Val Leu Phe Leu Asn Val Leu Glu Arg Pro Val Pro Leu Glu
225                 230                 235                 240

Gly Gln Pro Val Asp Pro Glu Val Arg Lys Ser Trp Gly Trp Trp Lys
                245                 250                 255

Val Lys Lys Trp Thr Val His Ile Leu Asn Arg Leu Tyr Thr Arg Phe
            260                 265                 270

Gly Asp Leu Lys Leu Gln Asn Pro Asp Asn Lys Val Phe Ala Gln Met
        275                 280                 285

Phe Gln Lys Asn Tyr Ala Gly Lys Ile Leu Glu Cys His Leu Asn Leu
    290                 295                 300

Leu Asn Val Ile Arg Val Gly Gly Tyr Leu Pro Asp Arg Val Ile Asn
305                 310                 315                 320

Leu Ile Leu Gln Tyr Leu Ser Asn Ser Ile Ser Arg Ser Asn Met Tyr
                325                 330                 335

Asn Leu Leu Gln Pro Arg Leu Asp Val Val Leu Phe Glu Ile Ile Phe
            340                 345                 350

Pro Leu Met Cys Phe Ser Asp Ala Asp Gln Lys Leu Trp Asp Glu Asp
        355                 360                 365

Pro His Glu Tyr Val Arg Lys Gly Tyr Asp Ile Ile Glu Asp Leu Tyr
    370                 375                 380

Ser Pro Arg Thr Ala Ala Met Asp Phe Val Ser Glu Leu Val Arg Lys
385                 390                 395                 400

Arg Gly Lys Glu Asn Leu Gln Lys Phe Ile Leu Phe Ile Val Glu Ile
                405                 410                 415

Phe Asn Arg Tyr Asn Glu Ala Val Pro Glu Tyr Lys Pro Phe Arg Gln
            420                 425                 430

Lys Asp Gly Ala Leu Leu Ala Ile Gly Ala Leu Cys Asp Lys Leu Lys
        435                 440                 445

Gln Thr Glu Pro Tyr Lys Ser Glu Leu Glu Arg Met Leu Val Gln His
    450                 455                 460

Val Phe Pro Glu Phe Ser Ser Pro Ala Gly His Leu Arg Ala Lys Ala
465                 470                 475                 480

Ala Trp Val Ala Gly Gln Tyr Ala His Val Asn Phe Ser Asp Pro Asn
                485                 490                 495

Asn Phe Arg Asn Ala Leu Arg Ser Val Val Ala Gly Met Arg Asp Pro
            500                 505                 510

Glu Leu Pro Val Arg Val Asp Ser Val Phe Ala Leu Arg Ser Phe Val
        515                 520                 525

Glu Ala Cys Lys Asp Leu Asp Glu Ile Arg Pro Ile Leu Pro Gln Leu
    530                 535                 540

Leu Asp Glu Phe Phe Lys Leu Met Asn Glu Val Glu Asn Glu Asp Leu
545                 550                 555                 560

Val Phe Thr Leu Glu Thr Ile Val Asp Lys Phe Gly Glu Glu Met Ala
                565                 570                 575

Pro Tyr Ala Leu Gly Leu Cys Gln Asn Leu Ala Ala Ala Phe Trp Lys
            580                 585                 590

Cys Leu Asn Thr Ala Glu Ala Asp Asp Glu Ala Asp Asp Pro Gly Ala
        595                 600                 605

Leu Ala Ala Val Gly Cys Leu Arg Ala Ile Ser Thr Ile Leu Glu Ser
    610                 615                 620

Val Ser Ser Leu Pro His Leu Phe Val Gln Ile Glu Pro Thr Leu Leu
625                 630                 635                 640

Pro Ile Met Arg Arg Met Leu Thr Thr Asp Gly Gln Glu Val Phe Glu
                645                 650                 655

Glu Val Leu Glu Ile Val Ser Tyr Met Thr Phe Phe Ser Pro Thr Ile
            660                 665                 670

Ser Met Asp Met Trp Ser Leu Trp Pro Leu Met Met Glu Ala Leu Ala
        675                 680                 685

Asp Trp Ala Ile Asp Phe Phe Pro Asn Ile Leu Val Pro Leu Asp Asn
    690                 695                 700

Tyr Val Ser Arg Ser Thr Val His Phe Leu Thr Cys Lys Glu Pro Asp
705                 710                 715                 720

Tyr Gln Gln Ser Leu Trp His Met Ile Ser Ser Ile Met Ala Asp Lys
                725                 730                 735

Asn Leu Glu Asp Gly Asp Ile Glu Pro Ala Pro Lys Leu Ile Gln Val
            740                 745                 750

Val Phe Gln Asn Cys Arg Gly Gln Val Asp His Trp Val Glu Pro Tyr
        755                 760                 765

Ile Arg Ile Ala Val Glu Arg Leu Arg Arg Thr Glu Lys Pro Tyr Leu
    770                 775                 780

Lys Cys Leu Leu Ile Glu Val Ile Ala Asp Ala Leu Tyr Tyr Asn Ala
785                 790                 795                 800

Ser Leu Thr Leu Ser Ile Leu His Lys Leu Gly Val Ala Ser Asp Ile
                805                 810                 815

Phe Ala Leu Trp Phe Gln Met Leu Gln Gln Thr Lys Lys Asn Gly Val
            820                 825                 830

Arg Ala Asn Phe Lys Arg Glu His Asp Lys Lys Val Cys Cys Leu Gly
        835                 840                 845

Met Thr Ser Leu Leu Ser Leu Pro Asn Asp Gln Phe Pro Ala Glu Ala
    850                 855                 860

Leu Glu Arg Val Phe Lys Ala Thr Leu Glu Leu Leu Val Ala Tyr Lys
865                 870                 875                 880

Glu Gln Leu Ala Glu Thr Glu Lys Glu Ala Ala Glu Asp Asn Asp Asp
                885                 890                 895

Met Asp Gly Leu Pro Thr Asp Asp Glu Asp Asp Asp Gly Asp Val Ser
            900                 905                 910

Asp Lys Glu Met Gly Ile Asp Asp Glu Asp Gly Asp Glu Ala Asp Ser
        915                 920                 925

Ile Lys Leu Gln Lys Leu Ala Ala Gln Ala Lys Ala Phe Arg Ser Thr
    930                 935                 940

Glu Asp Asp Glu Asp Asp Ser Asp Asp Asp Phe Ser Asp Asp Glu Glu
945                 950                 955                 960

Leu Gln Ser Pro Ile Asp Asp Val Asp Pro Phe Val Phe Phe Val Asp
                965                 970                 975

Thr Ile Lys Ala Leu Gln Ala Ser Asp Pro Leu Arg Phe Gln Asn Leu
            980                 985                 990

Thr Gln Thr Leu Asp Phe His Phe  Gln Ala Leu Ala Asn  Gly Val Ala
        995                 1000                1005

Gln His  Ala Glu Gln Arg Arg  Val Glu Ile Glu Lys  Glu Lys Met
    1010                1015                1020

Glu Lys  Ala Ser Ser Ala Ala  Val Gly Ala Ser
    1025                1030

<210>  24
<211>  1031
<212>  PRT
<213>  Triticum aestivum

<400>  24

Met Asp Leu Ser Asn Leu Thr Leu Val Leu Arg Ala Ala Leu Ser His
1               5                   10                  15

Ala Pro Glu Glu Arg Lys Ala Ala Glu Ala Ser Leu Glu Gln Leu Gln
            20                  25                  30

Tyr Thr Gln Gln His Leu Val Arg Leu Leu Gln Ile Ile Val Asp Gly
        35                  40                  45

Ser Cys Asp Met Ala Val Arg Gln Val Ala Ser Ile His Phe Lys Asn
    50                  55                  60

Phe Val Ser Lys Ala Trp Ser Pro Ile Asp Pro Asp Glu Thr Arg Lys
65                  70                  75                  80

Ile Pro Glu Gly Asp Lys Ser Met Val Arg Glu Asn Ile Leu Gly Phe
                85                  90                  95

Val Thr Gln Leu Pro Pro Leu Leu Arg Ala Gln Leu Gly Glu Ser Ile
            100                 105                 110

Lys Thr Leu Ile Leu Ala Asp Tyr Pro Glu Gln Trp Pro Ser Leu Leu
        115                 120                 125

His Trp Val Thr His Asn Met Glu Ser Gln Asp Gln Ile Phe Gly Ala
    130                 135                 140

Leu Tyr Val Leu Arg Ile Leu Ser Arg Lys Tyr Glu Phe Lys Ser Glu
145                 150                 155                 160

Glu Glu Arg Ile Pro Leu Tyr Gln Ile Val Glu Glu Cys Phe Pro Arg
                165                 170                 175

Leu Leu Asn Ile Phe Ser Thr Leu Val Gln Ile Val Asn Pro Pro Ile
            180                 185                 190

Glu Val Ala Asp Leu Ile Lys Leu Ile Cys Lys Ile Phe Trp Ser Ser
        195                 200                 205

Ile Tyr Leu Glu Ile Pro Lys Gln Leu Phe Glu Pro Asn Ile Phe Asn
    210                 215                 220

Ala Trp Ile Val Leu Phe Leu Asn Leu Leu Glu Arg Pro Val Pro Leu
225                 230                 235                 240

Glu Gly Gln Pro Ser Asp Pro Asp Ala Arg Lys Ala Trp Gly Trp Trp
                245                 250                 255

Lys Val Lys Lys Trp Ile Thr His Ile Leu Asn Arg Leu Tyr Ser Arg
            260                 265                 270

Phe Ala Asp Met Lys Val His Lys Pro Glu Ser Lys Ala Phe Ala Gln
        275                 280                 285

Met Phe Gln Lys Asn Tyr Ala Gly Lys Ile Leu Gly Cys His Leu Gln

290                 295                 300

Leu Leu Asn Ala Ile Arg Thr Gly Gly Tyr Leu Pro Asp Arg Val Ile
305                 310                 315                 320

Asn Leu Ile Leu Gln Tyr Leu Thr Asn Ser Leu Thr Lys Asn Ser Met
                325                 330                 335

Tyr Gln Leu Met Gln Pro Gln Ile Asp Ile Ile Leu Phe Glu Ile Ile
            340                 345                 350

Phe Pro Leu Met Cys Phe Asn Asp Asn Asp Gln Met Leu Trp Asp Glu
        355                 360                 365

Asp Pro His Glu Tyr Val Arg Lys Gly Tyr Asp Ile Ile Glu Asp Leu
    370                 375                 380

Tyr Ser Pro Arg Thr Ala Ala Met Asp Phe Val Ser Glu Leu Val Arg
385                 390                 395                 400

Lys Arg Gly Lys Gly Asn Leu Gln Lys Phe Ile Gln Phe Ile Val Glu
                405                 410                 415

Ile Phe Met Arg Tyr Asn Glu Ala Ser Ile Glu Val Lys Pro Tyr Arg
            420                 425                 430

Gln Lys Asp Gly Ala Leu Leu Ala Ile Gly Thr Leu Cys Asp Arg Leu
        435                 440                 445

Lys Gln Thr Asp Pro Tyr Lys Ala Glu Leu Glu Arg Met Leu Val Gln
    450                 455                 460

His Val Phe Pro Glu Phe Ser Ser His Val Gly His Leu Arg Ala Lys
465                 470                 475                 480

Ala Ala Trp Val Ala Gly Gln Tyr Ala His Ile Thr Phe Ser Asp Gln
                485                 490                 495

Asp Asn Phe Arg Lys Ala Met His Cys Val Ile Ser Gly Leu Arg Asp
            500                 505                 510

Pro Glu Leu Pro Val Arg Val Asp Ser Val Phe Ala Leu Arg Ser Phe
        515                 520                 525

Val Glu Ala Cys Lys Asp Leu Asp Glu Ile Arg Pro Ile Leu Pro Gln
    530                 535                 540

Leu Leu Asp Glu Phe Phe Lys Leu Met Gly Glu Val Glu Asn Glu Asp
545                 550                 555                 560

Leu Val Phe Thr Leu Glu Thr Ile Val Asp Arg Phe Gly Glu Glu Met
                565                 570                 575

Ala Pro Tyr Ala Leu Gly Leu Cys Gln Ser Leu Ala Ala Ala Phe Trp
            580                 585                 590

Arg Cys Met Ala Ser Ser Glu Ala Asp Asp Glu Val Glu Asp Ser Gly
        595                 600                 605

Ala Leu Ala Ala Val Gly Cys Leu Arg Ala Leu Ser Thr Ile Leu Glu
    610                 615                 620

Ser Ile Ser Ser Leu Pro His Leu Phe Ile Gln Ile Glu Pro Thr Leu
625                 630                 635                 640

Leu Pro Ile Leu Arg Arg Met Leu Thr Ser Asp Gly Gln Asp Val Tyr
                645                 650                 655

Glu Glu Val Leu Glu Ile Val Ser Tyr Leu Thr Phe Tyr Ser Pro Thr
            660                 665                 670

Ile Ser Leu Asp Met Trp Ser Leu Trp Pro Leu Met Met Glu Ala Leu
        675                 680                 685

Asn Asp Trp Ala Ile Asp Phe Phe Glu Asn Ile Leu Val Pro Leu Asp
    690                 695                 700

Asn Tyr Ile Ser Arg Gly Thr Glu His Phe Val Thr Cys Lys Asp Pro
705                 710                 715                 720

Asp Tyr Gln Gln Cys Leu Trp Lys Gly Leu Ser Ser Val Met Thr Asp
                725                 730                 735

Gln Asn Met Glu Asp Ser Asp Ile Val Pro Ala Pro Lys Leu Ile Glu
            740                 745                 750

Val Phe Phe Gln Asn Cys Lys Gly Gln Val Asp His Trp Val Glu Pro
        755                 760                 765

Tyr Leu Arg Leu Thr Ile Asp Arg Leu Arg Arg Ala Glu Lys Pro Tyr
    770                 775                 780

Leu Lys Ser Leu Leu Val Gln Val Ile Ala Asn Val Phe Tyr Tyr Asn
785                 790                 795                 800

Pro Ser Leu Thr Leu Ala Met Leu His Lys Leu Gly Val Ala Thr Glu
                805                 810                 815

Ile Phe Asn Leu Trp Phe Val Met Leu Gln Gln Val Lys Lys Ser Gly
            820                 825                 830

Lys Arg Val Asn Phe Lys Arg Glu His Asp Lys Lys Val Cys Cys Leu
        835                 840                 845

Gly Leu Thr Ser Leu Ile Gly Leu Pro Ala Asn His Ile Pro Ala Glu
    850                 855                 860

Ala Leu Glu Arg Ile Phe Lys Ala Thr Leu Glu Leu Leu Val Ala Tyr
865                 870                 875                 880

Lys Glu Gln Val Ala Glu Ser Lys Arg Gln Asn Ala Ala Ala Ala Asp
                885                 890                 895

Asp Val Asp Glu Phe Asp Ala Asp Glu Glu Glu Asn Glu Glu Asp Glu
            900                 905                 910

Asp Asp Gly Glu Met Gly Val Asp Asp Glu Asp Gln Asp Glu Val Asn
        915                 920                 925

Ser Leu Asn Ile Gln Lys Leu Val Gln Ala Arg Gly Phe Gln Leu His
    930                 935                 940

Asp Glu Asp Asp Asp Asp Asp Asp Ser Asp Asp Asp Phe Ser Asp Asp
945                 950                 955                 960

Glu Glu Leu Gln Thr Pro Ile Asp Glu Val Asp Pro Phe Ile Phe Phe
                965                 970                 975

Val Gly Thr Ile Gln Ala Val Gln Ala Ser Asp Pro Ala Arg Phe Gln
            980                 985                 990

Ser Leu Met Gln Thr Leu Asp Phe  His Tyr Gln Ala Leu  Ala Asn Gly
        995                 1000                 1005

Val Ala  Gln His Ala Glu Glu  Arg Lys Val Glu Ile  Glu Lys Glu
    1010                 1015                 1020

Lys Leu  Glu Lys Ala Asn Ala  Gln
    1025                 1030

<210>  25
<211>  86

<212>  PRT
<213>  Artificial Sequence

<220>
<223>  consensus sequence


<220>
<221>  misc_feature
<222>  (2)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (19)..(19)
<223>  Xaa can be any naturally occurring amino acid

<220>
<221>  misc_feature
<222>  (21)..(21)
<223>  Xaa can be any naturally occurring amino acid

<220>
<221>  misc_feature
<222>  (23)..(23)
<223>  Xaa can be any naturally occurring amino acid

<220>
<221>  misc_feature
<222>  (28)..(28)
<223>  Xaa can be any naturally occurring amino acid

<220>
<221>  misc_feature
<222>  (46)..(46)
<223>  Xaa can be any naturally occurring amino acid

<220>
<221>  misc_feature
<222>  (71)..(71)
<223>  Xaa can be any naturally occurring amino acid

<220>
<221>  misc_feature
<222>  (73)..(73)
<223>  Xaa can be any naturally occurring amino acid

<220>
<221>  misc_feature
<222>  (78)..(78)
<223>  Xaa can be any naturally occurring amino acid

<220>

<221>  misc_feature
<222>  (81)..(81)
<223>  Xaa can be any naturally occurring amino acid

<400>  25

Gly Xaa Xaa Asp Xaa Xaa Leu Pro Val Arg Xaa Asp Ser Val Phe Ala
1               5                   10                  15

Leu Arg Xaa Phe Xaa Glu Xaa Cys Lys Asp Leu Xaa Glu Ile Arg Pro
            20                  25                  30

Ile Leu Pro Gln Leu Leu Asp Glu Phe Phe Lys Leu Met Xaa Glu Val
        35                  40                  45

Glu Asn Glu Asp Leu Val Phe Thr Leu Glu Thr Ile Val Asp Lys Phe
    50                  55                  60

Gly Glu Glu Met Ala Pro Xaa Ala Xaa Gly Leu Cys Gln Xaa Leu Ala
65                  70                  75                  80

Xaa Phe Trp Arg Cys Met
                85

<210>  26
<211>  44
<212>  PRT
<213>  Artificial Sequence

<220>
<223>  consensus sequence


<220>
<221>  misc_feature
<222>  (6)..(6)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (13)..(15)
<223>  Xaa can be any naturally occurring amino acid

<220>
<221>  misc_feature
<222>  (20)..(20)
<223>  Xaa can be any naturally occurring amino acid

<220>
<221>  misc_feature
<222>  (28)..(28)
<223>  Xaa can be any naturally occurring amino acid

<220>
<221>  misc_feature
<222>  (37)..(37)
<223>  Xaa can be any naturally occurring amino acid

<220>
<221>  misc_feature
<222>  (40)..(40)
<223>  Xaa can be any naturally occurring amino acid

<220>
<221>  misc_feature
<222>  (42)..(42)
<223>  Xaa can be any naturally occurring amino acid

<400>  26

Glu Arg Pro Val Pro Xaa Glu Gly Gln Pro Xaa Asp Xaa Xaa Xaa Arg
1               5                   10                  15

Lys Ser Trp Xaa Trp Trp Lys Val Lys Lys Trp Xaa Val His Ile Leu
            20                  25                  30

Asn Arg Leu Tyr Xaa Arg Phe Xaa Asp Xaa Lys Leu
        35                  40